# EUROPEAN PATENT SPECIFICATION

(11) **EP 1 830 853 B1**
(45) Date of publication and mention of the grant of the patent: **10.08.2011**
(21) Application number: 05854156.6
(22) Date of filing: 14.12.2005
(51) Int. Cl.: A61K 31/497, A61K 31/551, C07D 401/00, A61P 9/10, A61P 9/12, A61P 11/00, A61P 11/06, A61P 19/10, A61P 27/02, A61P 27/06, A61P 29/00, A61P 35/00, A61P 43/00

(54) **AMINOPYRAZINE ANALOGS FOR TREATING GLAUCOMA AND OTHER RHO KINASE-MEDIATED DISEASES**
AMINOPYRAZIN-ANALOGA ZUR BEHANDLUNG VON GLAUKOMEN UND ANDEREN DURCH RHO-KINASE VERURSACHTEN KRANKHEITEN
ANALOGUES D'AMINOPYRAZINE SERVANT A TRAITER LE GLAUCOME ET D'AUTRES MALADIES OU ETATS MEDIES PAR RHO KINASE

(30) Priority: 27.12.2004 US 639389 P
(43) Date of publication of application: 12.09.2007
(73) Proprietor: Novartis AG, 4056 Basel (CH)
(72) Inventor: HELLBERG, Mark R., Arlington, TX 76017 (US); RUSINKO, Andrew, Arlington, TX 76017 (US); HENDERSON, Alan, J, Albany, New York 12208 (US); GUO, Cheng, Schenectady, New York 12303 (US); HADDEN, Mark, Albany, New York 12203 (US); DECORNEZ, Hélène, Y., Cliffton Park, New York 12065 (US)
(74) Representative: Teipel, Stephan
(86) International application number: PCT/US2005/045384
(87) International publication number: WO 2006/071548

(56) References cited:
- WO-A-03/045924
- WO-A-03/051870
- WO-A-2004/018437
- WO-A-2004/084824
- WO-A-2005/040151
- WO-A-2005/058876
- WO-A-2005/095384

## Description

The present invention is directed to aminopyrazine analogs useful to treat rho kinase-mediated diseases and conditions. The invention is particularly directed to lowering and/or controlling normal or elevated intraocular pressure (IOP) and treating glaucoma.

### Background of the Invention

The disease state referred to as glaucoma is characterized by a permanent loss of visual function due to irreversible damage to the optic nerve. The several morphologically or functionally distinct types of glaucoma are typically characterized by elevated IOP, which is considered to be causally related to the pathological course of the disease. Ocular hypertension is a condition wherein intraocular pressure is elevated, but no apparent loss of visual function has occurred; such patients are considered to be at high risk for the eventual development of the visual loss associated with glaucoma. Some patients with glaucomatous field loss have relatively low intraocular pressure. These normotension or low tension glaucoma patients can also benefit from agents that lower and control IOP. If glaucoma or ocular hypertension is detected early and treated promptly with medications that effectively reduce elevated intraocular pressure, loss of visual function or its progressive deterioration can generally be ameliorated.

Drug therapies that have proven to be effective for the reduction of intraocular pressure include both agents that decrease aqueous humor production and agents that increase the outflow facility. Such therapies are in general administered by one of two possible routes, topically (direct application to the eye) or orally. However, pharmaceutical ocular anti-hypertension approaches have exhibited various undesirable side effects. For example, miotics such as pilocarpine can cause blurring of vision, headaches, and other negative visual side effects. Systemically administered carbonic anhydrase inhibitors can also cause nausea, dyspepsia, fatigue, and metabolic acidosis. Certain prostaglandins cause hyperemia, ocular itching, and darkening of eyelashes and periorbital skin. Further, certain beta-blockers have increasingly become associated with serious pulmonary side-effects attributable to their effects on beta-2 receptors in pulmonary tissue. Sympathomimetics cause tachycardia, arrhythmia and hypertension. Such negative side-effects may lead to decreased patient compliance or to termination of therapy such that normal vision continues to deteriorate. Additionally, there are individuals who simply do not respond well when treated with certain existing glaucoma therapies. There is, therefore, a need for other therapeutic agents that control IOP.

The small GTPase Rho is involved in many cellular functions including cell adhesion, cell motility, cell migration, and cell contraction. One of the main effectors of such cellular functions is rho-associated coiled-coil-forming protein kinase (rho kinase) which appears to have an important role in the regulation of force and velocity of smooth muscle contraction, tumor cell metastasis and inhibition of neurite outgrowth. Rho kinase is a serine/threonine protein kinase that exists in two isoforms: ROCK1 (ROKβ) and ROCK2 (ROKα) [N. Wettschureck, S. Offersmanns, Journal of Molecular Medicine 80:629-638, 2002; M. Uehata et al., Nature 389:990-994, 1997; T. Ishizaki et al., Molecular Pharmacology 57:976-983, 2000, C. Loge et al., Journal of Enzyme Inhibition and Medicinal Chemistry 17:381-390, 2002 ].

It has been found that certain inhibitors of rho kinase effectively lower and control normal and elevated IOP [M. Honjo, et al., Investigative Ophthalmology and Visual Science 42:137-144, 2001; M. Honjo et al., Archives of Ophthalmology 119:1171-1178, 2001; P.V. Rao et. al., Investigative Ophthalmology and Visual Science 42:10291690, 2001; M. Waki, Current Eye Research 22:47-474, 2001; B. Tian et al., Archives of Ophthalmology 122:1171-1177, 2004]. Rho kinase inhibitors such as H-7 and Y-27632 inhibit ciliary muscle contraction and trabecular cell contraction, effects that may be related to the ocular hypotensive effect of this class of compounds [H. Thieme et al., Investigative Ophthalmology and Visual Science 41:4240-4246, 2001; C. Fukiage et al., Biochemical and Biophysical Research Communications 288:296-300, 2001].

Compounds that act as rho kinase inhibitors are well known and have shown a variety of utilities. Pyridine, indazole, and isoquinoline compounds that have rho kinase activity are described by Takami et al., Biorganic and Medicinal Chemistry 12:2115-2137, 2004. U.S. Patent Nos. 6,218,410 and 6,451,825 disclose the use of rho kinase inhibitors for the treatment of hypertension, retinopathy, cerebrovascular contraction, asthma, inflammation, angina pectoris, peripheral circulation disorder, immature birth, osteoporosis, cancer, inflammation, immune disease, autoimmune disease and the like. U.S. Patent No. 6,794,398 discloses the use of a compound with rho kinase activity for the prevention or treatment of liver diseases. U.S. Patent No. 6,720,341 discloses the use of compounds with rho kinase activity for the treatment of kidney disease. WO 99/23113 discloses the use of rho kinase inhibitors to block the inhibition of neurite outgrowth. WO 03/062227 discloses 2,4-diaminopyrimidine derivatives as rho kinase inhibitors. WO 03/059913 discloses bicyclic 4-aminopyrimidine analogs as rho kinase inhibitors. WO 02/100833 discloses heterocyclic compounds as rho kinase inhibitors. WO 01/68607 discloses amide derivatives as rho kinase inhibitors. WO 04/024717 discloses amino isoquinoline derivatives as rho kinase inhibitors. WO 04/009555 discloses 5-substituted isoquinoline derivatives as rho kinase inhibitors useful for treating glaucoma, bronchial asthma and chronic obstructive pulmonary disease. EP1034793 discloses the use of rho kinase inhibitors for the treatment of glaucoma.

U.S. Patent Nos. 6,503,924, 6,649,625, and 6,673,812 disclose the use of amide derivatives that are rho kinase inhibitors for the treatment of glaucoma. U.S. Patent Nos. 5,798,380 and 6,110,912 disclose a method for treating glaucoma using serine/threonine kinase inhibitors. U.S. Patent No. 6,586,425 discloses a method for treating glaucoma using serine/threonine kinase inhibitors. U.S. Patent Application Publication No. 20020045585 discloses a method for treating glaucoma using serine/threonine kinase inhibitors.

The following references disclose the activity of isoquinoline sulfonamide analogs as rho kinase inhibitors: Y. Sasaki, Cellular Biology Molecular Letters 6:506, 2001; S. Satoh et al., Life Sciences 69:1441-1453, 2001; Y. Sasaki, Pharmacology and Therapeutics 93:225-232, 2002; C. Loge et al., Journal of Enzyme Inhibition and Medicinal Chemistry 18:127-138. The use of certain isoquinolinesulfonyl compounds for the treatment of glaucoma has been disclosed in U.S. Patents 6,271,224 and 6,403,590. Also, WO 04/000318 discloses the use of amino-substituted monocycles as AKT-1 kinase modulators.

Several publications have described the synthesis of pyrazines. WO 04/084824 describes the preparation of biaryl substituted 6-membered heterocycles for use as sodium channel blockers. WO 04/085409 describes the preparation of libraries of compounds, including pyrazines, that are capable of binding to the active site of protein kinase. Other pyrazine synthesis publications include: Sato et al., Journal of Chemical Research 7:250-1, 1997; Sato et al., Synthesis 9:931-4, 1994; Sato, Journal of the Chemical Society 7:885-8, 1994; Sato, Journal of Organic Chemistry 43(2):341-3, 1978; Adachi, J et al., Journal of Organic Chemistry 37(2):221-5, 1972.

### Summary of the Invention

The present invention is directed to aminopyrazine analogs such as 2-aminopyrazine and 5-substituted 2,3 diaminopyrazines and derivatives described herein for the treatment of rho kinase-mediated diseases and conditions. The subject compounds of Formula (I), described below, can be used to lower and/or control IOP associated with normal-tension glaucoma, ocular hypertension, and glaucoma in warm blooded animals, including man. In certain embodiments, when used to treat normal-tension glaucoma or ocular hypertension, the compounds may be formulated in pharmaceutically acceptable compositions suitable for topical delivery to the eye.

In other embodiments, the described rho kinase inhibitors of Formula (I) can be used for the manufacture of an ophtalmic pharmaceutical composition for the treatment of glaucoma, and/or control of intraocular pressure.

An embodiment of the present invention contemplates an ophthalmic pharmaceutical composition useful in the treatment of glaucoma and control of intraocular pressure, comprising an effective amount of a compound according to Formula (I) disclosed below.

As used herein, the term "rho kinase-mediated disease" or "rho kinase-mediated condition," means any disease or other deleterious condition in which rho kinase is known to play a role. Such conditions include hypertension, glaucoma, retinopathy, cerebrovascular contraction, ocular hypertension, normal-tension glaucoma, chronic obstructive pulmonary disease, asthma, inflammation, angina pectoris, peripheral circulation disorder, immature birth, osteoporosis, cancer, inflammation, immune disease, autoimmune disease.

The foregoing brief summary broadly describes the features and technical advantages of certain embodiments of the present invention. Additional features and technical advantages will be described in the detailed description of the invention that follows.

### Detailed Description of the Invention

The compounds disclosed and utilized in embodiments of the present invention have the following formula: 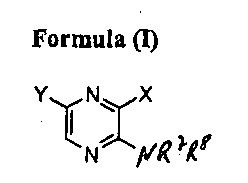 in which Y is selected from the following groups: 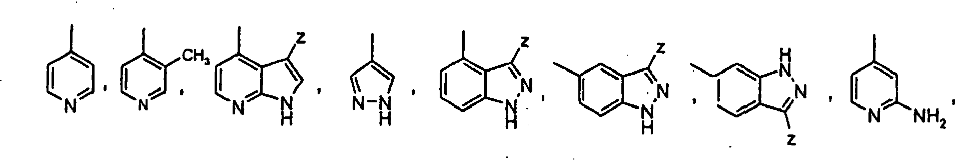 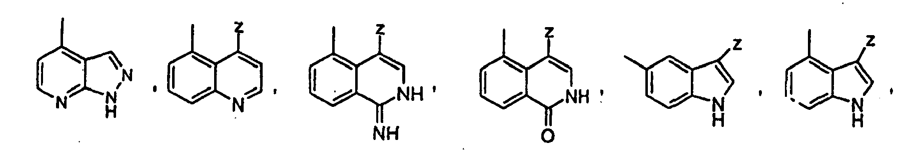 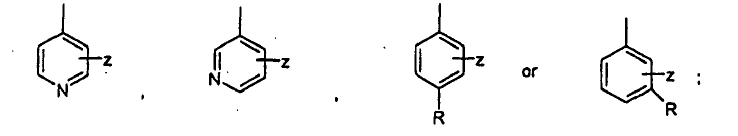 where:
X = OR¹, NR²R³_{;}
z = H, OR⁶, halogen, CF₃, or C₁-C₄ alkyl;
R is OH, OR⁴, or S(O)ₙR⁶;
n is 0, 1 or 2;
R¹, R², R³ independently = H, C₁-C₆ alkyl optionally substituted by NR⁴R⁵, OH, OR⁶, aryl, heterocyclyl or heteroaryl, C₃-C₈ cyclic alkyl optionally substituted by NR⁴R⁵, OH, OR⁶, aryl, heterocyclyl, or heteroaryl, and heterocyclyl;
R² and R³ together can form a heterocyclic ring;
R⁴, R⁵ independently = H, C₁-C₆ alkyl optionally substituted by OH, OR⁶, aryl, heterocyclyl, or heteroaryl;
R⁶= C₁-C₆ alkyl, aryl, or CF₃;
R⁷, R⁸ independently = H, C₁-C₆ alkyl optionally substituted by NR⁴R⁵, OH, OR⁶, or heterocycl, C₃-C₈ cyclic alkyl optionally substituted by NR⁴R⁵, OH, OR⁶, or heterocyclyl, and heterocyclyl; and
R⁷ and R⁸ together can form a heterocyclic ring.

The compounds utilized in preferred embodiments are those structures according to Formula (I) in which Y is selected from the following groups: 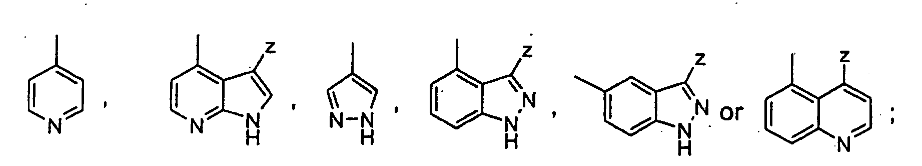 where:
z = H, C₁ alkyl;
X = OR¹ NR²R³_{;}
R¹ = C₁-C₆ alkyl optionally substituted by NR⁴R⁵, OH, OR⁶, or heterocyclyl, C₃-C₈ cyclic alkyl optionally substituted by NR⁴R⁵, OH, OR⁶, or heterocyclyl, or 4-8 membered heterocyclic ring;
R² = H, C₂-C₄ alkyl optionally substituted by NR⁴R⁵, OH, OR⁶;
R³ = C₂-C₄ alkyl optionally substituted by NR⁴R⁵, OH, OR⁶, C₃-C₈ cyclic alkyl optionally substituted by NR⁴R⁵, OH, OR⁶, or 4-8 membered heterocyclic ring;
R² and R³ together can form a 4-8 membered heterocyclic ring;
R⁴, R⁵ independently = H, C₁-C₄ alkyl optionally substituted by OH, OR⁶;
R⁶=C₁-C₄ alkyl;
R⁷, R⁸ independently = H, C₁-C₄ alkyl optionally substituted by NR⁴R⁵, OH, OR⁶, or heterocycl, C₃-C₈ cyclic alkyl optionally substituted by NR⁴R⁵, OH, OR⁶, or heterocyclyl, or heterocyclyl;
R⁷ and R⁸ together can form a 4- 7 membered heterocyclic ring.

It is recognized that compounds of Formula (I) can contain one or more chiral centers. This invention contemplates all enantiomers, diastereomers, and mixtures thereof. Furthermore, certain embodiments of the present invention comprise pharmaceutically acceptable salts of compounds according to Formula (I).

The term "aryl" as used herein refers to a monocyclic, bicyclic or tricyclic ring system having a total of five to fourteen ring members, wherein at least one ring in the system is aromatic and wherein each ring in the system contains 3 to 7 ring members. The term "aryl" may be used interchangeably with the term "aryl ring".

The term "heterocycle", "heterocyclyl", or "heterocyclic" as used herein means non-aromatic, monocyclic, bicyclic or tricyclic ring systems having three to fourteen ring members in which one or more ring members is a heteroatom, wherein each ring in the system contains 3 to 7 ring members.

The term "heteroaryl" refers to monocyclic, bicyclic or tricyclic ring systems having three to fourteen ring members wherein at least one ring in the system is aromatic, at least one ring in the system contains one or more heteroatoms, and wherein each ring in the system contains 3 to 7 ring members.

In the above definitions, the total number of carbon atoms in a substituent group is indicated by the Cᵢ₋ⱼ prefix, where the numbers i and j define the number of carbon atoms; this definition includes straight chain, branched chain, and cyclic alkyl or (cyclic alkyl)alkyl groups.

It is important to recognize that a substituent may be present either singly or multiply when incorporated into the indicated structural unit. For example, the substituent halogen, which means fluorine, chlorine, bromine, or iodine, would indicate that the unit to which it is attached may be substituted with one or more halogen atoms, which may be the same or different.

### SYNTHESIS:

Compounds according to Formula (I) can be synthesized using the general and specific examples set forth below.

### Experimental Introduction:

Unless otherwise noted, reagents and solvents were used as received from commercial suppliers. Purifications using the notation 'column' were carried out on an automated Combiflash unit consisting of a gradient mixing system, Foxy 200 fraction collector and a UV/visible detector. Proton and carbon nuclear magnetic resonance spectra were obtained on a Bruker AC 300 or a Bruker AV 300 spectrometer at 300 MHz for proton and 75 MHz for carbon, or on a Bruker AMX 500 spectrometer at 500 MHz for proton and 125 MHz for carbon. Spectra are given in ppm (δ) and coupling constants, *J*, are reported in Hertz. Tetramethylsilane was used as an internal standard for proton spectra and the solvent peak was used as the reference peak for carbon spectra. Mass spectra were obtained on a Finnigan LCQ Duo LCMS ion trap electrospray ionization (ESI) mass spectrometer. Thin-layer chromatography (TLC) was performed using Analtech silica gel plates and visualized by ultraviolet (UV) light unless otherwise stated. HPLC analyses were obtained using a Luna C18(2) column (250 x 4.6 mm, Phenonemex) with UV detection at 254 nm using a standard solvent gradient program (Table 1). Liquid chromatography- mass spectrometry was obtained on a Varian 1200L single quadrapole mass spectrometer using ESI and a Luna C18(2) column (50 x 4.6 mm, Phenonemex) with UV detection at 254 nm using a standard solvent gradient program (Table 2).

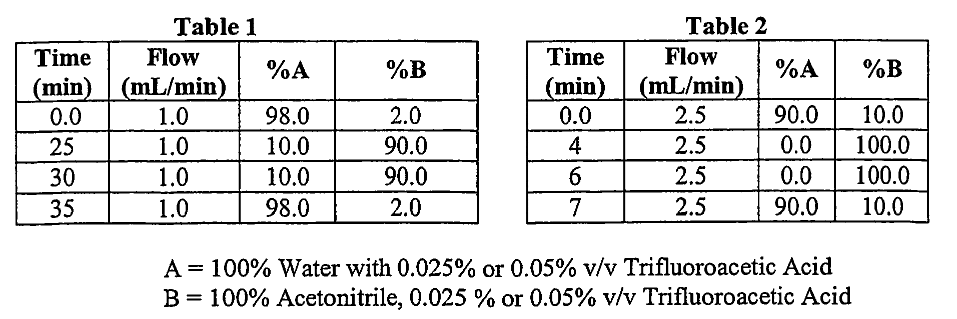

### General Synthesis Scheme:

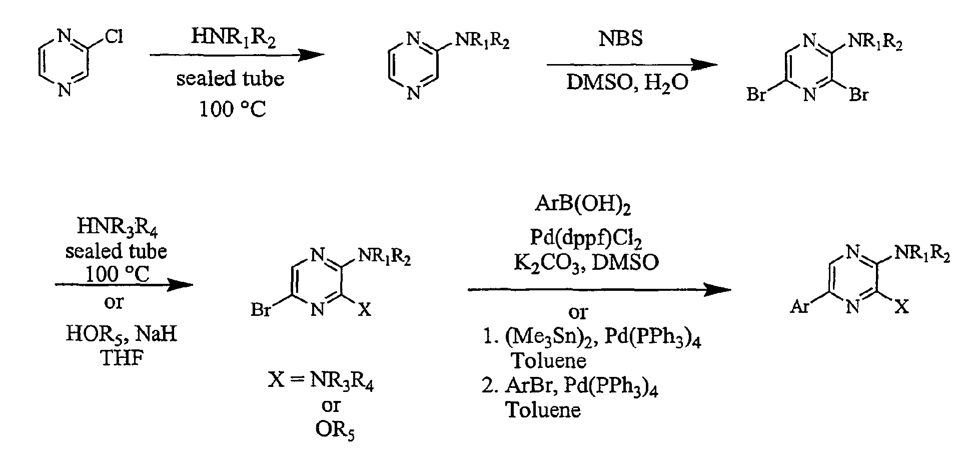

### General Procedure 1: Preparation of Substituted Amine 2 from Primary Amine 1

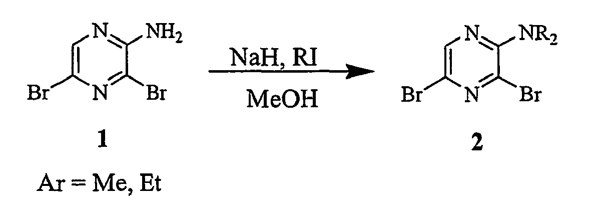

To a stirred solution of amine **1** (1.0 mmol) in dry DMF (3 mL) was added sodium hydride (60% dispersion in mineral oil, 2.2 mmol) and alkyl iodide (4.0 mmol). The resulting mixture was stirred under nitrogen at room temperature for 25 minutes. The reaction mixture was then quenched with water (6mL), extracted with diethyl ether (2 x 12 mL) and the combined organics were washed with brine, dried over sodium sulfate and concentrated. Biorg. Med. Chem. 2001, 9, 1149-1154.

### General Procedure 2: Preparation of Aminopyrazine 4 from Chloropyrazine 3.

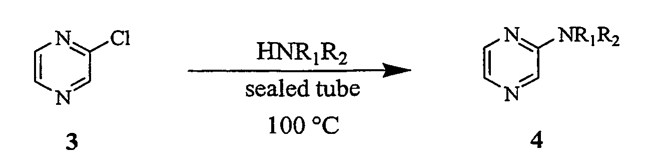

A mixture of chloropyrazine **3** (5.0 mmol) and HNR₁R₂ (10.0 mmol) was heated at 100 °C in a sealed tube for 15 h. The reaction mixture was cooled to room temperature, diluted with methylene chloride (50 mL), washed with saturated sodium bicarbonate solution (10 mL), dried over sodium sulfate and concentrated to provide the product 4, which could be purified by column chromatography, but was usually used in the next step without purification.

### General Procedure 3: Preparation of 3,5-Dibromopyrazin-2-amine 2 from Aminopyrazine 4.

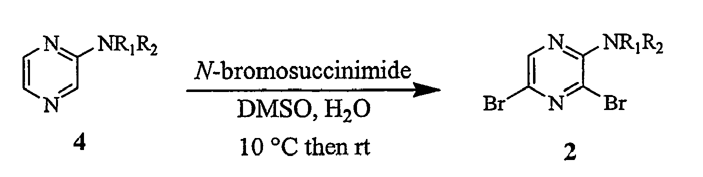

To a stirred solution of aminopyrazine **4** (5.0 mmol) in dimethyl sulfoxide (10 mL)/water (0.20 mL) at 10 °C was added N-bromosuccinimide (10 mmol) in portions. The reaction mixture was then allowed to warm to room temperature slowly and stirred at that temperature overnight. An additional aliquot of *N*-bromosuccinimide (10 mmol) was then added at room temperature. After stirring for 6.5 h, the reaction mixture was poured onto ice (30 g). The precipitate was collected, washed with cold water (2 × 10 mL), and dried to provide the product 2, which could be purified by column chromatography, but was usually used in the next step without purification.

### General Procedure 4: Amination of 2-Amino-3,5-dibromopyrazines

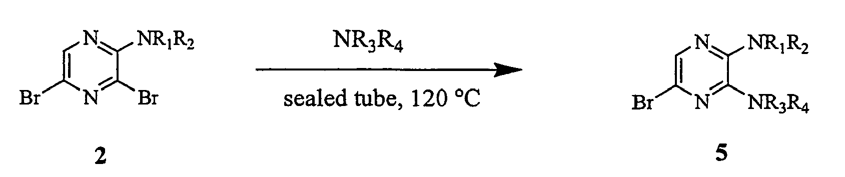

### Method 1

Amine (1 ml) was added to 2-amino-3,5-dibromopyrazine **2** (0.791 mmol) and the mixture was heated to 120 °C in a sealed tube. The reaction was allowed to proceed for 18 h. The solution was cooled, partitioned between methylene chloride and water (1:1, 200 ml) and the organic phase removed. The aqueous phase was extracted with methylene chloride (50 ml) and the combined organic layers were dried over Na₂SO₄ and concentrated giving the product **5**.

### Method 2

Amine (1.58 mmoll) was added to 2-amino-3,5-dibromopyrazine **2** (0.791 mmol) in DMSO or ethanol (0.5 ml) and the mixture was heated to 120°C in a sealed tube. If the amine was in short supply, one equivalent of amine and one equivalent of *i*Pr₂NEt were used. The reaction was allowed to proceed for 18 h. The solution was cooled, partitioned between methylene chloride and water (1:1, 200 ml) and the organic phase removed. The aqueous phase was extracted with methylene chloride (50 ml) and the combined organic layers were dried over Na₂SO₄ and concentrated giving the product 5.

### General Procedure 5: Preparation of 5-Bromo-3-alkoxypyrazin-2-amine 6 from 3, 5-Dibromopyrazin-2-amine 2.

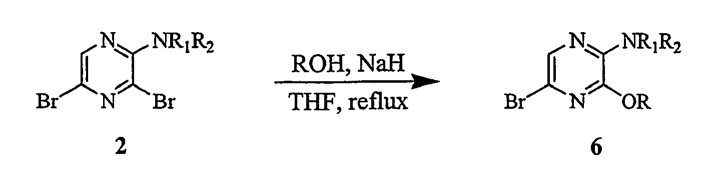

To a stirred solution of the alcohol ROH (1.80 mmol) in tetrahydrofuran (3 mL) at room temperature was added sodium hydride (60% dispersion in mineral oil; 1.80 mmol). The mixture was stirred at ambient temperature for 30 min. The 3,5-dibromopyrazin-2-amine **2** (0.59 mmol) was then added and the reaction mixture was heated at reflux overnight, cooled to room temperature, quenched with water (3 mL) and extracted with ethyl acetate (3 × 5 mL). The combined extracts were washed with brine (10 mL), dried over sodium sulfate and concentrated to provide the product 6, which could be purified by column chromatography, but was usually used in the next step without purification.

### General Procedure 6: Suzuki Coupling of 2,3-Diamino-5-bromopyrazine/2-Amino-3-alkoxy-5-bromopyrazine 5 and 6 with Boronic Acids

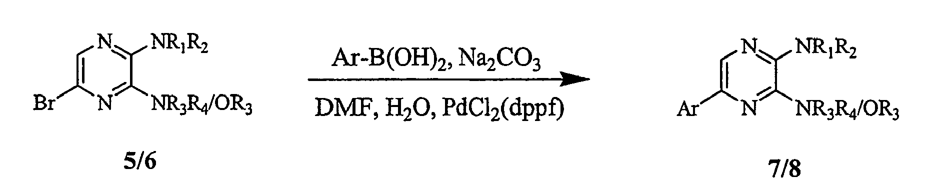

### Method 1

To a stirred solution of aryl bromide **5/6** (0.699 mmol) in dry DMSO (2 mL) was added the boronic acid (2.10 mmol, 3 eq.), K₂CO₃ (3.50 mmol, 5 eq.) and Pd(dppf)Cl₂ (0.07 mmol, 0.1 eq.). The resulting mixture was degassed under high vacuum for 10 min, then flushed with nitrogen. This process was repeated twice more. The reaction mixture was then heated to 100 °C and held for 2 h, or until all the starting bromide had been consumed. The mixture was cooled, quenched with water (100 ml) and extracted with ethyl acetate (2 x 100 ml). The combined organic phases were washed with sat. NaCl (2 x 100 ml), dried over Na₂SO₄ and concentrated giving the coupled product **7/8**.

### Method 2

Aryl bromide **5/6** (0.699 mmol), boronic acid (2.10 mmol, 3 eq.) and K₂CO₃ (3.50 mmol, 5 eq.) were stirred in DMF (7 mL) and water (3 mL) and the resulting mixture was degassed with a nitrogen stream as the temperature was increased to 100 °C. After degassing at this temperature for 10 min, Pd(dppf)Cl₂ (0.07 mmol, 0.1 equiv) was added and the reaction was stirred at 100°C under a nitrogen atmosphere for 18 h. Upon cooling the mixture was poured into water (100 mL) and stirred for 10 min. The mixture was extracted with ethyl acetate and the combined organic extracts washed with 5% lithium chloride (5x), dried over sodium sulfate and concentrated to provide the coupled product **7/8**.

### General Procedure 7: Preparation of Aryl Stannane 9/10 from Aryl Bromide 5/6.

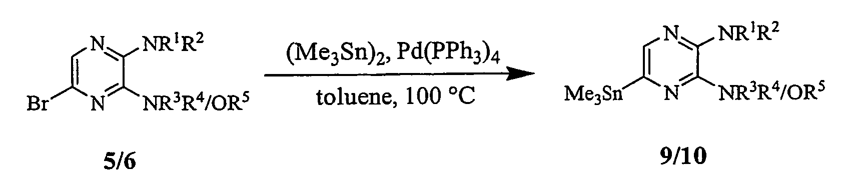

Aryl bromide **5/6** (2.0 mmol) and hexamethylditin (3.0 mmol) were stirred dry toluene (10 mL) and degassed with a nitrogen stream as the temperature was increased to 100°C. Palladium tetrakistriphenylphosphine (0.2 mmol) was added and the reaction maintained at 100 °C under a nitrogen atmosphere for 2-16 h. Upon cooling the mixture was concentrated and purified without work-up providing the desired stannane.

### General Procedure 8 : Stille-Coupling of Stannane 9/10 with Aryl Bromides to Provide Biaryls 11/12

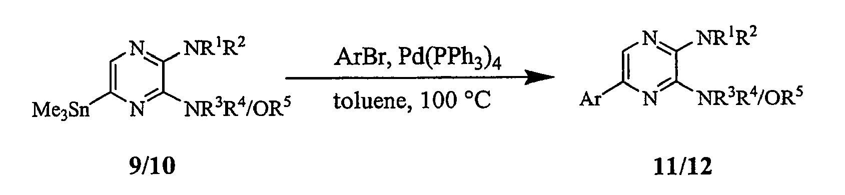

Stannane **9/10** (2.0 mmol) and aryl bromide (3.0 mmol, 1.5 equiv) were stirred in dry toluene (10 mL) and degassed with a nitrogen stream as the temperature was increased to 100 °C. Palladium tetrakistriphenylphosphine (0.2 mmol) was added and the reaction maintained at 100 °C under a nitrogen atmosphere for 16 h. Upon cooling the mixture was concentrated and purified without work-up providing the coupled product.

### Specific Examples:

### Example 1

### 3-(4-Methyl-1,4-diazeyan-1-yl)-5-(pyridin-4-yl)pyrazin-2-amine

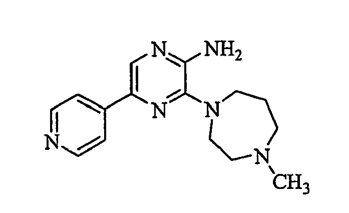

**Step A:** 5-Bromo-3-(4-methyl-1,4-diazepan-1-yl)pyrazin-2-amine Prepared from commercially available 2-amino-3,5-dibromopyrazine and 1-methylhomopiperazine according to general procedure **4** (method 1) providing the diaminopyrazine (204 mg, 90%) as a dark oil; ¹H NMR (500 MHz, CDCl₃) δ 7.65 (s, 1H), 4.49 (br s, 2H), 3.51-3.48 (m, 4H), 2.74-2.73 (m, 2H), 2.71-2.67 (m, 2H), 2.41 (s, 3H), 2.00-1.93 (m, 2H); ES-MS: (M + H) = 286, 288 m/z.
**Step B:** Prepared from the product of Step A and 4-pyridylboronic acid according to general procedure 6 (method 1). Purification by column chromatography (12 g ISCO column eluting with methylene chloride and a 10:1 methanol/ammonium hydroxide mixture; gradient 100% methylene chloride to 95% then 90% and finally 85% methylene chloride) provided the title compound (123 mg, 62%) as a light green solid; ¹H NMR (500 MHz, CDCl₃) δ 8.63-8.62 (d, *J* = 5.4 Hz, 2H), 8.17 (s, 1H), 7.80-7.79 (d, *J* = 5.3 Hz, 2H), 4.83 (br s, 2H), 3.61-3.57 (m, 4H), 2.80-2.78 (m, 2H), 2.74-2.72 (m, 2H), 2.44 (s, 3H), 2.03-2.01 (m, 2H); ¹³C NMR (125 MHz, CDCl₃) δ 150.2, 147.8, 146.9, 144.8, 136.8, 131.6, 119.5, 58.8, 57.5, 50.2, 49.4, 47.2, 28.4; HPLC t_{R} = 3.57 min (Eluent 90:10 to 10:90 water/acetonitrile over 20 min then hold for 10 min), 100%; ES-MS: (M + H) = 285 m/z.

### Example 2

### 3-(4-Methyl-1,4-diazepan-1-yl)-5-(3-methylpyridin-4-yl)pyrazin-2-amine hydrochloride

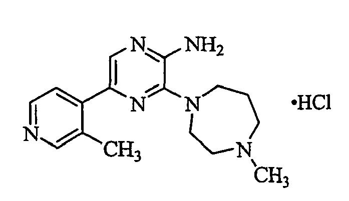

Prepared from the product of Step A in example 1 and 3-methyl-4-pyridylboronic acid under similar conditions. Purification by column chromatography (12 g ISCO column eluting with methylene chloride and methanol/ammonia mixture (10:1); gradient 100% methylene chloride to 80% methylene chloride over 30 min at 25 mL/min) followed by conversion to the hydrochloride salt with 2N HCl in diethyl ether provided the title compound (41 mg, 34%) as brown solid; ¹H NMR (500 MHz, CD₃OD) δ 8.5 (s, 1H), 8.47-8.46 (d, *J* = 5.4 Hz, 1H), 8.07 (s, 1H), 7.76-7.75 (d, *J* = 5.5 Hz, 1H), 3.78-3.76 (t, *J* = 4.7 Hz, 2H), 3.55 (br s, 6H), 2.97 (s, 3H), 2.55 (s, 3H), 2.28-2.23 (qui, *J* = 5.9 Hz, 2H); ¹³C NMR (125 MHz, CD₃OD) δ 150.2, 149.7, 149.2, 146.8, 145.0, 137.9, 137.8, 134.1, 125.2, 58.1, 57.0, 50.6, 47.1, 45.1, 26.1, 18.6; HPLC t_{R} = 7.2 min, >99%; ES-MS: (M + H) = 299 m/z.

### Example 3

### 3-(4-Methyl-1,4-diazepan-1-yl)-5-(1H-pyrazol-3-yl)pyrazin-2-amine

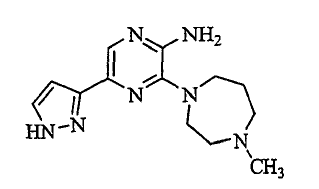

### Step A: 4-(4,4,5,5-Tetramethyl-1,3,2-dioxaborolan-2-yl)-1-((2-(trimethylsilyl)ethoxy) methyl)-1H-pyrazole

4-(4,4,5,5-Tetramethyl-1,3,2-dioxaborolan-2-yl)-1*H*-pyrazole (348 mg, 1.8 mmol) was dissolved in DMF (5 mL) and sodium hydride (60% dispersion, 86 mg, 2.15 mmol) added. The mixture heated to 60 °C for 5 min. Upon cooling and stirring for an additional 15 min, trimethylsilylethoxymethyl chloride (358 mg, 2.15 mmol, 381 µL) was added dropwise over 5 min and mixture stirred for 16 h. The reaction mixture was diluted with ethyl acetate (25 mL), washed with 5% lithium chloride (5x), dried over sodium sulfate and concentrated. The residue was purified by column chromatography (40 g ISCO column eluting with hexanes and ethyl acetate; gradient 100% hexanes to 50% hexanes over 30 min at 30 mL/min) to provide the SEM-protected pyrazole (360 mg, 61%) as a colorless oil; ¹H NMR (500 MHz, CDCl₃) δ 7.84 (s, 1H), 7.80 (s, 1H), 5.42 (s, 2H), 3.56-3.53 (t, *J* = 8.3 Hz, 2H), 1.31 (s, 12H), 0.91-0.87 (t, *J* = 8.3 Hz, 2H), -0.03 (s, 9H).

### Step B: 3-(4-Methyl-1,4-diazepan-1-yl)-5-(1-((2-(trimethylsilyl)ethoxy)methyl)-1H-pyrazol-3-yl)pyrazin-2-amine

The product from Step A was reacted with the product from Step A in example 1 according to general procedure 6 (method 2) and the product purified by column chromatography (12 g ISCO column eluting with methylene chloride and methanol/ammonia mixture (10:1); gradient 100% methylene chloride to 80% methylene chloride over 30 min at 25 mL/min) providing the coupled product (137 mg, 81%) as a brown oil; ¹H NMR (500 MHz, CDCl₃) δ 7.94 (s, 1H), 7.91 (s, 1H), 7.84 (s, 1H), 5.46 (s, 2H), 4.50-4.49 (m, 2H), 3.62-3.53 (m, 6H), 2.79-2.72 (m, 4H), 2.45 (s, 3H), 2.02-1.99 (m, 2H), 0.95-0.92 (t, *J* = 8.2 Hz, 2H), -0.03 (s, 9H).

**Step C:** The product from Step B (137 mg, 0.34 mmol) was heated to 60 °C in a mixture of TFA (5 mL) and water (1 mL) for 1 h. Upon cooling the reaction mixture was concentrated and partitioned between ethyl acetate and saturated sodium carbonate solution and the organic layer removed and concentrated. Purification by column chromatography (12 g ISCO column eluting with methylene chloride and methanol/ammonia mixture (10:1); gradient 100% methylene chloride to 80% methylene chloride over 30 min at 25 mL/min) provided the title compound (37 mg, 27%) as a green solid; ¹H NMR (300 MHz, CD₃OD) δ 7.96 (s, 2H), 7.79 (s, 1H), 3.60-3.51 (m, 4H), 2.84-2.75 (m, 4H), 2.42 (s, 3H), 2.03-1.99 (m, 2H); ¹³C NMR (75 MHz, CD₃OD) δ 148.4, 147.7, 135.9, 129.9, 122.0, 59.2, 58.3, 50.7, 50.3, 47.0, 28.8 (two aromatic signals missing due to overlap); HPLC t_{R} = 6.3 min, >99%; ES-MS: (M + H) = 274 m/z.

### Example 4

### 3-(4-Methyl-1,4-diazepan-1-yl)-5-(1H-pyrrolo[2,3-b]pyridin-4-yl)pyrazin-2-amine

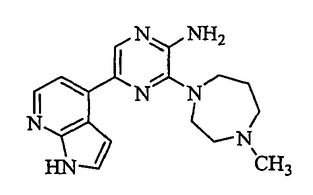

### Step A: 3-(4-Methyl-1,4-diazepan-1-yl)-5-(trimethylstannyl)pyrazin-2-amine

Prepared from the product of Step A in example 1 and hexamethylditin according to general procedure 7. Purification by column chromatography (12 g ISCO column eluting with methylene chloride and methanol/ammonia mixture (10:1); gradient 100% methylene chloride to 80% methylene chloride over 30 min at 25 mL/min) provided the aryl stannane (500 mg, 77%) as yellow solid; ¹H NMR (500 MHz, CD₃OD) δ 7.46 (s, 1H), 3.54-3.51 (m, 2H), 3.47-3.44 (t, *J* = 6.1 Hz, 2H), 2.87-2.82 (m, 4H), 2.64 (s, 3H), 2.02-1.97 (qui ,*J* = 5.8 Hz, 2H), 0.26 (s, 9H).

**Step B:** Prepared from the product of Step A and 4-bromoazaindole (C. Thibault et.al. Org. Lett. 2003, 5(26), 5023-5025) according to general procedure 8. Purification by semi-preparatory HPLC (eluting with acetonitrile (0.05% TFA)/water (0.05% TFA); 5% acetonitrile (0.05% TFA) to 90% acetonitrile (0.05% TFA) over 40 minutes) provided the title compound (35 mg, 26%) as a yellow solid; ¹H NMR (500 MHz, CD₃OD) δ 8.25 (s, 1H), 8.19-8.18 (d, *J* = 5.2 Hz, 1H), 7.54-7.53 (d, *J* = 5.1 Hz,1H), 7.43-7.42 (d, *J* = 4.5 Hz, 1H), 7.02-7.01 (d, *J* = 3.6 Hz, 1H), 3.67-3.65 (t, *J =* 5.2 Hz, 2H), 3.62-3.60 (t, *J* = 6.0 Hz, 2H), 2.89-2.87 (t, *J* = 5.0 Hz, 2H), 2.82-2.80 (t, *J* = 5.7 Hz, 2H), 2.45(s, 3H), 2.06-2.04 (m, 2H); ¹³C NMR (125 MHz, CD₃OD) δ 149.2, 148.3, 147.2, 142.0, 138.4, 137.7, 133.1, 125.8, 117.6, 112.5, 101.2, 57.8, 57.4, 49.8, 49.3, 45.9, 27.6; HPLC t_{R} = 7.1 min, 98.5%; ES-MS: (M + H) = 324 m/z.

### Example 5

### 5-(1H-indazol-4-yl)-3-(4-methyl-1,4-diazepan-1-yl)pyrazin-2-amine dihydrochloride

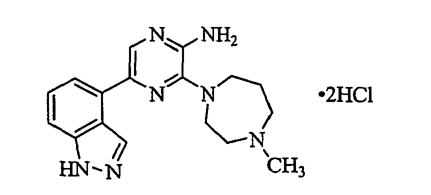

Prepared from the product of Step A (example 4) and 4-bromoindazole according to general procedure 8. Purification by column chromatography (12 g ISCO column eluting with methylene chloride and methanol/ammonia mixture (10:1); gradient 100% methylene chloride to 75% methylene chloride over 30 min at 25 mL/min followed by semi-preparatory HPLC (eluting with acetonitrile (0.05% TFA)/water (0.05% TFA); 5% acetonitrile (0.05% TFA) to 90% acetonitrile (0.05% TFA) over 40 minutes) provided a yellow oil that was treated with 2N HCl in diethyl ether to provide the title compound (12 mg, 8%) as a yellow solid; ¹H NMR (500 MHz, CD₃OD) δ 8.62 (s, 1H), 8.02 (s, 1H), 7.67-7.66 (d, *J* = 7.5 Hz, 2H), 7.57-7.54 (t, *J* = 7.9 Hz, 1H), 4.12-4.02 (m, 2H), 3.91-3.86 (m, 1H), 3.82-3.74 (m, 2H), 3.70-3.61 (m, 2H), 3.46-3.41 (m, 1H), 2.98 (s, 3H), 2.41-2.30 (m, 2H); ¹³C NMR (125 MHz, CD₃OD) δ 150.8, 144.0, 139.9, 134.2, 129.9, 128.91, 121.0, 118.4, 112.6, 57.5, 57.0, 50.4, 46.8, 44.9, 25.5 (two aromatic signals missing due to overlap); HPLC t_{R} = 9.9 min, >100%; ES-MS: (M + H) = 324 m/z.

### Example 6

### 5-(1H-Indazol-6-yl)-3-(4-methyl-1,4-diazepan-1-yl)pyrazin-2-amine dihydrochloride

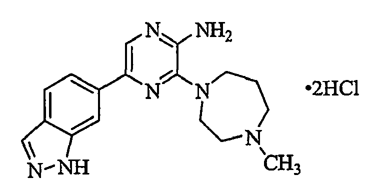

### Step A: 6-Bromo-1H-indazole:

6-Aminoindazole (1.33 g, 10 mmol) was dissolved in 48% hydrobromic acid (5 mL) and water (16 mL). To the resulting solution at 0 °C was added dropwise a solution of sodium nitrite (0.77 g, 11 mmol) in water (9 mL). The mixture was stirred at 0 °C for 15 min. Urea (0.40 g) was added to remove excess nitrous acid. After stirring for 10 min, this solution was added dropwise to a stirred mixture of copper(I) bromide (4.3 g, 30 mmol), 48% hydrobromic acid (10 mL) and water (24 mL) at room temperature. The reaction mixture was heated at 75-80 °C for 1.5 h, cooled to room temperature, basified with concentrated ammonium hydroxide, and extracted with chloroform (4 × 30 mL). The combined extracts were dried over sodium sulfate and concentrated to provide the bromoindazole (0.96 g, 48%) as a greenish yellow solid; ¹H NMR (500 MHz, DMSO-*d*₆) δ 13.16 (s, 1H), 8.09 (s, 1H), 7.67 (s, 1H), 7.74-7.72 (d, *J* = 8.5 Hz, 1H), 7.25-7.23 (dd, *J* = 8.5, 1.4 Hz, 1H).

### Step B: 6-Bromo-2-((2-(trimethylsilyl)ethoxy)methyl)-2H-indazole and 6-Bromo-1-((2-(trimethylsilyl)ethoxy)methyl)-1H-indazole:

To a stirred solution of the product from Step A (0.48 g, 2.4 mmol) in *N,N-*dimethylformamide (4 mL) at room temperature was added sodium hydride (60% dispersion in mineral oil, 96 mg, 2.4 mmol). After stirring for 45 min, 2-(trimethylsilyl)ethoxymethyl chloride (0.51 mL, 2.9 mmol) was added dropwise. Stirring was continued for 18 h. The reaction was quenched with water (10 mL) and extracted with ethyl acetate (4 × 10 mL). The combined extracts were washed with 5% lithium chloride (15 mL), dried over sodium sulfate, and concentrated. The residue was purified by column chromatography (12 g ISCO column eluting with hexanes and ethyl acetate; gradient 100% hexanes to 85% hexanes) to provide 6-bromo-2-((2-(trimethylsilyl)ethoxy)methyl)-2*H*-indazole (0.45 g, 56%) as a yellow oil and 6-bromo-1-((2-(trimethylsilyl)ethoxy)methyl)-1*H*-indazole (0.21 g, 27%) as a yellow oil.
6-Bromo-2-((2-(trimethylsilyl)ethoxy)methyl)-2*H*-indazole: ¹H NMR (500 MHz, DMSO-*d*₆) δ 8.16 (d, *J* = 0.8 Hz, 1H), 8.06 (s, 1H), 7.77-7.75 (d, *J* = 8.5 Hz, 1H), 7.34-7.31 (dd, *J* = 8.5, 1.6 Hz, 1H), 5.75 (s, 2H), 3.51-3.48 (t, *J* = 8.0 Hz, 2H), 0.80-0.77 (t, *J* = 8.0 Hz, 2H), -0.11 (s, 9H).
6-Bromo-1-((2-(trimethylsilyl)ethoxy)methyl)-2H-indazole: ¹H NMR (500 MHz, DMSO-*d*₆) δ 8.58 (d, *J* = 0.8 Hz, 1H), 7.91 (s, 1H), 7.75-7.73 (d, *J* = 8.8 Hz, 1H), 7.17-7.15 (dd, *J* = 8.8, 1.6 Hz, 1H), 5.71 (s, 2H), 3.61-3.58 (t, *J* = 8.0 Hz, 2H), 0.86-0.83 (t, *J* = 8.0 Hz, 2H), -0.06 (s, 9H).

### Step C: 3-(4-Methyl-1, 4-diazepan-1-yl)-5-(2-((2-(trimethylsilyl)ethoxy)methyl)-2H-indazol-6-yl)pyrazin-2-amine:

Prepared from the product of Step A (example 4) and Step B according to general procedure 8. Purification by column chromatography (12 g ISCO column eluting with methylene chloride and methanol/concentrated ammonium hydroxide (10:1); gradient 100% methylene chloride to 90% methylene chloride) provided the coupled product (0.34 g, 96%) as tan solid : ¹H NMR (500 MHz, CDCl₃) δ 8.24 (s, 1H), 8.05 (d, *J* = 0.7 Hz, 1H), 8.00 (d, *J* = 0.7 Hz, 1H), 7.78-7.76 (dd, *J* = 8.5, 0.7 Hz, 1H), 7.74-7.72 (dd, *J* = 8.5, 0.7 Hz, 1H), 4.95 (br s, 2H), 3.81 (br s, 2H), 3.59-3.56 (m, 4H), 3.15 (br s, 4H), 2.71 (br s, 4H), 2.40 (s, 3H), 0.90-0.87 (t, *J* = 8.2 Hz, 2H), -0.07 (s, 9H); ES-MS: (M + H) = 454 m/z.

**Step D:** A solution of the product from Step C (0.34 g, 0.75 mmol) in 6 N HCl (20 mL)/ethanol (20 mL) was heated at reflux for 3 h, cooled to room temperature, diluted with water (20 mL), neutralized with potassium carbonate, and concentrated to dryness. The residue was purified by column chromatography (12 g ISCO column eluting with methylene chloride and methanol/concentrated ammonium hydroxide (10:1); gradient 100% methylene chloride to 85% methylene chloride) to provide a yellow solid. Re-purification by preparative TLC gave a yellow viscous oil, which was converted to the bis-HCl salt using 2 M HCl in diethyl ether to provide the title compound (25 mg, 8%) as a yellow solid; ¹H NMR (500 MHz, CD₃OD) δ 8.15 (br s, 2H), 8.01 (s, 1H), 7.90-7.88 (d, *J* = 8.5 Hz, 1H), 7.74-7.72 (d, *J* = 8.4 Hz, 1H), 4.14-4.10 (dd, *J =* 16.2, 5.4 Hz, 1H), 4.02-3.98 (dd, *J =* 15.2, 8.5 Hz, 1H), 3.90-3.87 (m, 1H), 3.82-3.79 (m, 2H), 3.69-3.68 (m, 2H), 3.44-3.40 (t, *J* = 10.6 Hz, 1H), 3.00 (s, 3H), 2.38-2.35 (m, 2H); ¹³C NMR (125 MHz, CD₃OD) δ 150.3, 144.0, 141.8, 139.1, 137.4, 133.5, 123.4, 121.4, 117.2, 109.0, 57.4, 57.0, 50.1, 46.6, 45.0, 25.5 (one aromatic signal missing due to overlap); HPLC t_{R} = 9.80 min, >99%; ES-MS: (M + H) = 324 m/z.

### Example 7

### 5-(1H-Indazol-5-yl)-3-(4-methyl-1,4-diazepan-1-yl)pyrazin-2-amine dihydrochloride

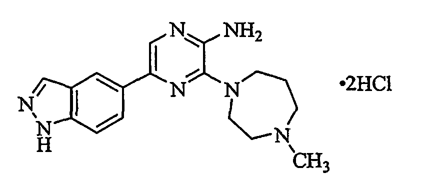

### Step A: 5-Bromo-1H-indazole

Prepared from 5-aminoindazole in a similar manner described in Step A (example 6) providing the bromoindazole (1.32 g, 66%) as an orange-yellow solid; ¹H NMR (500 MHz, DMSO-*d*₆) δ 13.24 (s, 1H), 8.05 (s, 1H), 8.00 (s, 1H), 7.53-7.51 (d, *J* = 8.7 Hz, 1H), 7.45-7.43 (d, *J* = 8.8 Hz, 1H).

### Step B: 5-Bromo-2-((2-(trimethylsilyl)ethoxy)methyl)-2H-indazole and 5-Bromo-1-((2-(trimethylsilyl)ethoxy)methyl)-1H-indazole

Prepared from the product of Step A in a similar manner described in Step B (example 6) providing the mixture of SEM-protected indazoles (0.56 g, 85%) as a yellow oil; is NMR (500 MHz, DMSO-*d*₆) δ major isomer: 8.12 (d, *J* = 0.6 Hz, 1H), 8.04-8.03 (d, *J* = 1.6 Hz, 1H), 7.74-7.73 (d, *J* = 8.9 Hz, 1H), 7.57-7.55 (dd, *J* = 8.8, 1.8 Hz, 1H), 5.75 (s, 2H), 3.51-3.48 (t, *J* = 8.0 Hz, 2H), 0.80-0.77 (t, *J* = 8.0 Hz, 2H), -0.11 (s, 9H); minor isomer: 8.52-8.51 (d, *J* = 0.7 Hz, 1H), 8.01 (d, *J* = 1.5 Hz, 1H), 7.64-7.62 (d, *J* = 9.1 Hz, 1H), 7.35-7.33 (dd, *J* = 9.1, 1.9 Hz, 1H), 5.72 (s, 2H), 3.61-3.58 (t, *J* = 8.0 Hz, 2H), 0.86-0.83 (t, *J* = 8.0 Hz, 2H), -0.06 (s, 9H); ES-MS: (M + H) = 328 m/z.

**Step C:** 3-(4-Methyl-1,4-diazepan-1-yl)-5-(1-((2-(trimethylsilyl)ethoxy)methyl)-1H-indazol-5-yl)pyrazin-2-amine and 3-(4-Methyl-1, 4-diazepan-1-yl)-5-(2-((2-(trimethylsilyl)ethoxy)methyl)-2H-indazol-5-yl)pyrazin-2-amine Prepared from the product of Step B in a similar manner described in Step C (example 6) providing the mixture of coupled indazoles (0.22 g, 68%) as a tan solid.

**Step D:** Prepared from the product of Step C in a similar manner described in Step D (example 6). Purification by column chromatography (12 g ISCO column eluting with methylene chloride and methanol/concentrated ammonium hydroxide (10:1); gradient 100% methylene chloride to 85% methylene chloride) provided a yellow solid. Repurification by preparative TLC gave a yellow viscous oil, which was converted to the bis-HCl salt using 2 M HCl in diethyl ether to provide the title compound (13 mg, 7%) as a yellow solid: ¹H NMR (500 MHz, CD₃OD) δ 8.45 (s, 1H), 8.34 (s, 1H), 8.07-8.05 (d, *J* = 8.9 Hz, 1H), 7.96 (s, 1H), 7.71-7.69 (d, *J* = 8.8 Hz, 1H), 4.14-4.10 (dd, *J =* 16.1, 5.3 Hz, 1H), 4.04-4.00 (dd, *J =* 15.8, 8.4 Hz, 1H), 3.91-3.87 (m, 1H), 3.83-3.77 (m, 2H), 3.69-3.65 (m, 2H), 3.46-3.39 (t, *J* = 11.3 Hz, 1H), 3.00 (s, 3H), 2.44-2.40 (m, 1H), 2.33-2.30 (m, 1H); ¹³C NMR (125 MHz, CD₃OD) δ 150.4, 143.5, 141.7, 140.2, 134.8, 130.4, 127.8, 124.1, 120.1, 116.0, 112.4, 57.5, 57.2, 50.2, 46.7, 45.1, 25.7; HPLC t_{R} = 9.14 min, >99%; ES-MS: (M + H) = 324 m/z.

### Example 8

### 5-(2-Aminopyridin-4-yl)-3-(4-methyl-1,4-diazepan-1-yl)pyrazin-2-amine trihydrochloride

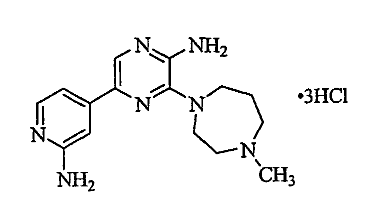

**Step A:** Methyl 4-(5-amino-6-(4-methyl-1,4-diazepan-1-yl)pyrazin-2-yl)picolinate Prepared from the product of Step A (example 4) and 4-chloropyridine-2-carboxylic acid methyl ester according to general procedure 8. Purification by column chromatography (12 g ISCO column eluting with methylene chloride and methanol/ammonia mixture (10:1); gradient 100% methylene chloride to 80% methylene chloride over 30 min at 25 mL/min) provided the coupled product (150 mg, 45%) as a yellow oil; ¹H NMR (300 MHz, CDCl₃) δ 8.74-8.73 (d, *J* = 4.8 Hz, 1H), 8.62 (s, 1H), 8.24 (s, 1H), 8.00-7.99 (dd, *J* = 4.8, 1.5 Hz, 1H), 4.91 (br s, 2H), 4.04 (s, 3H), 3.62-3.58 (m, 4H), 2.82-2.75 (m, 4H), 2.46 (s, 3H), 2.05-2.00 (m, 2H).

### Step B: 4-(5-Amino-6-(4-methyl-1,4-diazepan-1-yl)pyrazin-2-yl)picolinic acid

The product from Step A (150 mg, 0.44 mmol) and lithium hydroxide monohydrate (52 mg, 2.2 mmol) were stirred in a mixture of THF (3 mL) and water (1 mL) for 16 h. The solution was concentrated and then dissolved in methanol (2 mL) and loaded onto an Isolute SCX-2 (5 g) column. Elution with methanol and concentration of the eluent provided the carboxylic acid (122 mg, 84%) as a yellow oil; ¹H NMR (500 MHz, CDCl₃) δ 8.58-8.57 (d, *J* = 1.5 Hz, 1H), 8.53-8.52 (d, *J* = 5.2 Hz, 1H), 8.22 (s, 1H), 7.96-7.95 (dd, *J* = 5.2, 1.8 Hz, 1H), 3.67-3.59 (m, 4H), 2.90-2.88 (m, 2H), 2.82-2.80 (m, 2H), 2.45 (s, 3H), 2.06-2.03 (m, 2H).

### Step C: tert-Butyl 4-(5-amino-6-(4-methyl-1,4-diazepan-1-yl)pyrazin-2-yl)pyridin-2-ylcarbamate

The product from Step B (122 mg, 0.37 mmol), triethylamine (41 mg, 0.45 mmol, 57 µL) and diphenylphorazide (122 mg, 0.45 mmol) were heated to 90°C in a mixture of DMF (1 mL) and *tert*-butanol (1 mL) for 16 h. The mixture was diluted with ethyl acetate. washed with 5% lithium chloride (5X), dried over sodium sulfate and concentrated to provide a yellow oil. Purification by preparatory TLC eluting with methylene chloride/methanol/ammonia (160:18:2) provided the carbamate (26 mg, 17%) as a colorless oil; ¹H NMR (500 MHz, CDCl₃) δ 8.47 (s, 1H), 8.28 (s, 1H), 8.25-8.24 (d, *J* = 5.4 Hz, 1H), 7.49 (s, 1H), 7.42-7.40 (dd, *J* = 5.4, 1.3 Hz, 1H), 5.15 (br s, 2H), 3.59-3.26 (br m, 8H), 2.90 (s, 3H), 1.57-1.45 (m, 2H), 1.53 (s, 9H).

**Step D:** The product from Step C (26 mg, 0.065 mmol) was stirred in TFA (2 mL) for 2 h and then concentrated. The residue was dissolved in methanol and loaded onto an Isolute SCX-2 (5 g) column. Elution with 7N NH₃ in methanol and concentration of the eluent provided the free-base which was subsequently converted to the *tris* hydrochloride salt with 2N HCl in diethyl ether to yield the title compound (7 mg, 26%) as an orange solid; ¹H NMR (500 MHz, CDCl₃) δ 8.23 (s, 1H), 7.88-7.86 (d, *J =* 6.9 Hz, 1H), 7.66-7.65 (d, *J* = 1.1 Hz, 1H), 7.42-7.40 (dd, + 6.9, 1.7 Hz, 1H), 4.09-3.96 (m, 2H), 3.89-3.83 (m, 1H), 3.81-3.73 (m, 2H), 3.68-3.61 (m, 2H), 3.45-3.40 (m, 1H), 2.99 (s, 3H), 2.40-2.28 (m, 2H); ¹³C NMR (125 MHz, CD₃OD) δ 156.1, 151.4, 149.5, 146.9, 136.7, 133.7, 123.0, 109.9, 109.5, 57.3, 57.1, 50.0, 46.5, 45.0, 25.6; HPLC t_{R} = 7.9 min, 96.2%; ES-MS: (M + H) = 300 m/z.

### Example 9

### 2,2'-(3-Amino-6-(pyridin-4-yl)pyrazin-2-ylazanediyl)diethanol

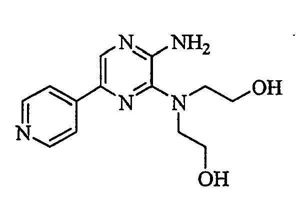

### Step A: 2,2'-(3-amino-6-bromopyrazin-2-ylazanediyl)diethanol

Prepared from 2-amino-3,5-dibromopyrazine and diethanolamine according to general procedure 4 (method 1) providing the diaminopyrazine (121 mg, 55%) as a yellow solid; ¹H NMR (500 MHz, CD₃OD) δ 7.51 (s, 1H), 3.72-3.78 (t, *J* = 5.3 Hz, 4H), 3.43-3.41 (t, *J*= 5.4 Hz, 4H); ES-MS: (M + H) = 277, 279 m/z.

**Step B:** Prepared from the product of Step A and 4-pyridylboronic acid according to general procedure 6 (method 1). Purification by column chromatography (12 g ISCO column eluting with methylene chloride and a 10:1 methanol/ammonium hydroxide mixture; gradient 100% methylene chloride to 95% then 90% and finally 85% methylene chloride) provided the title compound (40 mg, 37%) as a green solid; ¹H NMR (500 MHz, CD₃OD) δ 8.50-8.49 (dd, *J*= 4.7, 1.5 Hz, 2H), 8.19 (s, 1H), 7.93-7.92 (dd, *J*= 4.7, 1.6 Hz, 2H), 3.80-3.78 (t, *J*= 5.4 Hz, 4H), 3.53-3.51 (t, *J*= 5.4 Hz, 4H); ¹³C NMR (75 MHz, CD₃OD) δ 151.2, 150.7, 147.9, 146.7, 135.6, 133.2, 121.1, 60.6, 52.7; HPLC t_{R} = 6.40 min, 100% (Eluent 90:10 to 10:90 water/acetonitrile over 20 min then hold for 10 min); ES-MS: (M + H) = 276 m/z.

### Example 10

### 3-(Piperidin-1-yl)-5-(pyridin-4-yl)pyrazin-2-amine

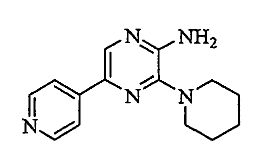

### Step A: 5-Bromo-3-(piperidin-1-yl)pyrazin-2-amine

Prepared from 2-amino-3,5-dibromopyrazine and piperidine according to general procedure 4 (method 2) providing the diaminopyrazine (199 mg, 97%) as a yellow solid; ¹H NMR (500 MHz, CDCl₃) δ 7.72 (s, 1H), 4.53 (s, 2H), 3.12-3.10 (t, *J* = 5.3 Hz, 4H), 1.71-1.67 (m, 4H), 1.65-1.61 (m, 2H).

**Step B:** Prepared from the product of Step A and 4-pyridylboronic acid according to general procedure 6 (method 2). Purification by trituration with methanol provided the title compound (89 mg, 45%) as a yellow-brown solid; ¹H NMR (500 MHz, DMSO-*d*₆) δ 8.56-8.54 (m, 2H), 8.36 (s, 1H), 7.89-7.87 (m, 2H), 6.35 (s, 2H), 3.13-3.11 (m, 4H), 1.71-1.70 (m, 4H), 1.59-1.58 (m, 2H); ¹³C NMR (75 MHz, DMSO-*d*₆) δ 149.6, 148.9, 145.47, 144.2, 133.3, 132.8, 118.4, 48.4, 24.7, 23.7; HPLC t_{R} = 11.1 min, >99%; ES-MS: (M + H) = 256 m/z.

### Example 11

### (1-(3-Amino-6-(pyridin-4-yl)pyrazin-2-yl)piperidin-4-yl)methanol

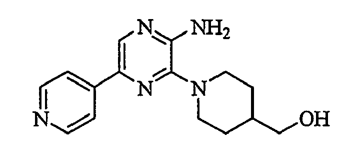

### Step A: (1-(3-Amino-6-bromopyrazin-2-yl)piperidin-4-yl)methanol

Prepared from 2-amino-3,5-dibromopyrazine and 4-hydroxymethylpiperidine according to general procedure 4 (method 2). Purification by trituration with methylene chloride/hexanes provided the diaminopyrazine (185 mg, 79%) as a light yellow solid; ¹H NMR (500 MHz, DMSO-*d*₆) δ 7.73 (s, 1H), 4.53 (s, 2H), 3.60-3.57 (m, 4H), 2.80-2.74 (m, 2H), 1.89-1.87 (d, *J* = 11.1 Hz, 2H), 1.72 (m, 1H), 1.40-1.37 (m, 2H), 1.33-1.31 (m, 1H).

**Step B:** Prepared from the product of Step A and 4-pyridylboronic acid according to general procedure 6 (method 2). Purification by trituration with methylene chloride/hexanes provided the title compound (102 mg, 56%) as a light yellow solid; ¹H NMR (500 MHz, DMSO-*d*₆) δ 8.55-8.54 (m, 2H), 8.36 (s, 1H), 7.89-7.87 (m, 2 H), 6.34 (s, 1H), 4.51-4.49 (t, *J* = 5.3 Hz, 1H), 3.63-3.60 (d, *J* = 12.5 Hz, 2H), 3.32-3.26 (m, 2H, partially masked by solvent), 2.72-2.67 (m, 2H), 1.78-1.75 (m, 2H), 1.57-1.56 (m, 1H), 1.45-1.39 (m, 2H); ¹³C NMR (75 MHz, DMSO-*d*₆) δ 149.9, 149.2, 145.6, 144.4, 133.6, 133.0, 118.7, 65.9, 47.8, 28.2 (one aliphatic signal masked by solvent); HPLC t_{R} = 9.16min, >99%; ES-MS: (M + H) = 286 m/z.

### Example 12

### 1-(3-Amino-6-(pyridin-4-yl)pyrazin-2-yl)piperidin-4-ol

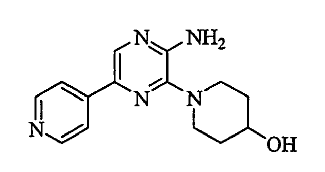

### Step A: 1-(3-Amino-6-bromopyrazin-2-yl)piperidin-4-ol

Prepared from 2-amino-3,5-dibromopyrazine and piperidin-4-ol according to general procedure 4 (method 2) providing the diaminopyrazine (185 mg, 79%) as a pale yellow solid; ¹H NMR (500 MHz, DMSO-*d*₆) 6 7.75 (s, 1H), 4.53 (s, 2H), 3.91-3.88 (m, 1H), 3.51-3.47 (m, 2H), 2.95-2.90 (m, 2H), 2.06-2.01 (m, 2H), 1.70-1.66 (m, 2H), 1.50-1.49 (m, 1H).

**Step B:** Prepared from the product of Step A and 4-pyridylboronic acid according to general procedure 6 (method 2). Purification by trituration with methylene chloride/hexanes provided the title compound (39 mg, 19%) as a pale yellow solid; ¹H NMR (300 MHz, DMSO-*d*₆) δ 8.56-8.54 (m, 2H), 8.37 (s, 1H), 7.89-7.88 (m, 2H), 6.39 (s, 2H), 4.71-4.70 (m, 1H), 3.68-3.65 (m, 1H), 3.50-3.46 (m, 2H), 2.90-2.83 (m, 2H), 1.90-1.86 (m, 2H), 1.68-1.62 (m, 2H); ¹³C NMR (75 MHz, DMSO-*d*₆) δ 149.9, 149.1, 145.4, 144.4, 133.6, 133.0, 118.7, 66.1, 45.5, 33.8; HPLC t_{R} = 8.48 min, 98%; ES-MS: (M + H) = 272 m/z.

### Example 13

### 3-(4-(Dimethylamino)piperidin-1-yl)-5-(pyridin-4-yl)pyrazin-2-amine

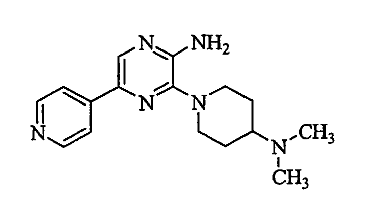

### Step A: 5-bromo-3-(4-(dimethylamino)piperidin-1-yl)pyrazin-2-amine

Prepared from 2-amino-3,5-dibromopyrazine and and *N*,*N*-dimethylpiperidin-4-amine according to general procedure 4 (method 2). Purification by column chromatography (12 g ISCO column eluting with methylene chloride and methanol; gradient 100% methylene chloride to 90% methylene chloride) provided the diaminopyrazine (203 mg, 83%) as a white solid; ¹H NMR (500 MHz, CDCl₃) δ 7.74 (s, 1H), 4.53 (s, 2H), 3.63-3.60 (d, *J* = 13.1 Hz, 2H), 2.78-2.72 (m, 2H), 2.33 (s, 6H), 2.28 (m, 1H), 1.99-1.97 (d, *J* = 12.6 Hz, 2H), 1.61-1.54 (m, 2H, partially masked by solvent).

**Step B:** Prepared from the product of Step A and 4-pyridylboronic acid according to general procedure 6 (method 2). Purification by column chromatography (12 g ISCO column eluting with methylene chloride and methanol; gradient 100% methylene chloride to 70% methylene chloride) followed by trituration with methylene chloride/hexanes provided the title compound (69 mg, 34%) as an off-white solid; ¹H NMR (300 MHz, DMSO-*d*₆) δ 8.56-8.54 (d, *J* = 5.9 hZ, 2H), 8.37 (s, 1H), 7.89-7.87 (d, *J* = 6.1 Hz, 2H), 6.41 (s, 2H), 3.65-3.61 (d, *J* = 12.2 Hz, 2H), 2.74-2.66 (t, *J* = 11.6 Hz, 2H), 2.25-2.21 (m, 7H), 1.86-1.82 (m, 2H), 1.72-1.61 (m, 2H); ¹³C NMR (75 MHz, DMSO-*d*₆) δ 150.1, 149.4, 145.5, 144.7, 133.8, 133.4, 118.9, 61.7, 47.7, 41.7, 27.8; HPLC t_{R} = 8.5 min, 98.1%; ES-MS: (M + H) = 299 m/z

### Example 14

### N,N-(1-Methylpiperidin-4-yl)-6-(pyridin-4-yl)pyrazine-2,3-diamine

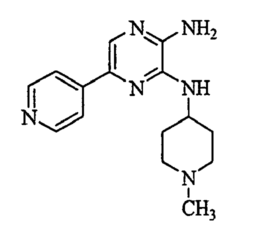

### Step A: 6-bromo-N²-(1-methylpiperidin-4-yl)pyrazine-2,3-diamine

Prepared from 2-amino-3,5-dibromopyrazine and *N*,*N*-dimethylpiperidin-4-amine according to general procedure 4 (method 2). Purification by trituration with ethyl acetate/hexanes provided the diaminopyrazine (127 mg, 83%) as an off-white solid; ¹H NMR (300 MHz, CDCl₃) δ 7.45 (s, 1H), 4.03 (br, 2H), 3.93-3.88 (m, 2H), 2.84-2.79 (m, 2H), 2.30 (s, 3H), 2.21-2.08 (m, 4H), 1.57-1.52 (m, 2H, partially masked by solvent).

**Step B:** Prepared from the product of Step A and 4-pyridylboronic acid according to general procedure 6 (method 2). Purification by passing through a plug of silica, eluting with methylene chloride and methanol; gradient 90% methylene chloride to 45% methylene chloride) followed by trituration with ethyl acetate/hexanes provided the title compound (59 mg, 46%) as a light brown solid; ¹H NMR (500 MHz, DMSO-*d*₆) δ 8.52-8.51 (d, *J* = 4.1 Hz, 2H), 7.95 (s, 1H), 7.82-7.81 (d, *J* = 4.1 Hz, 2H), 6.19-6.18 (m, 1H), 6.48 (s, 2H), 3.91-3.90 (m, 1H), 2.80-2.78 (d, *J* = 10.0 Hz, 2H), 2.20 (s, 3H), 2.08-2.00 (m, 4H), 1.54-1.50 (m, 2H); ¹³C NMR (75 MHz, DMSO-*d*₆) δ 150.0, 145.4, 145.0, 141.6, 133.2, 126.7, 118.8, 54.6, 47.6, 46.2, 31.7; HPLC t_{R} = 10.1 min, >99%; ES-MS: (M + H) = 285 m/z.

### Example 15

### N,N-(1-Methylpiperidin-3-yl)-6-(pyridin-4-yl)pyrazine-2,3-diamine

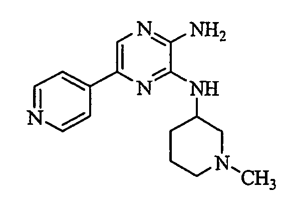

### Step A: 6-bromo-N²-(1-methylpiperidin-3-yl)pyrazine-2,3-diamine

Prepared from 2-amino-3,5-dibromopyrazine and 3-amino-1-methylpiperidine dihydrochloride according to general procedure 4 (method 2). Purification by column chromatography (12 g ISCO column eluting with methylene chloride and methanol; gradient 100% methylene chloride to 75% methylene chloride) provided the diaminopyrazine (85 mg, 25%) as a yellow-brown solid; ¹H NMR (300 MHz, CDCl₃) δ 7.60 (s, 1H), 5.25-5.22 (m, 1H), 4.40-4.30 (m, 3H), 2.69-2.67 (m, 2H), 2.47-2.44 (m, 1H), 2.29 (s +m, 4H), 2.16-2.14 (m, 1H), 1.63-1.54 (m, 3H).

**Step B:** Prepared from the product of Step A and 4-pyridylboronic acid according to general procedure 6 (method 2). Purification by column chromatography (40 g ISCO column eluting with methylene chloride and methanol; gradient 100% methylene chloride to 65% methylene chloride) provided the title compound (57 mg, 43%) as a brown solid; ¹H NMR (300 MHz, DMSO-*d*₆) δ 8.53-8.51 (d, *J* = 4.7 Hz, 2H), 7.97 (s, 1H), 7.84-7.82 (d, *J* = 5.1 Hz, 2H), 6.52 (s, 2H), 6.17-6.15 (d, *J* = 6.7 Hz, 1H), 4.19 (br, 1H), 2.99-2.95 (d, *J* = 8.1 Hz, 1H), 2.70-2.61 (m, 1H), 2.19 (s, 3H), 2.00-1.89 (m, 3H), 1.72-1.59 (m, 2H), 1.34-1.31 (m, 1H); ¹³C NMR (75 MHz, DMSO-*d*₆) δ 150.0, 145.0, 141.5, 126.8, 118.8, 60.6, 55.5, 47.1, 46.4, 29.6, 23.8 (two aromatic signals missing due to overlap); HPLC t_{R} = 10.9min, >99%; ES-MS: (M + H) = 285m/z.

### Example 16

### N,N-2-(Piperidin-4-yl)-6-(pryidin-4-yl)pyrazine-2,3-diamine

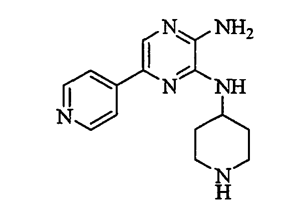

**Step A:** Ethyl 4-(3-amino-6-bromopyrazin-2-ylamino)piperidine-1-carboxylate Prepared from 2-amino-3,5-dibromopyrazine and ethyl-4-amino-1-piperidine-carboxylate according to general procedure 4 (method 2). Purification by trituration with methylene chloride provided the diaminopyrazine (140 mg, 100%) as an off white solid; ¹H NMR (300 MHz, DMSO-*d*₆) δ 7.17 (s, 1H), 6.32-6.30 (d, *J* = 7.1 Hz, 1H), 6.19 (br, 2H), 4.08-4.01 (m, 2H), 3.95-3.90 (m, 3H), 3.05-2.85 (m, 2H), 1.95-1.90 (m, 2H), 1.38-1.25 (m, 2H), 1.21-1.18 (m, 3H).

**Step B:** Prepared from the product of Step A and 4-pyridylboronic acid according to general procedure 6 (method 2). The protecting group was removed from the coupled product by heating at reflux with potassium hydroxide (18 equiv) in ethanol/water (1.5 mL, 6:2) for 26h. Purification by column chromatography (12 g ISCO column eluting with methylene chloride and methanol; gradient 100% methylene chloride to 85% methylene chloride) provided the title compound (19 mg, 18% over 2 steps) as a yellow solid; ¹H NMR (300 MHz, DMSO-*d*₆) δ 8.52-8.5 (d, *J* = 4.4 Hz, 2H), 7.95 (s, 1H), 7.84-7.82 (d, *J* = 4.5 Hz, 2H), 6.50 (s, 2H), 6.23-6.21 (d, *J* = 6.2 Hz, 1H), 4.03 (br, 2H), 3.07-3.02 (d, *J* = 12.0 Hz, 2H), 2.71-2.63 (t, *J* = 11.2 Hz, 2H), 2.02-1.99 (m, 2H), 1.42-1.38 (m, 2H); ¹³C NMR (75 MHz, DMSO-*d*₆) δ 149.7, 144.7, 141.1, 126.4, 118.5, 47.8, 44.8, 32.2 (two aromatic signals missing due to overlap); HPLC t_{R} = 10.6 min, >99%; ES-MS: (M + H) = 271 m/z.

### Example 17

### N,N-(Piperidin-3-yl)-6-(pyridin-4-yl)pyrazine-2,3-diamine

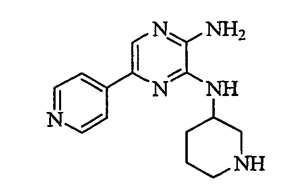

**Step A:** *tert*-Butyl 3-(3-amino-6-bromopyrazin-2-ylamino)piperidine-1-carboxylate Prepared from 2-amino-3,5-dibromopyrazine and 3-amino-1-boc-piperidine according to general procedure 4 (method 2). Purification by column chromatography (12 g ISCO column eluting with methylene chloride and methanol; gradient 100% methylene chloride to 85% methylene chloride) provided the diaminopyrazine (230 mg, 58%) as a brown solid; ¹H NMR (300 MHz, DMSO-*d*₆) δ 7.20 (s, 1H), 6.36-6.21 (m, 3H), 3.76-3.69 (m, 3H), 1.99-1.93 (m, 2H), 1.79-1.75 (m, 2H), 1.55-1.38 (m, 2H, partially masked by solvent), 1.29 (br, 9H).

**Step B**: Prepared from the product of Step A and 4-pyridylboronic acid according to general procedure 6 (method 2). Removal of the protecting group was achieved by dissolving the coupled product in 4 ml methanol, adding 2M HCl in diethyl ether (5 ml) at 0 °C, then stirring at room temperature for 24h. The reaction mixture was concentrated, diluted with water, neutralized with sodium bicarbonate and extracted with chloroform. The organics were washed with brine, dried over sodium sulfate and concentrated. Purification by column chromatography (12 g ISCO column eluting with methylene chloride and 10% ammonium hydroxide in methanol; gradient 100% methylene chloride to 50% methylene chloride) provided the title compound (107 mg, 67%) as a yellow solid; ¹H NMR (500 MHz, CD₃OD) δ 8.49-8.48, (d, *J* = 5.7 Hz, 2H), 7.98-7.97 (d, *J* = 5.7 Hz, 2H), 7.90 (s, 1H), 4.24-4.22 (m, 1H), 3.44-3.42 (d, *J* = 11.5 Hz, 1H), 3.02-3.00 (d, *J* = 12.4 Hz, 1H), 2.68-2.64 (t, *J* = 10.3 Hz, 1H), 2.54-2.50 (t, *J* = 10.5 Hz, 1H), 2.17-2.15 (m, 1H), 1.87-1.86 (m, 1H), 1.74-1.63 (m, 1H), 1.61-1.57 (m, 1H); ¹³C NMR (75 MHz, CD₃OD) δ 150.4, 148.4, 143.8, 136.0, 127.4, 121.1, 52.1, 31.9, 26.3 (two aromatic signals missing due to overlap; one aliphatic signal masked by solvent); HPLC t_{R} = 10.8min, >99%; ES-MS: (M + H) = 271m/z.

### Example 18

### N,N-Methyl-N,N-(1-methylpiperidin-4-yl)-6-(pyridin-4-yl)pyrazine-2,3-diamine

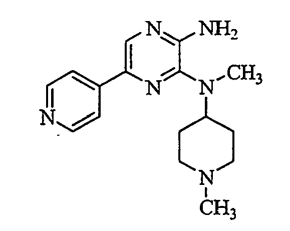

**Step A:** 6-bromo-*N*²-methyl-*N*²-(1-methylpiperidin-4-yl)pyrazine-2,3-diamine Prepared from 2-amino-3,5-dibromopyrazine and 1-methyl-4-(methylamino)piperidine according to general procedure 4 (method 2). Purification by column chromatography (12 g ISCO column eluting with methylene chloride and methanol; gradient 100% methylene chloride to 90% methylene chloride) provided the diaminopyrazine (130 mg, 51%) as a yellow solid; ¹H NMR (300 MHz, CDCl₃) δ 7.73 (s, 1H), 4.54 (s, 2H), 3.37 (m, 1H), 2.92-2.89 (m, 2H), 2.74 (s, 3H), 2.28 (s, 3H), 2.06-1.86 (m, 4H), 1.70-1.61 (m, 2H, partially masked by solvent).

**Step B:** Prepared from the product of Step A and 4-pyridylboronic acid according to general procedure 6 (method 2). Purification by column chromatography (12g ISCO column eluting with methylene chloride and methanol; gradient 100% methylene chloride to 85% methylene chloride) provided the title compound (90 mg, 61%) as a brown solid; ¹H NMR (300 MHz, DMSO-*d*₆) δ 8.56-8.54 (m, 2H), 8.37 (s, 1H), 7.88-7.86 (m, 2H), 6.39 (s, 2H), 3.52-3.50 (m, 2H), 2.96 (m, 2H), 2.71 (s, 3H), 2.31 (br, 4H), 1.79 (m, 4H); ¹³C NMR (75 MHz, DMSO-*d*₆) δ 150.2, 150.0, 145.6, 144.7, 133.8, 133.4, 118.9, 54.5, 54.2, 44.9, 32.5, 27.6; HPLC t_{R} = 11.5 min, >99%; ES-MS: (M + H) = 299 m/z

### Example 19

### 6-(Pyridin-4-yl)-N²-(pyrrolidin-3-yl)pyrazine-2,3-diamine

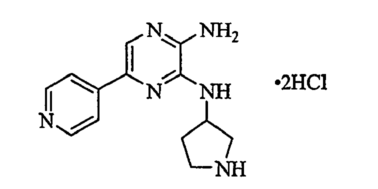

**Step A:** *tert*-Butyl 3-(3-amino-6-bromopyrazin-2-ylamino)pyrrolidinyl-1-carboxylate Prepared from 2-amino-3,5-dibromopyrazine and 3-amino-1-boc-pyrrolidine according to general procedure 4 (method 2). Purification by Combiflash chromatography (12 g ISCO column eluting with methylene chloride and methanol; gradient 100% methylene chloride to 85% methylene chloride) provided the diaminopyrazine (475 mg, 52%) as a yellow solid; ¹H NMR (500 MHz, CDCl₃/CD₃OD) δ 7.25 (s, 1H), 4.56-4.51 (m, 1H), 3.76-3.73 (dd, *J* = 6.4, 11.3 Hz, 1H), 3.52-3.43 (m, 2H), 3.28-3.22 (m, 1H), 2.30-2.22 (m, 1H), 1.97-1.95 (m, 1H), 1.47 (s, 9H).

**Step B:** Prepared from the product of Step A and 4-pyridylboronic acid according to general procedure 6 (method 2). Purification by Combiflash chromatography (12g ISCO column eluting with methylene chloride and 10% ammonium hydroxide in methanol; gradient 100% methylene chloride to 80% methylene chloride) provided the title compound (114 mg, 63%) as an orange oil; ¹H NMR (500 MHz, CDCl₃) δ 8.63-8.61 (d, *J* = 6.0 Hz, 2H), 8.01 (s, 1H), 7.80-7.78 (d, *J* = 6.0 Hz, 2H), 4.89-4.69 (m, 1H), 3.77-3.66 (m, 1H), 3.48-3.38 (m, 3H), 2.28-2.77 (m, 1H), 2.06-2.02 (m, 1H), 1.48 (s, 9H)..

**Step C:** Prepared from Step B was stirred in TFA (2 mL) for 2 h and the reaction mixture concentrated and partitioned between methylene chloride and saturated sodium carbonate solution. The organic layer was removed, dried over sodium sulfate and concentrated to provide a yellow oil. Purification by Combiflash chromatography (12g ISCO column eluting with methylene chloride and /10% ammonium hydroxide in methanol; gradient 100% methylene chloride to 80% methylene chloride) followed by conversion to the bis-HCl salt provided the title compound (69 mg, 65%) as a yellow solid; ¹H NMR (500 MHz, CD₃OD) δ 8.61-8.59 (d, *J* = 6.7 Hz, 2H), 8.34-8.32 (d, *J* = 6.7 Hz, 2H), 8.10 (s, 1H), 4.81-4.78 (m, 1H), 3.80-3.76 (dd, *J* = 6.2, 12.2 Hz, 1H), 3.62-3.57 (m, 1H), 3.52-3.46 (m, 1H), 3.45-3.41 (m, 1H), 2.54-2.47 (m, 1H), 2.30-2.24 (m, 1H); ¹³C NMR (125 MHz, CD₃OD) δ 153.5, 148.0, 144.7, 143.4, 133.13, 131.9, 121.6, 52.26, 51.86, 31.11, 25.29; HPLC t_{R} = 7.21 min, >99%; ES-MS: (M+H) = 257m/z.

### Example 20

### 3-(1-Methylpiperidin-4-yloxy)-5-(pyridin-4-yl)pyrazin-2-amine

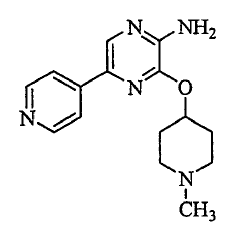

### Step A: 5-Bromo-3-(1-methylpiperidin-4-yloxy)pyrazin-2-amine:

Prepared from 2-amino-3,5-dibromopyrazine and 1-methyl-4-hydroxoypiperidine according to general procedure 5. Purification by column chromatography (12 g ISCO column eluting with methylene chloride and methanol/concentrated ammonium hydroxide (10:1); gradient 100% methylene chloride to 90% methylene chloride) provided the alkoxypyrazine (0.21 g, 64%) as a tan solid; ¹H NMR (300 MHz, CDCl₃) δ 7.61 (s, 1H), 5.11-5.07 (m, 1H), 4.75 (br s, 2H), 2.76-2.65 (m, 2H), 2.36-2.31 (m, 2H), 2.31 (s, 3H), 2.06-2.05 (m, 2H), 1.90-1.79 (m, 2H); ES-MS: (M + H) = 288 m/z.

**Step B:** Prepared from the product of Step A and 4-pyridylboronic acid according to general procedure 6 (method 2) and purified by column chromatography (12 g ISCO column eluting with methylene chloride and methanol/concentrated ammonium hydroxide (10:1); gradient 100% methylene chloride to 90% methylene chloride) to provide the title compound (0.11 g, 54%) as a light brown solid; ¹H NMR (500 MHz, DMSO-*d*₆) δ 8.55-8.54 (dd, *J* = 4.6, 1.5 Hz, 2H), 8.28 (s, 1H), 7.84-7.83 (dd, *J* = 4.6, 1.5 Hz, 2H), 6.75 (br s, 2H), 5.17-5.12 (m, 1H), 2.69-2.63 (m, 2H), 2.25-2.17 (m, 2H), 2.20 (s, 3H), 2.07-2.01 (m, 2H), 1.81-1.75 (m, 2H); ¹³C NMR (125 MHz, CD₃OD) δ 149.9, 147.0, 145.9, 144.1, 131.6, 131.5, 118.5, 70.9, 52.3, 45.8, 30.0; HPLC t_{R} = 7.36 min, >99%; ES-MS: (M + H) = 286 m/z.

### Example 21

### 3-(1-Methylpiperidin-3-yloxy)-5-(pyridin-4-yl)pyrazin-2-amine

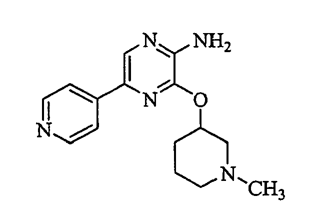

### Step A: 5-Bromo-3-(1-methylpiperidin-3-yloxy)pyrazin-2-amine:

Prepared from 2-amino-3,5-dibromopyrazine and 1-methyl-3-hydroxoypiperidine according to general procedure 5 and used in the next step without purification.

**Step B:** Prepared from the product of Step A and 4-pyridylboronic acid according to general procedure 6 (method 2) and purified by column chromatography (12 g ISCO column eluting with methylene chloride and methanol/concentrated ammonium hydroxide (10:1); gradient 100% methylene chloride to 90% methylene chloride) to provide the title compound (0.16 g, 57%) as light brown solid; ¹H NMR (500 MHz, DMSO-*d*₆) δ 8.56-8.54 (dd, *J* = 6.1, 1.3 Hz, 2H), 8.28 (s, 1H), 7.84-7.83 (dd, *J* = 6.2, 1.4 Hz, 2H), 6.70 (br s, 2H), 5.23-5.19 (m, 1H), 2.88-2.86 (d, *J* = 9.1 Hz, 1H), 2.50 (m, 1H, masked by solvent), 2.32-2.26 (t, *J* = 8.9 Hz, 1H), 2.19 (s, 3H), 2.14-2.11 (t, *J* = 8.6 Hz, 1H), 1.99-1.97 (m, 1H), 1.83-1.81 (dd, *J* = 9.5, 3.2 Hz, 1H), 1.63-1.50 (m, 2H); ¹³C NMR (125 MHz, CD₃OD) δ 150.0, 147.1, 145.9, 144.1, 131.8, 131.5, 118.6, 70.5, 58.7, 54.9, 46.1, 28.5, 22.3; HPLC t_{R} = 7.54 min, 97.9%; ES-MS: (M + H) = 286 m/z.

### Example 22

### 3-(Piperidin-4-yloxy)-5-(pyridin-4-yl)pyrazin-2-amine

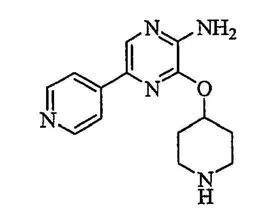

### Step A: tert-Butyl 4-(3-amino-6-bromopyrazin-2-yloxy)piperidine-1-carboxylate

Prepared from 2-amino-3,5-dibromopyrazine and *tert*-butyl 4-hydroxypiperidine-1-carboxylate according to general procedure 5 and used in the next step without purification.

**Step B:** *tert*-Butyl 4-(3-amino-6-(pyridin-4-yl)pyrazin-2-yloxy)piperidine-1-carboxylate Prepared from the product of Step A and 4-pyridylboronic acid according to general procedure 6 (method 2) and purified by column chromatography (12 g ISCO column eluting with methylene chloride and methanol/concentrated ammonium hydroxide (10:1); gradient 100% methylene chloride to 90% methylene chloride) to provide the coupled product (0.24 g, 66%) as a yellow solid; ¹H NMR (500 MHz, CDCl₃) δ 8.63-8.62 (d, *J* = 5.9 Hz, 2H), 8.15 (s, 1H), 7.73-7.72 (d, *J* = 5.9 Hz, 2H), 5.42-5.38 (m, 1H), 4.99 (s, 2H), 3.85-3.79 (m, 2H), 3.38-3.33 (m, 2H), 2.11-2.05 (m, 2H), 1.84-1.82 (m, 2H), 1.48 (s, 9H).

**Step C:** To a stirred solution of the product from Step B (0.24 g, 0.66 mmol) in methanol (4 mL) at room temperature was added 2 M HCl in diethyl ether (2.0 mL). The reaction mixture was stirred overnight and concentrated. The residue was purified by column chromatography (12 g ISCO column eluting with methylene chloride and methanol/concentrated ammonium hydroxide (10:1); gradient 100% methylene chloride to 90% methylene chloride) to provide the title compound (0.12 g, 68%) as an off-white solid; ¹H NMR (500 MHz, DMSO-*d*₆) δ 8.55-8.54 (dd, *J* = 4.6, 1.6 Hz, 2H), 8.27 (s, 1H), 7.84-7.83 (dd, *J*= 4.6, 1.6 Hz, 2H), 6.68 (s, 2H), 5.25-5.20 (m, 1H), 3.10-2.89 (m, 3H), 2.69-2.64 (m, 2H), 2.02-1.98 (m, 2H), 1.66-1.60 (m, 2H); ¹³C NMR (125 MHz, DMSO-*d*₆) δ 149.9, 147.1, 145.8, 144.1, 131.6, 131.5, 118.5, 72.0, 43.3, 31.6; HPLC t_{R} = 7.41 min, 96.8%; ES-MS: (M + H) = 272 m/z.

### Example 23

### 3-(Piperidin-3-yloxy)-5-(pyridin-4-yl)pyrazin-2-amine

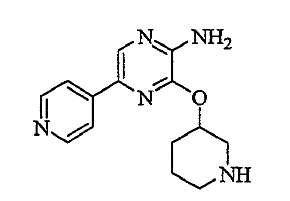

### Step A: tert-Butyl 3-(3-amino-6-bromopyrazin-2-yloxy)piperidine-1-carboxylate

Prepared from 2-amino-3,5-dibromopyrazine and *tert*-butyl 3-hydroxypiperidine-1-carboxylate according to general procedure 5 and used in the next step without purification.

**Step B**: *tert*-Butyl 3-(3-amino-6-(pyridin-4-yl)pyrazin-2-yloxy)piperidine-1-carboxylate Prepared from the product of Step A and 4-pyridylboronic acid according to general procedure 6 (method 2) and purified by column chromatography (12 g ISCO column eluting with methylene chloride and methanol/concentrated ammonium hydroxide (10:1); gradient 100% methylene chloride to 90% methylene chloride) to provide the coupled product (0.21 g, 56%) as a yellow solid; ¹H NMR (500 MHz, CDCl₃) δ 8.63-8.61 (d, *J* = 5.9 Hz, 2H), 8.15 (s, 1H), 7.76-7.75 (d, *J* = 5.9 Hz, 2H), 5.25-5.22 (m, 1H), 5.00 (s, 2H), 4.18-3.06 (m, 5H), 2.06-1.73 (m, 3H), 1.26 (s, 9H).

**Step C**: Prepared from the product of Step B in a similar manner to that described for Step C (example 22) and purified by column chromatography (12 g ISCO column eluting with methylene chloride and methanol/concentrated ammonium hydroxide (10:1); gradient 100% methylene chloride to 90% methylene chloride) to provide the title compound (0.11 g, 70%) as an off-white solid; ¹H NMR (500 MHz, DMSO-*d*₆) δ 8.55-8.54 (dd, *J* = 4.6, 1.5 Hz, 2H), 8.27 (s, 1H), 7.84-7.83 (dd, *J* = 4.6, 1.5 Hz, 2H), 6.79 (s, 2H), 5.11-5.07 (m, 1H), 3.30 (m, 1H, masked by solvent), 3.08-3.05 (dd, *J* = 12.7, 2.6 Hz, 1H), 2.77-2.62 (m, 3H), 2.00-1.96 (m, 1H), 1.83-1.77 (m, 1H), 1.74-1.65 (m, 1H), 1.47-1.37 (m, 1H); ¹³C NMR (125 MHz, DMSO-*d*₆) δ 149.9, 147.2, 145.9, 144.2, 131.6, 131.4, 118.5, 70.4, 49.7, 45.5, 28.8, 23.5; HPLC t_{R} = 7.45 min, 97.1%; ES-MS: (M + H) = 272 m/z.

### Example 24

### 3-(4-(3-Amino-6-(pyridin-4-yl)pyrazin-2-yl)piperazin-1-yl)propan-1-ol

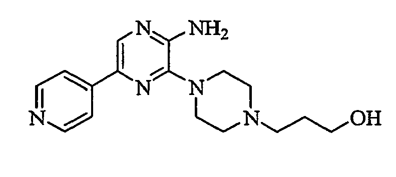

### Step A: tert-Butyl 4-(3-amino-6-bromopyrazin-2-yl)piperazine-1-carboxylate

Prepared from 2-amino-3,5-dibromopyrazine and *tert*-butylpiperazine-1-carboxylate according to general procedure 4 (method 2). Purification by column chromatography (12 g ISCO column eluting with hexanes and ethyl acetate; gradient 100% hexanes to 70% hexanes) followed by trituration with ethyl acetate/hexanes provided the diaminopyrazine (406 mg, 57%) as a pale yellow solid; ¹H NMR (300 MHz, CDCl₃) δ 7.79 (s, 1H), 4.55 (s, 2H), 3.58-3.55 (t, *J* = 5.1 Hz, 4H), 3.16-3.1.3 (t, *J*= 5.1 Hz, 4H), 1.48 (s, 9H).

### Step B: tert-Butyl 4-(3-amino-6-(pyridin-4-yl)pyrazin-2-yl)piperazine-1-carboxylate

Prepared from the product of Step A and 4-pyridylboronic acid according to general procedure 6 (method 2). Purification by trituration with ethyl acetate/hexanes provided the coupled product (394 mg, 97%) as a light brown solid; ¹H NMR (300 MHz, CDCl₃) δ 8.65-8.63 (m, 2H), 8.29 (s, 1H), 7.81-7.79 (m, 2H), 4.81 (s, 2H), 3.64-3.61 (m, 4H), 3.26-3.23 (m, 4H), 1.50 (s, 9H).

### Step C: 3-(Piperazin-1-yl)-5-(pyridin-4-yl)pyrazin-2-amine trifluoroacetate

Trifluororacetic acid (2.5 mL, 0.324 mmol) was added to a cooled mixture (0 °C) of the product from Step B (393 mg, 1.10 mmol) in methylene chloride (5 mL). The mixture was stirred at room temperature for 20h. Concentration of the reaction mixture provided the de-protected piperazine (879 mg, quant) as a green oil; ¹H NMR (300 MHz, CD₃OD) δ 8.74-8.72 (m, 3H), 8.57-8.55 (m, 2H), 3.56-3.52 (m, 8H).

**Step D**: The product from step C (879 mg, 2.37 mmol), 3-chloropropanol (224 mg, 2.37 mmol), potassium iodide (788 mg, 4.75 mmol) and potassium carbonate (656 mg, 4.75 mmol) in 10 mL of acetonitrile were refluxed for 15h. Upon cooling the mixture was poured into saturated sodium bicarbonate, extracted with ethyl acetate and the organics were washed with 0.1N NaS₂O₃, dried over sodium sulfate and concentrated. Purification by trituration with methanol/hexanes provided the title compound (52 mg, 15%) as a pale yellow solid; ¹H NMR (300 MHz, DMSO-*d*₆) δ 8.56-8.54 (d, *J*= 5.7 Hz, 2H), 8.38 (s, 1H), 7.90-7.88 (d, *J* = 5.7 Hz, 2H), 6.41 (s, 2H), 4.51 (br, 1H), 3.49-3.45 (t, *J* = 6.1 Hz, 2H), 3.18 (br, 4H), 2.59 (br, 4H), 2.44-2.39 (t, *J* = 7.0 Hz, 2H), 1.65-1.60 (m, 2H); ¹³C NMR (75 MHz, DMSO-*d*₆) δ 150.2, 149.3, 145.2, 144.7, 133.9, 133.5, 119.0, 59.7, 55.5, 52.7, 47.8, 29.8; HPLC t_{R} = 11.6 min, 98.5%; ES-MS: (M + H) = 315 m/z.

### Example 25

### N-Methyl-3-(4-methyl-1,4-diazepan-1-yl)-5-(pyridin-4-yl)pyrazin-2-amine hydrochloride

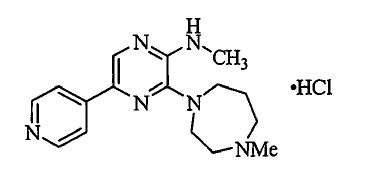

### Step A: N-Methylpyrazin-2-amine

Prepared from 2-chloropyrazine and diethanolamine according to general procedure 2 providing the aminopyrazine (700 mg, quant.) as an oil; ¹H NMR (300 MHz, CDCl₃) δ 7.80-7.99 (dd, *J* = 2.7, 1.5 Hz, 1H), 7.90-7.89 (d, *J* = 1.5 Hz, 1H), 7.79-7.80 (d, *J* = 2.8 Hz, 1H), 4.78 (br s, 1H), 2.80-2.79 (d, *J*= 4.9 Hz, 3H).

### Step B: 3,5-Dibromo-N-methylpyrazin-2-amine

Prepared from the product of Step A according to general procedure 3 providing the dibromopyrazine (700 mg, 44%) as a yellow solid; ¹H NMR (300 MHz, CDCl₃) δ 8.07 (s, 1H), 5.26 (br s, 1H), 3.03-3.01 (d, *J*= 5.0 Hz, 3H).

### Step C: 5-Bromo-N-methyl-3-(4-methyl-1,4-diazepan-1-yl)pyrazin-2-amine

Prepared from the product of Step B and 1-methylhomopiperazine according to general procedure 4 (method 1) providing the diaminopyrazine (374 mg, 95%) as a golden oil, which contained some unreacted 1-methylhomopiperazine; ¹H NMR (300 MHz, CDCl₃) δ 7.74 (s, 1H), 4.82 (br s, 1H), 3.43-3.37 (m, 2H), 2.98-2.92 (m, 2H), 2.73-2.71 (m, 2H), 2.68-2.57 (m, 2H), 2.42 (s, 3H), 2.38 (s, 3H), 1.97-1.90 (m, 1H), 1.84-1.76 (m, 1H).

**Step D**: Prepared from the product of Step C and 4-pyridylboronic acid according to general procedure 6 (method 1). Purification by column chromatography (12 g ISCO column eluting with methylene chloride and a 10:1 methanol/ammonium hydroxide mixture; gradient 100% methylene chloride to 95% then 90% and finally 85% methylene chloride) provided the free base of the title compound as an oil. This was converted to the HCl salt (2N HCl in ether, 1 equiv.) providing the salt (325 mg, 78%) as a yellow solid; ¹H NMR (500 MHz, *d*₆-DMSO) δ 10.76 (br s, 1H), 8.62-8.61 (d, *J* = 6.2 Hz, 2H), 8.57 (s, 1H), 8.03-8.01 (d, *J*= 6.3 Hz, 2H), 6.94-6.93 (m, 2H), 3.79-3.75 (m, 1H), 3.67-3.64 (m, 1H), 3.52-3.39 (m, 3H), 3.38-3.35 (m, 3H), 2.91-2.90 (d, *J* = 4.6 Hz, 3H), 2.30-2.27 (m, 1H), 2.10-2.08 (m, 1H); ¹³C NMR (75 MHz, *d*₆-DMSO) δ 148.8, 148.0, 145.9, 145.7, 134.4, 131.5, 119.0, 55.4, 54.7, 49.2, 45.3, 43.5, 28.1, 24.0; HPLC t_{R} = 7.80 min, 99.0%; ES-MS: (M + H) = 299 m/z.

### Example 26

### N²-Methyl-N³-(piperidin-3-yl)-5-(pyridin-4-yl)pyrazine-2,3-diamine hydrochloride

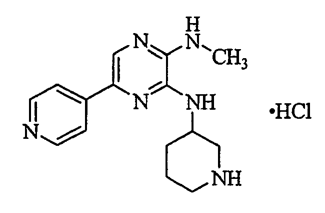

### Step A: tert-Butyl 3-(6-bromo-3-(methylamino)pyrazin-2-ylamino)piperidine-1-carboxylate

Prepared from the product of Step B (example 25) and 3-amino-1-boc-piperazine according to general procedure 4 (method 2). Purification by column chromatography (12 g ISCO column eluting with hexanes and ethyl acetate; gradient 100% hexanes to 80% hexanes) provided the diaminopyrazine (188 mg, 37%) as an off-white foamy solid; ¹H NMR (500 MHz, CDCl₃) δ 7.54 (s, 1H), 4.08-4.02 (m, 2H), 3.60-3.27 (m, 4H), 2.94-2.93 (d, *J* = 3.9 Hz, 3H), 1.91-1.86 (m, 3H), 1.59-1.57 (m, 2H), 1.41 (s, 9H).

### Step B: tert-Butyl 3-(3-(methylamino)-6-(pyridin-4-yl)pyrazin-2-ylamino)piperidine-1-carboxylate

Prepared from the product of Step A and 4-pyridylboronic acid according to general procedure 6 (method 1). Purification by column chromatography (12 g ISCO column eluting with hexanes and ethyl acetate; gradient 100% hexanes to 0% hexanes) provided the coupled product (75 mg, 41%) as a yellow solid; ¹H NMR (300 MHz, CDCl₃) δ 8.61-8.60 (dd, *J*= 4.6, 1.6 Hz, 2H), 8.11 (s, 1H), 7.83-7.81 (dd, *J*= 4.7, 1.6 Hz, 2H), 4.44-4.42 (m, 1H), 4.21-4.20 (m, 1H), 3.73-3.62 (m, 1H), 3.61-3.51 (m, 2H), 3.41-3.35 (m, 1H), 3.05-3.03 (d = 4.6 Hz, 3H), 3.02-3.01 (m, 1H), 1.95-1.94 (m, 2H), 1.77-1.76 (m, 2H), 1.26 (s, 9H).

**Step C**: The product from Step B (75 mg, 0.195 mmol) was dissolved in methanol (3 ml) and 2 N HCl in ether (10 ml) was added. The mixture was allowed to stir for 3 h, after which time a yellow precipitate had formed. The mixture was concentrated, dissolved in 10% ammonium hydroxide in methanol solution (5 ml) and reconcentrated. Purification by column chromatography (4 g ISCO column eluting with methylene chloride and a 10:1 methanol/ammonium hydroxide mixture; gradient 100% methylene chloride to 90% then 85% methylene chloride) provided the free base of the title compound as an oil. This was converted to the HCl salt (2N HCl in ether, 1 equiv.) providing the salt (50 mg, 80%) as an orange solid; ¹H NMR (500 MHz, CD₃OD) δ 8.74-8.72 (d, *J*= 7.0 Hz, 2H), 8.67-8.66 (d, *J*= 7.0 Hz, 2H), 8.35 (s, 1H), 4.68-4.64 (m, 1H), 3.73-3.70 (dd, *J* =12.3, 3.6 Hz, 1H), 3.41-3.38 (m, 1H), 3.16 (s, 3H), 3.14-3.09 (m, 1H), 3.05-2.99 (m, 1H), 2.22-2.15 (m, 2H), 2.05-1.99 (m, 1H), 1.93-1.87 (m, 1H); ¹³C NMR (75 MHz, CD₃OD) δ 155.5, 145.6, 144.3, 142.3, 131.3, 122.8, 48.1, 47.2, 45.0, 29.1, 28.9, 22.1 (one aromatic signal missing due to overlap); HPLC t_{R} = 11.40 min, 97.7%; ES-MS: (M + H) = 285 m/z.

### Example 27

### N-(2-Methoxyethyl)-3-(4-methyl-1,4-diazepan-1-yl)-5-(pyridin-4-yl)pyrazin-2-amine hydrochloride

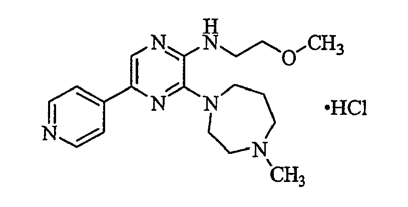

### Step A: N-(2-Methoxyethyl)pyrazin-2-amine

Step A: Prepared from 2-chloropyrazine and 1-methoxyethylamine according to general procedure 2 providing the aminopyrazine (490 mg, 52%) as an oil; ¹H NMR (300 MHz, CDCl₃) δ 7.98-7.97 (dd, *J* = 2.6, 1.5 Hz, 1H), 7.91-7.90 (d, *J* = 1.4 Hz, 1H), 7.80-7.79 (d, *J* = 2.7 Hz, 1H), 4.94 (br s, 1H), 3.60-3.55 (m, 4H), 3.40 (s, 3H).

### Step B: 3,5-Dibromo-N-(2-methoxyethyl)pyrazin-2-amine

Prepared from the product of Step A according to general procedure 3 providing the dibromopyrazine (435 mg, 44%) as an off-white solid; ¹H NMR (300 MHz, CDCl₃) δ 8.03 (s, 1H), 5.58 (br s, 1H), 3.63-3.56 (m, 4H), 3.41 (s, 3H).

### Step C: 5-Bromo-N-(2-methoxyethyl)-3-(4-methyl-1,4-diazepan-1-yl)pyrazin-2-amine

Prepared from the product of Step B and 1-methylhomopiperazine according to general procedure 4 (method 1) providing the diaminopyrazine (221 mg, quant.) as an oil; ¹H NMR (500 MHz, CDCl₃) δ 7.68 (s, 1H), 5.08 (br s, 1H), 3.61-3.59 (m, 2H), 3.56-3.53 (m, 2H), 3.44-3.40 (m, 4H), 3.39 (s, 3H), 2.73-2.70 (m, 4H), 2.42 (s, 3H), 1.99-1.94 (m, 2H).

**Step D**: Prepared from the product of Step C and 4-pyridylboronic acid according to general procedure 6 (method 1). Purification by column chromatography (12 g ISCO column eluting with methylene chloride and a 10:1 methanol/ammonium hydroxide mixture; gradient 100% methylene chloride to 90% and finally 80% methylene chloride) provided the free base of the title compound as an oil. This was converted to the HCl salt (2N HCl in ether, 1 equiv.) providing the salt (170 mg, 70%) as a yellow solid, that darkened on standing: ¹H NMR (500 MHz, CD₃OD) δ 8.63-8.61 (s+d, 3H), 8.32-8.31 (d, *J*= 6.7 Hz, 2H), 3.82-3.54 (4xm, 12H), 3.40 (s, 3H), 3.01 (s, 3H), 2.29-2.28 (m, 2H); ¹³C NMR (75 MHz, CD₃OD) δ 149.8, 149.0, 146.4, 144.9, 136.2, 130.9, 120.5, 70.3, 58.3, 55.6, 55.2, 55.0, 50.0, 45.8, 44.0, 24.4; HPLC t_{R} = 8.33 min, 97.6%; ES-MS: (M + H) = 343 m/z.

### Example 28

### 2-(3-(4-Methyl-1,4-diazepan-1-yl)-5-(pyridin-4-yl)pyrazin-2-ylamino)ethanol hydrochloride

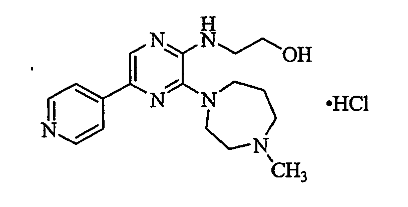

### Step A: 2-(Pyrazin-2-ylamino)ethanol

Prepared from 2-chloropyrazine and ethanolamine according to general procedure 2 providing the aminopyrazine (800 mg, 94%) as an oil; ES-MS: (M + H) = 140 m/z.

### Step B: 2-(3,5-dibromopyrazin-2-ylamino)ethanol

Prepared from the product of Step A according to general procedure 3 except the reaction mixture was partitioned between ethyl acetate and water. The organic phase was then dried over Na₂SO₄ and concentrated providing the dibromopyrazine (1.7 g, quant) as an oil; ¹H NMR (300 MHz, CDCl₃) δ 8.02 (s, 1H), 5.69 (br s, 1H), 3.86-3.83 (t, *J*= 5.2 Hz, 2H), 3.64-3.61 (t, *J* = 5.3 Hz, 2H), 3.00 (s, 1H).

### Step C: 2-(5-Bromo-3-(4-methyl-1,4-diazepan-1-yl)pyrazin-2-ylamino)ethanol

Prepared from the product of Step B and 1-methylhomopiperazine according to general procedure 4 (method 1) providing the diaminopyrazine (339 mg, 61%) as a dark oil; ¹H NMR (500 MHz, CDCl₃) δ 7.67 (s, 1H), 5.31 (br s, 1H), 3.85-3.83 (t, *J* = 5.1 Hz, 2H), 3.57-3.55 (t, *J* = 5.2 Hz, 2H), 3.44-3.40 (m, 4H), 2.74-2.69 (m, 4H), 2.42 (s, 3H), 1.97-1.92 (m, 2H).

**Step D**: Prepared from the product of Step C and 4-pyridylboronic acid according to general procedure 6 (method 1). Purification by column chromatography (12 g ISCO column eluting with methylene chloride and a 10:1 methanol/ammonium hydroxide mixture; gradient 100% methylene chloride to 95% then 90% and finally 85% methylene chloride) provided the free base of the title compound as an oil. This was converted to the HCl salt (2N HCl in ether, 1 equiv.) providing the salt (209 mg, 56%) as a yellow solid; ¹H NMR (500 MHz, CD₃OD) δ 8.58 (br d, 2H), 8.52 (s, 1H), 8.17-8.16 (d, *J* = 3.7 Hz, 2H), 3.82-3.80 (m, 4H), 3.65-3.48 (m, 8H), 3.01 (s, 3H), 2.31-2.29 (m, 2H); ¹³C NMR (75 MHz, CD₃OD) δ 150.9, 148.1, 148.8, 147.7, 137.1, 133.9, 121.6, 61.9, 58.4, 57.4, 51.8, 47.9, 45.7, 45.0, 26.7; HPLC t_{R} = 7.40 min, 95.5%; ES-MS: (M + H) = 329 m/z.

### Example 29

### N-(2-Methoxyethyl)-N-methyl-3-(4-methyl-1,4-diazepan-1-yl)-5-(pyridin-4-yl)pyrazin-2-amine hydrochloride

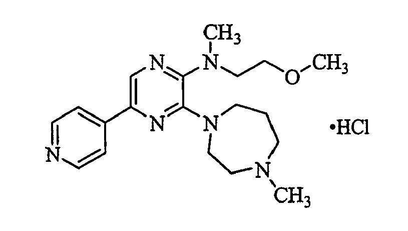

### Step A: 3,5-Dibromo-N-(2-methoxyethyl)-N-methylpyrazin-2-amine

Sodium hydride (60% suspension in mineral oil, 35 mg, 0.89 mmol) was added to a solution of the product of Step B (example 15) (230 mg, 0.74 mmol) in tetrahydrofuran (2 ml) under nitrogen at room temperature. After stirring for 5 min, methyl iodide (115 mg, 0.81 mmol) was added and the mixture held for 1 h. LC-MS analysis showed starting material still remained, so a further aliquot of sodium hydride followed by methyl iodide was added. After 30 min no starting material remained. The mixture was quenched with water (50 ml) and extracted with ethyl acetate (2 x 50 ml). The combined organic layers were dried over sodium sulfate and concentrated providing the substituted aminopyrazine (233 mg, 97%) as an oil; ¹H NMR (500 MHz, CDCl₃) δ 8.09 (s, 1H), 3.70-3.68 (m, 2H), 3.65-3.62 (m, 2H), 3.33 (s, 3H), 3.12 (s, 3H).

### Step B: 5-Bromo-N-(2-methoxyethyl)-N-methyl-3-(4-methyl-1,4-diazepan-1-yl)pyrazin-2-amine

Prepared from the product of Step A and 1-methylhomopiperazine according to general procedure 4 (method 1). Purification by column chromatography (12 g ISCO column eluting with methylene chloride and a 10:1 methanol/ammonium hydroxide mixture; gradient 100% methylene chloride to 90% methylene chloride) provided the diaminopyrazine (101 mg, 40%) as a clear oil; ¹H NMR (300 MHz, CDCl₃) δ 7.63 (s, 1H), 3.80-3.76 (t, *J* = 4.7 Hz, 2H), 3.69-3.65 (t, *J* = 6.0 Hz, 2H), 3.47-3.43 (m, 4H), 3.25 (s, 3H), 2.79 (s, 3H), 2.62-2.59 (t, *J*= 4.7 Hz, 2H), 2.56-2.53 (t, *J* = 5.6 Hz, 2H), 2.40 (s, 3H), 1.95-1.92 (m, 2H).

**Step C**: Prepared from the product of Step B and 4-pyridylboronic acid according to general procedure 6 (method 1). Purification by column chromatography (12 g ISCO column eluting with methylene chloride and a 10:1 methanol/ammonium hydroxide mixture; gradient 100% methylene chloride to 95% then 90% and finally 85% methylene chloride) provided the free base of the title compound as an oil. This was converted to the HCl salt (2N HCl in ether, 1 equiv.) providing the salt (96 mg, 86%) as an orange solid, that darkened on standing: ¹H NMR (500 MHz, CD₃OD) δ 8.68 (s, 2H), 8.58 (s, 1H), 8.41-8.40 (d, *J* = 5.4 Hz, 2H), 4.07-3.87 (m, 2H), 3.82-3.80 (m, 4H), 3.67-3.54 (m, 6H), 3.19 (s, 3H), 3.07 (s, 3H), 2.95 (s, 3H), 2.27-2.23 (m, 2H); ¹³C NMR (75 MHz, CD₃OD) δ 152.7, 150.9, 147.0, 145.1, 135.3, 134.2, 122.3, 70.5, 58.9, 57.7, 57.6, 51.0, 45.6, 45.2, 37.8, 25.8 (one aliphatic signal masked by solvent); HPLC t_{R} = 8.65 min, 96.0%; ES-MS: (M + H) = 357 m/z.

### Example 30

### 2-(4-(3-(Dimethylamino)-6-(pyridin-4-yl)pyrazin-2-yl)piperazin-1-yl)ethanol hydrochloride

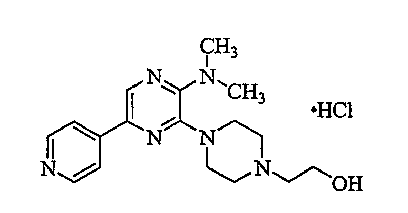

### Step A: 3,5-Dibromo-N,N-dimethylpyrazin-2-amine

Prepared from 2-amino-3,5-dibromopyrazine and iodomethane (568 mg, 4.00 mmol) according to general procedure 1. Purification by column chromatography (12 g ISCO column eluting with hexanes and ethyl acetate; gradient 100% hexanes to 70% hexanes) provided the substituted aminopyrazine (171 mg, 61%) as a yellow oil; ¹H NMR (300 MHz, CDCl₃) δ 8.11 (s, 1H), 3.07 (s, 6H).

**Step B**: 2-(4-(6-Bromo-3-(dimethylamino)pyrazin-2-yl)piperazin-1-yl)ethanol Prepared from the product of Step A and 2-(piperazin-1-yl)ethanol according to general procedure 4 (method 2). Purification by column chromatography (12 g ISCO column eluting with methylene chloride and methanol; gradient 100% methylene chloride to 85% methylene chloride) provided the diaminopyrazine (121 mg, 63%) as a pale yellow oil; ¹H NMR (500 MHz, CDCl₃) δ 7.45 (s, 1H), 3.65 (br, 2H), 3.40 (br, 4H), 2.88 (s, 6H), 2.64-2.60 (m, 6H).

**Step C**: Prepared from the product of Step B and 4-pyridylboronic acid according to general procedure 6 (method 2). Purification by column chromatography (12 g ISCO column eluting with methylene chloride and methanol; gradient 100% methylene chloride to 70% methylene chloride) followed by conversion to the HCl salt using 2M HCl in diethyl ether provided the title compound (106 mg, 80%) as an orange-yellow solid; ¹H NMR (300 MHz, CD₃OD) δ 8.79-8.75 (m, 3H), 8.63-8.61 (m, 2H), 4.23-4.18 (m, 2H), 3.98-3.95 (m, 2H), 3.82-3.77 (m, 2H), 3.45-3.36 (m, 4H), 3.31-3.27 (m, 1H), 3.22 (s, 6H); ¹³C NMR (75 MHz, CD₃OD) δ 155.7, 152.0, 146.3, 142.7, 137.1, 133.7, 123.1, 60.1, 56.8, 53.1, 45.5, 39.8; HPLC t_{R} = 12.9 min, >99%; ES-MS: (M + H) = 329 m/z.

### Example 31

### 2-(4-(3-(Dimethylamino)-6-(1H-pyrrolo[2,3-b]pyridin-4-yl)pyrazin-2-yl)piperazin-1-yl)ethanol

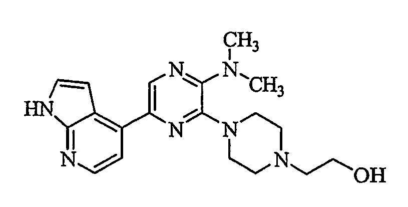

### Step A: 2-(4-(3-(Dimethylamino)-6-(trimethylstannyl)pyrazin-2-yl)piperazin-1-yl)ethanol

Prepared from the product of Step B (example 30) and hexamethylditin according to general procedure 7. Purification by column chromatography (12 g ISCO column eluting with hexanes and ethyl acetate; gradient 100% hexanes to 50% hexanes) provided the stannane (158 mg, 62%) as a clear, colorless oil; ¹H NMR (500 MHz, CDCl₃) δ 7.72 (s, 1H), 3.67-3.64 (t, *J*= 5.3 Hz, 2H), 3.38 (br, 4H), 2.92 (s, 6H), 2.67-2.58 (m, 6H), 0.28 (s, 9H).

**Step B**: Prepared from the product of Step A and 4-bromoindazole according to general procedure 8. Purification by column chromatography (12 g ISCO column eluting with methylene chloride/10% ammonium hydroxide in methanol; gradient 100% methylene chloride to 85% methylene chloride) provided the title compound (63 mg, 45%) as a yellow solid; ¹H NMR (500 MHz, DMSO-*d*₆) δ 11.71 (s, 1H), 8.49 (s, 1H), 8.26-8.25 (d, *J* = 5.0 Hz, 1H), 7.57-7.56 (d, *J*= 5.0 Hz, 1H), 7.53-7.52 (t, *J*= 2.8 Hz, 1H), 7.05-7.04 (m, 1H), 4.57-4.56 (br, 1H), 3.60 (br, 3H), 3.50 (m, 2H, masked by solvent), 3.00 (s, 7H), 2.96-2.59 (m, 6H); ¹³C NMR (75 MHz, DMSO-*d*₆) δ 149.7, 147.6, 145.7, 142.5, 138.7, 136.0, 132.1, 126.3, 116.2, 112.2, 100.7, 59.9, 58.0, 52.7, 46.0 (one aliphatic signal masked by solvent); HPLC t_{R} = 9.5min, 98.5%; ES-MS: (M + H) = 368m/z.

### Example 32

### N,N-dimethyl-3-(4-methyl-1,4-diazepan-1-yl)-5-(pyridin-4-yl)pyrazin-2-amine hydrochloride

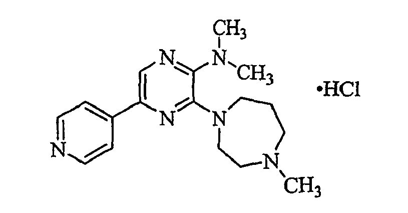

### Step A: 5-Bromo-N,N-dimethyl-3-(4-methyl-1,4-diazepan-1-yl)pyrazin-2-amine

Prepared from the product of Step A (example 23) and 1-methylhomopiperazine according to general procedure 4 (method 2). Purification by column chromatography (12 g ISCO column eluting with methylene chloride and methanol; gradient 100% methylene chloride to 80% methylene chloride) provided the diaminopyrazine (245 mg, 76%) as a yellow oil; ¹H NMR (500 MHz, CDCl₃) δ 7.63 (s, 1H), 3.78-3.76 (m, 2H), 3.68-3.66 (t, *J* = 6.1 Hz, 2H), 2.75 (s, 6H), 2.62-2.60 (m, 2H), 2.55-2.53 (t, *J* = 5.5 Hz, 2H), 2.36 (s, 3H), 1.94-1.91 (m, 2H).

**Step B**: Prepared from the product of Step A and 4-pyridylboronic acid according to general procedure 6 (method 2). Purification by column chromatography (12 g ISCO column eluting with methylene chloride and methanol; gradient 100% methylene chloride to 85% methylene chloride) followed by conversion to the HCl salt with 2M HCl in diethyl ether provided the title compound (78 mg, 61%) as an orange solid; ¹H NMR (300 MHz, CD₃OD) δ 8.53-8.43 (m, 2H), 8.38 (s, 1H), 8.18-8.16 (m, 2H), 4.23-4.06 (m, 1H), 3.81-3.72 (m, 3H) 3.65-3.43 (m, 3H), 3.22-3.20 (m, 1H, partially masked by solvent), 2.89 (s, 6H), 2.85 (s, 3H), 2.21-2.13 (m, 2H); ¹³C NMR (75 MHz, DMSO-*d*₆) δ 151.3, 151.1, 147.3, 147.0, 136.5, 133.7, 122.4, 58.0, 57.9, 45.7, 45.4, 39.8, 26.1 (one aliphatic signal masked by solvent); HPLC t_{R} = 8.9 min, >99%; ES-MS: (M + H) = 313 m/z.

### Example 33

### N,N-Dimethyl-3-(4-methyl-1,4-diazepan-1-yl)-5-(1H-pyrazolo[3,4-b]pyridin-4-yl)pyrazin-2-amine dihydrochloride

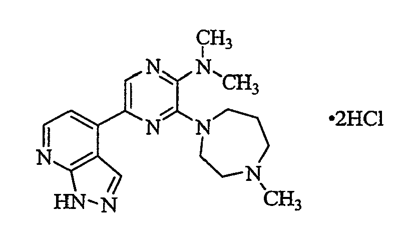

### Step A: N,N-Dimethyl-3-(4-methyl-1,4-diazepan-1-yl)-5-(trimethylstannyl)pyrazin-2-amine

Prepared from the product of Step A in example 32 and hexamethylditin according to general procedure 7. Purification by Combiflash chromatography (40g ISCO column eluting with methylene chloride and methanol; gradient 100% methylene chloride to 85% methylene chloride) provided the stannane (577 mg, 72%) as a brown oil; ¹H NMR (500 MHz, CDCl₃) δ 7.61 (s, 1H), 3.76-3.73 (m, 2H), 3.68-3.62 (m, 2H), 2.76 (s, 6H), 2.63-2.56 (m, 2H), 2.52-2.46 (m, 2H), 2.32 (s, 3H), 1.96-1.87 (m, 2H), 0.29 (s, 9H).

### Step B: 2-Chloro-4-(5-(dimethylamino)-6-(4-methyl-1,4-diazepan-1-yl)pyrazin-2-yl)nicotinaldehyde

Prepared from the product of Step A and 2-chloro-4-iodopyridine-3-carboxaldehyde according to general procedure 8 and purified by column chromatography (12 g ISCO column eluting with methylene chloride and methanol/ammonia mixture (10:1); gradient 100% methylene chloride to 80% methylene chloride over 30 min at 25 mL/min) proving the coupled product (76 mg, 61%) as yellow oil; ¹H NMR (500 MHz, CD₃OD) δ 10.31 (s, 1H), 8.46-8.44 (d, *J*= Hz, 1H), 8.10 (s, 1H), 7.56-7.55 (d, *J* = 5.2 Hz, 1H), 3.75-3.74 (m, 2H), 3.64-3.62 (m, 2H), 2.91 (s, 6H), 2.64-2.59 (m, 4H), 2.38 (s, 3H), 1.95-1.93 (m, 2H).

**Step C**: The product from Step B (76 mg, 0.20 mmol) was dissolved in ethanol (2 mL) and acetic hydrazide (74 mg, 1 mmol) added. The mixture was stirred for 24 h, concentrated and hydrazine monohydrate (3 mL) and ethanol (1 ml) were added and reflux continued for 8 h. The reaction was concentrated and purified by semi-preparatory HPLC (eluting with acetonitrile (0.05% TFA)/water (0.05% TFA); 5% acetonitrile (0.05% TFA) to 90% acetonitrile (0.05% TFA) over 40 minutes) to provide a yellow oil (41 mg). This oil was converted to the *bis* hydrochloride salt with 2N HCl in Et₂O to provide the title compound (41 mg, 26%) as a red solid; ¹H NMR (500 MHz, CD₃OD) δ 9.20 (s, 1H), 8.79 (s, 1H), 9.73-8.72 (d, *J* = 6.2 Hz, 1H), 8.10-8.09 (d, *J* = 6.2 Hz, 1H), 4.27-4.23 (dd, *J =* 16.1, 6.0 Hz, 1H), 4.00-3.95 (dd, *J =* 16.0, 9.0 Hz, 1H), 3.85-3.83 (t, *J*= 5.6 Hz, 2H), 3.75-3.71 (dd, *J* = 14.0, 6.2 Hz, 1H), 3.65-3.62 (dd, *J =* 9.4, 4.1 Hz, 1H), 3.55-3.51 (dd, *J* = 13.4, 9.4 Hz, 1H), 3.34 (m, 1H), 3.24 (m, 1H, masked by solvent), 3.18 (s, 6H), 2.95 (s, 3H), 2.29-2.09 (t, *J*= 5.6 Hz, 2H); ¹³C NMR (125 MHz, CD₃OD) δ 152.3, 151.1, 148.3, 147.1, 143.3, 136.5, 134.7, 132.6, 115.5, 112.3, 57.8, 57.0, 45.8, 45.0, 39.6, 25.3; HPLC t_{R} = 9.3 min, 98.5%; ES-MS: (M + H) = 353 m/z.

### Example 34

### N,N-(Piperidin-3-yl)-6-(pyridin-4-yl)pyrazine-2,3-diamine

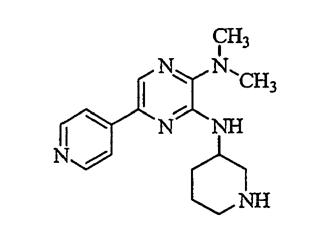

### Step A: tert-Butyl 3-(6-bromo-3-(dimethylamino)pyrazin-2-ylamino)piperidine-1-carboxylate

Prepared from the product of Step A (example 30) and 3-amino-1-boc-piperidine according to general procedure 4 (method 2). Purification by column chromatography (12 g ISCO column eluting with methylene chloride and methanol; gradient 100% methylene chloride to 85% methylene chloride) provided the diaminopyrazine (127 mg, 33%) as a brown oil; ¹H NMR (500 MHz, CDCl₃) δ 7.56 (s, 1H), 5.06-5.05 (m, 1H), 4.04 (br, 1H), 3.61-3.56 (m, 3H), 3.29 (br, 1H), 2.71 (s, 6H), 1.90-1.86 (m, 1H), 1.79-1.69 (m, 2H), 1.61-1.55 (m, 1H, partially masked by solvent peak), 1.41 (9H).

**Step B**: Prepared from the product of Step A and 4-pyridylboronic acid according to general procedure 6 (method 2). Removal of the protecting group was achieved by dissolving the coupled product in methanol (4 mL) and adding 2M HCl in diethyl ether (10 ml) at 0 °C; the mixture was then stirred at room temperature for 24h. The reaction mixture was concentrated, diluted with water, neutralized with sodium bicarbonate and extracted with chloroform. The organics were washed with brine, dried over sodium sulfate and concentrated. Purification by preparative thin-layer chromatography (Analtech No.21521 plates eluting with 90:10:1 methylene chloride/methanol/ammonium hydroxide) provided the title compound (32 mg, 16% over 2 steps) as a yellow solid; ¹H NMR (500 MHz, DMSO-*d*₆) δ 8.61-8.60, (d, *J* = 4.1 Hz, 2H), 8.17 (s, 1H), 7.93-7.92 (d, *J* = 4.0 Hz, 2H), 6.05-6.04 (d, *J* = 7.3 Hz, 1H), 4.09 (s, 1H), 3.09-3.07 (d, *J*= 9.8 Hz, 1H), 2.80 (s, 5H), 2.59 (s, 3H, partially masked by solvent peak), 1.87 (s, 1H), 1.66 (m, 2H), 1.50 (m, 1H); ¹³C NMR (75 MHz, DMSO-*d*₆) δ 150.0, 147.8, 145.5, 144.4, 138.2, 125.6, 119.5, 50.6, 46.9, 45.7, 29.6, 24.3 (one aliphatic signal masked by solvent); HPLC t_{R} = 8.9min, >99%; ES-MS: (M + H) = 299 m/z.

### Example 35

### N,N-Dimethyl-3-(piperidin-4-yloxy)-5-(pyridin-4-yl)pyrazin-2-amine hydrochloride

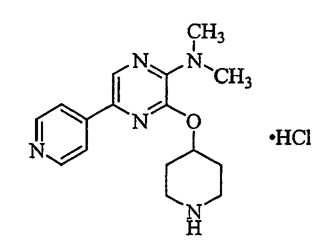

### Step A: tert-Butyl 4-(6-bromo-3-(dimethylamino)pyrazin-2-yloxy)piperidine-1-carboxylate

Prepared from the product of Step A (example 30) and 4-hydroxy-*N-*Boc-piperidine according to general procedure 5 and used in the next step without purification.

### Step B: tert-Butyl 4-(3-(dimethylamino)-6-(pyridin-4-yl)pyrazin-2-yloxy)piperidine-1-carboxylate:

Prepared from the product of Step A and 4-pyridylboronic acid according to general procedure 6 and used in the next step without purification.

**Step C**: Prepared from the product of Step B in a similar manner to that described in Step C (example 22) and purified by column chromatography (12 g ISCO column eluting with methylene chloride and methanol/concentrated ammonium hydroxide (10:1); gradient 100% methylene chloride to 90% methylene chloride) to provide the free base of the title compound. This was converted to the HCl salt using 1M HCl in ether providing the salt (197 mg, 58% over 3 steps) as a yellow solid; ¹H NMR (500 MHz, CD₃OD) δ 8.63-8.61 (d, *J=* 6.8 Hz, 2H), 8.59 (s, 1H), 8.34-8.32 (d, *J* = 6.8 Hz, 2H), 5.63-5.59 (m, 1H), 3.44-3.36 (m, 4H), 3.34 (s, 6H), 2.39-2.33 (m, 2H), 2.27-2.14 (m, 2H); ¹³C NMR (125 MHz, CD₃OD) δ 152.2, 149.9, 148.5, 145.2, 136.0, 131.6, 121.6, 69.6, 42.8, 41.1, 28.4; HPLC t_{R} = 8.85 min, >99%; ES-MS: (M + H) = 300 m/z.

### Example 36

### 3-(3-(Dimethylamino)propoxy)-N,N-dimethyl-5-(pyridin-4-yl)pyrazin-2-amine hydrochloride

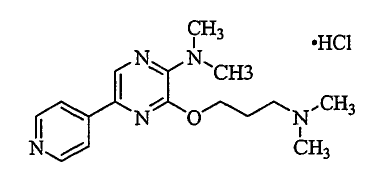

**Step A**: 5-Bromo-3-(3-(dimethylamino)propoxy)-N,*N-*dimethylpyrazin-2-amine: Prepared from the product of Step A (example 30) and 3-(*N*,*N-*Dimethyl)-propan-1-ol according to general procedure 5 and used in the next step without purification.

**Step B**: Prepared from the product of Step A and 4-pyridylboronic acid according to general procedure 6 and purified by column chromatography (12 g ISCO column eluting with methylene chloride and methanol/concentrated ammonium hydroxide (10:1); gradient 100% methylene chloride to 90% methylene chloride) to provide the , title compound as the free base. This was converted to the HCl salt using 1M HCl in ether providing the salt (143 mg, 42%) as a yellow solid; ¹H NMR (500 MHz, CD₃OD) δ 8.66-8.64 (d, *J*= 7.0 Hz, 2H), 8.65 (s, 1H), 8.48-8.46 (d, *J* = 7.0 Hz, 2H), 4.63-4.59 (t, *J* = 6.0 Hz, 2H), 3.39-3.34 (m, 2H), 3.36 (s, 6H), 2.95 (s, 6H), 2.39-2.30 (m, 2H); ¹³C NMR (125 MHz, CD₃OD) δ 154.9, 150.0, 149.4, 142.6, 137.3, 130.4, 122.0, 64.9, 56.6, 43.7, 41.2, 25.4; HPLC t_{R} = 9.02 min, 95.6%; ES-MS: (M + H) = 302 m/z.

### Example 37

### N,N-Diethyl-3-(4-methyl-1,4-diazepan-1-yl)-5-(pyridin-4-yl)pyrazin-2-amine hydrochloride

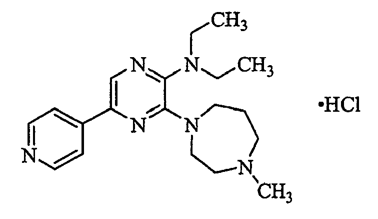

### Step A: 3,5-Dibromo-N,N-diethylpyrazin-2-amine

Prepared from 2-amino-3,5-dibromopyrazine and iodoethane according to general procedure 1. Purification by column chromatography (12 g ISCO column eluting with hexanes and ethyl acetate; gradient 100% hexanes to 50% hexanes) provided the substituted aminopyrazine (220 mg, 59%) as a yellow oil; ¹H NMR (300 MHz, CDCl₃) δ 8.09 (s, 1H), 3.52-3.45 (q, *J* = 14.1 Hz, *J* = 7.0 Hz, 4H), 1.21-1.17 (t, *J*= 7.0 Hz, 6H).

### Step B: 5-Bromo-N,N-diethyl-3-(4-methyl-1,4-diazepan-1-yl)pyrazin-2-amine

Prepared from the product of Step A and 1-methylhomopiperazine according to general procedure 4 (method 2). Purification by column chromatography (12 g ISCO column eluting with methylene chloride and methanol; gradient 100% methylene chloride to 80% methylene chloride) provided the diaminopyrazine (144 mg, 59%) as a yellow oil; ¹H NMR (300 MHz, CDCl₃) δ 7.65 (s, 1H), 3.79-3.78 (m, 2H), 3.71-3.67 (t, *J* = 6.2 Hz, 2H), 3.26-3.19 (q, *J* = 14.1 Hz, *J* = 7.1 Hz, 4H), 2.59-2.52 (m, 2H), 2.51-2.49 (m, 2H), 2.36 (s, 3H), 1.94-1.92 (m, 2H), 1.00-0.95 (t, *J*= 7.1 Hz, 6H).

**Step C**: Prepared from the product of Step B and 4-pyridylboronic acid according to general procedure 6 (method 2). Purification by column chromatography (12 g ISCO column eluting with methylene chloride and methanol; gradient 100% methylene chloride to 70% methylene chloride) followed by conversion to the HCl salt with 2M HCl in diethyl ether provided the title compound (154 mg, 98%) as an orange solid; ¹H NMR (300 MHz, CD₃OD) δ 8.73-8.71 (d, *J*= 6.0 Hz, 2H), 8.66 (s, 1H), 8.48-8.46 (d, *J* = 6.2 Hz, 2H), 4.27-3.33 (m, 12H, partially masked by solvent), 2.96 (s, 3H), 2.28-2.24 (m, 2H), 1.14-1.09 (t, *J* = 6.9 Hz, 6H); ¹³C NMR (75 MHz, CD₃OD) δ 153.4, 149.7, 147.5, 144.64, 135.3, 134.4, 122.6, 57.7, 45.7, 45.3, 44.1, 25.9, 13.2 (two aliphatic signals masked by solvent); HPLC t_{R} = 10.0 min, >99%; ES-MS: (M + H) = 341 m/z.

### Example 38

### 1-Methyl-4-(6-(pyridin-4-yl)-3-(pyrrolidin-1-yl)pyrazin-2-yl)-1,4-diazepane hydrochloride

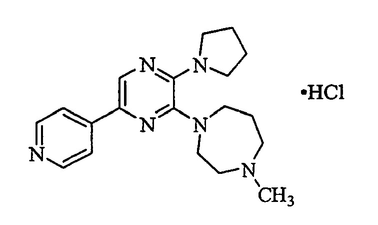

### Step A: 2-(Pyrrolidin-1-yl)pyrazine

Prepared from chloropyrazine and pyrrolidine according to general procedure 2 providing the aminopyrazine as a tan solid (0.92 g, crude); ¹H NMR (500 MHz, CDCl₃) δ 8.02-8.01 (dd, *J* = 2.6, 1.5 Hz, 1H), 7.87 (d, *J*= 1.4 Hz, 1H), 7.75-7.76 (d, *J* = 2.7 Hz, 1H), 3.51-3.47 (m, 4H), 2.07-2.00 (m, 4H); ES-MS: (M + H) = 150 m/z.

### Step B: 3,5-Dibromo-2-(pyrrolidin-1-yl)pyrazine

Prepared from the product of Step A and *N-*bromosuccinimide according to general procedure 3 providing the dibromopyrazine (0.46 g, 30%) as a tan solid; ¹H NMR (300 MHz, CDCl₃) δ 8.01 (s, 1H), 3.71-3.67 (m, 4H), 1.98-1.94 (m, 4H); ES-MS: (M + H) = 306 m/z.

**Step C**: 1-(6-Bromo-3-(pyrrolidin-1-yl)pyrazin-2-yl)-4-methyl-1, 4-diazepane Prepared from the product of Step B and 1-methylhomopiperazine (0.20 mL, 1.5 mmol) according to general procedure 4 (method 1) providing the diaminopyrazine (400 mg, quant) as a brown oil.

**Step D**: Prepared from the product of step C and 4-pyridylboronic acid according to general procedure 6 (method 2) and purified by column chromatography (12 g ISCO column eluting with methylene chloride and methanol/concentrated ammonium hydroxide (10:1); gradient 100% methylene chloride to 90% methylene chloride) providing the free base of the title compound. This was converted to the HCl salt using 2M HCl in ether providing the salt (215 mg, 76%) as an orange-red solid; ¹H NMR (500 MHz, CD₃OD) δ 8.61-8.60 (dd, *J*= 5.2, 1.4 Hz, 2H), 8.54 (s, 1H), 8.25-8.24 (dd, *J* = 5.1, 1.5 Hz, 2H), 4.13-3.89 (m, 2H), 3.63-3.44 (m, 10H), 2.96 (s, 3H), 2.23-2.19 (m, 2H), 2.00-1.95 (m, 4H); ¹³C NMR (125 MHz, CD₃OD) δ 151.2, 150.4,146.9, 146.5, 135.6, 134.3, 121.6, 57.8, 57.6, 50.3, 50.2, 46.4, 45.2, 26.3, 25.7; HPLC t_{R} = 6.84 min, 95.2%; ES-MS: (M + H) = 339 m/z.

### Example 39

### 4-(6-(4-Methyl-1,4-diazepan-1-yl)-5-(pyrrolidin-1-yl)pyrazin-2-yl)-1H-pyrrolo[2,3-b]pyridine dihydrochloride

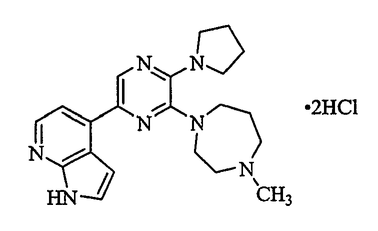

### Step A: 1-Methyl-4-(3-(pyrrolidin-1-yl)-6-(trimethylstannyl)pyrazin-2-yl)-1,4-diazepane

Prepared from the product of Step C (example 38) and hexamethylditin according to general procedure 7. Purification by column chromatography (12 g ISCO column eluting with methylene chloride and methanol/ammonia mixture (10:1); gradient 100% methylene chloride to 80% methylene chloride over 30 min at 25 mL/min) provided the aryl stannane (180 mg, 33%) as a yellow oil; ¹H NMR (500 MHz, CDCl₃) δ 7.74-7.73 (t, *J*= 3.2 Hz, 1H), 3.97-3.95 (t, *J* = 4.7 Hz, 2H), 3.71 (br s, 2H), 3.33-3.26 (m, 8H), 2.79 (s, 3H), 2.31 (br s, 2H), 1.93-1.89 (m, 4H), 0.28 (s, 9H).

**Step B**: Prepared from the product of Step A and 4-bromoazaindole according to general procedure 8. Purification by column chromatography (12 g ISCO column eluting with methylene chloride and methanol/ammonia mixture (10:1); gradient 100% methylene chloride to 80% methylene chloride over 30 min at 25 mL/min) followed by purification by semi-preparatory HPLC (eluting with acetonitrile (0.05% TFA)/water (0.05% TFA); 5% acetonitrile (0.05% TFA) to 90% acetonitrile (0.05% TFA) over 40 minutes) provided an orange oil (26 mg). This oil was converted to the *bis* hydrochloride salt with 2N HCl in ether providing the title compound (26 mg, 13%) as an orange solid; ¹H NMR (500 MHz, CD₃OD) δ 8.67 (s, 1H), 8.35-8.33 (d, *J* = 6.4 Hz, 1H), 8.05-8.04 (d, *J* = 6.4 Hz, 1H), 7.71-7.70 (d, *J* = 3.6 Hz, 1H), 7.41-7.40 (d, *J* = 3.6 Hz, 1H), 4.09-4.05 (dd, *J*= 16.0, 5.9 Hz, 1H), 3.87-3.83 (dd, *J* = 16.1, 8.3 Hz, 1H), 3.73-3.57 (m, 8H), 3.53-3.48 (m, 1H), 3.36-3.32 (m, 1H), 2.97 (s, 3H), 2.27-2.21 (m, 2H), 2.04-2.00 (m, 4H); ¹³C NMR (125 MHz, CD₃OD) δ 149.8, 147.1, 147.0, 141.3, 137.8, 134.9, 133.9, 130.0, 122.3, 113.2, 105.2, 57.7, 57.4, 50.7, 50.2, 46.6,45.1,26.3,25.4; HPLC t_{R} = 10.8 min, 95.6%; ES-MS: (M + H) = 378 m/z.

### Example 40

### 1-(6-(Pyridin-4-yl)-3-(pyrrolidin-1-yl)pyrazin-2-yl)-1,4-diazepane hydrochloride

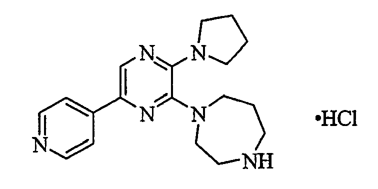

### Step A: tert-Butyl 4-(6-bromo-3-(pyrrolidin-1-yl)pyrazin-2-yl)-1,4-diazepane-1-carboxylate

Prepared from the product of Step B (example 38) and 1-boc-homopiperazine according to general procedure 4 (method 2) providing the diaminopyrazine (1.78 g, quant.) as a dark oil; ¹H NMR (500 MHz, CDCl₃) δ 7.68-7.66 (d, *J* = 6.8 Hz, 1H), 3.56 (s, 4H), 3.52-3.37 (m, 4H), 3.31-3.29 (m, 4H), 1.91-1.90 (m, 4H), 1.88-1.83 (m, 2H), 1.46-1.45 (d, *J*= 3.5 Hz, 9H); ES-MS: (M + H) = 426, 428 m/z.

### Step B: tert-Butyl 4-(6-(pyridin-4-yl)-3-(pyrrolidin-1-yl)pyrazin-2-yl)-1,4-diazepane-1-carboxylate

Prepared from the product of Step A and 4-pyridylboronic acid according to general procedure 6 (method 1). Purification by column chromatography (12 g ISCO column eluting with methylene chloride and a 10:1 methanol/ammonium hydroxide mixture; gradient 100% methylene chloride to 95% then 90% and finally 85% methylene chloride) provided the coupled product (200 mg, 75%) as an orange solid; ¹H NMR (500 MHz, CDCl₃) δ 8.62-8.61 (d, *J*= 3.9 Hz, 2H), 8.22 (s, 1H), 7.78 (s, 2H), 3.64-3.53 (m, 4H), 3.52-3.49 (m, 2H), 3.43-3.41 (m, 5H), 3.35-3.32 (m, 1H), 1.94-1.91 (m, 6H), 1.44-1.43 (d, *J* = 3.9 Hz, 9H).

**Step C**: The product from Step B (150 mg, 0.354 mmol) was dissolved in methanol (5 ml) and 2 N HCl in ether (10 ml) was added. The mixture was allowed to stir for 3 h, after which time a yellow precipitate had formed. The mixture was concentrated, dissolved in 10% ammonium hydroxide in methanol solution (5 ml) and reconcentrated. Purification by column chromatography (4 g ISCO column eluting with methylene chloride and a 10:1 methanol/ammonium hydroxide mixture; gradient 100% methylene chloride to 90% then 85% methylene chloride) provided the free base of the title compound as an oil. This was converted to the HCl salt (2N HCl in ether, 1 equiv.) providing the salt (81 mg, 63%) as a yellow solid; ¹H NMR (500 MHz, CD₃OD) δ 8.54-8.53 (d, *J =* 4.7 Hz, 2H), 8.41 (s, 1H), 7.99-7.98 (d, *J* = 5.0 Hz, 2H), 3.89-3.88 (m, 2H), 3.66-3.64 (m, 2H), 3.51-3.50 (m, 6H), 3.38-3.36 (m, 2H), 2.15-2.14 (m, 2H), 1.98-1.97 (m, 4H); ¹³C NMR (75 MHz, CD₃OD) δ 149.0, 148.6, 146.2, 145.6, 135.1, 132.5, 119.8, 49.1, 47.1, 46.4, 45.8, 25.9, 25.0 (one aliphatic carbon signal masked by solvent); HPLC t_{R} = 9.64 min, 100%; ES-MS: (M + H) = 325 m/z.

### Example 41

### 4-(6-(1,4-Diazepan-1-yl)-5-(pyrrolidin-1-yl)pyrazin-2-yl)-1H-pyrrolo[2,3-b]pyridine

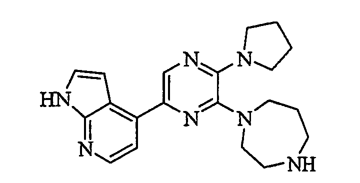

### Step A: tert-Butyl 4-(3-(pyrrolidin-1-yl)-6-(trimethylstannyl)pyrazin-2-yl)-1,4-diazepane-1-carboxylate

Prepared from the product of Step A (example 40) and hexamethylditin according to general procedure 7. Purification by column chromatography (12 g ISCO column eluting with hexanes and ethyl acetate; gradient 100% hexanes to 80% hexanes) provided the aryl stannane (190 mg, 79%) as a thick oil that solidified on standing: ¹H NMR (300 MHz, CDCl₃) δ 7.64-7.63 (d, *J* = 3.7 Hz, 1H), 3.58-3.53 (m, 4H), 3.47-3.41 (m, 2H), 3.36-3.22 (m, 6H), 1.92-1.87 (m, 4H), 1.85-1.81 (m, 2H), 1.47-1.45 (d, *J* = 4.1 Hz, 9H), 0.36-0.18 (t, *J* = 26.8 Hz, 9H).

### Step B: tert-Butyl 4-(3-(pyrrolidin-1-yl)-6-(1H-pyrrolo[2,3-b]pyridin-4-yl)pyrazin-2-yl)-1,4-diazepane-1-carboxylate

Prepared from the product of Step A and 4-bromoazaindole according to general procedure 8. Purification by column chromatography (12 g ISCO column eluting with hexanes and ethyl acetate; gradient 100% hexanes to 0% hexanes) provided the coupled product (86 mg, 50%) as a yellow foamy solid that contained some PPh₃ residues: ¹H NMR (500 MHz, CDCl₃) δ 9.11 (br s, 1H), 8.91 (s, 1H), 8.35-8.34 (d, *J* = 4.9 Hz, 1H), 7.48-7.47 (m, 1H), 7.38-7.37 (m, 1H), 7.04-7.02 (m, 1H), 3.69-3.60 (m, 4H), 3.56-3.46 (m, 7H), 3.38-3.36 (m, 1H), 2.05-1.93 (m, 6H), 1.45-1.44 (d, *J* = 4.6 Hz, 9H).

**Step C**: Prepared from the product of Step B in a similar manner to that described for Step C (example 40). Purification by column chromatography (4 g ISCO column eluting with methylene chloride and a 10:1 methanol/ammonium hydroxide mixture; gradient 100% methylene chloride to 90% then 85% methylene chloride) provided the title compound (48 mg, 72%) as a yellow solid; ¹H NMR (500 MHz, CDCl₃) δ 9.37 (br s, 1H), 8.39 (s, 1H), 8.35-8.34 (d, *J*= 5.1 Hz, 1H), 7.59-7.58 (d, *J*= 5.1 Hz, 1H), 7.38-7.37 (d, *J* = 3.6 Hz, 1H), 7.06-7.05 (d, *J* = 3.6 Hz, 1H) 3.67-3.64 (m, 4H), 3.49-3.47 (m, 4H), 3.08-3.06 (m, 2H), 2.95-2.93 (m, 2H), 1.98-1.95 (m, 4H), 1.91-1.86 (m, 2H); ¹³C NMR (75 MHz, CDCl₃) δ 149.6, 147.1, 146.7, 143.2, 138.2, 138.0, 132.4, 124.8, 116.9, 113.3, 101.8, 52.9, 49.4, 49.3, 48.4, 48.2, 31.0, 25.1 (one aliphatic carbon signal masked by solvent); HPLC t_{R} = 10.7 min, 100%; ES-MS: (M + H)= 364 m/z.

### Example 42

### 5-(6-(1,4-Diazepan-1-yl)-5-(pyrrolidin-1-yl)pyrazin-2-yl)-1H-indazole hydrochloride

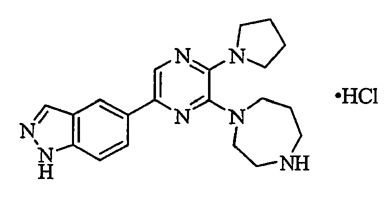

### Step A: tert-Butyl 4-(6-(1H-indazol-5-yl)-3-(pyrrolidin-1-yl)pyrazin-2-yl)-1,4-diazepane-1-carboxylate

Prepared from the product of Step A (example 41) and 5-bromoindazole according to general procedure 8. Purification by column chromatography (12 g ISCO column eluting with hexanes and ethyl acetate; gradient 100% hexanes to 0% hexanes) provided the coupled product (105 mg, 46%) as a yellow foamy solid that contained some PPh₃ residues. This was taken forward without characterization.

**Step B**: Prepared from the product of Step A in a similar manner to that described for Step C (example 40). Purification by column chromatography (4 g ISCO column eluting with methylene chloride and a 10:1 methanol/ammonium hydroxide mixture; gradient 100% methylene chloride to 90% then 85% methylene chloride) provided the free base of the title compound. This was converted to the HCl salt (2N HCl in ether, 1 equiv.) providing the salt (73 mg, 81%) as a yellow solid; ¹H NMR (500 MHz, CD₃OD) δ 8.40 (s, 1H), 8.18 (s, 1H), 8.01-7.99 (d, *J*= 8.7 Hz, 1H), 7.84 (s, 1H), 7.67-7.65 (d, *J* = 8.7 Hz, 1H), 3.97-3.96 (m, 2H), 3.81-3.80 (m, 4H), 3.72-3.71 (m, 2H), 3.62-3.61 (m, 2H), 3.45-3.44 (m, 2H), 2.24-2.23 (m, 2H), 2.14-2.13 (m, 4H); ¹³C NMR (75 MHz, CD₃OD) δ 151.1, 142.6, 140.2, 129.6, 126.8, 120.2, 118.0, 112.6, 52.7, 47.3, 47.1, 26.8, 26.7 (three aromatic signals missing due to overlap; two aliphatic signals masked by solvent); HPLC t_{R} = 11.69 min, 98.4%; ES-MS: (M + H) = 364 m/z.

### Example 43

### 5-(6-(1,4-Diazepan-1-yl)-5-(pyrrolidin-1-yl)pyrazin-2-yl)-3-methyl-1H-indazole hydrochloride

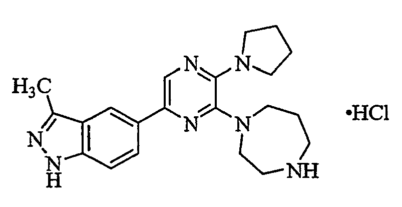

### Step A: 1-(5-Bromo-2-fluorophenyl)ethanol

Methylmagnesium bromide (3M solution in tetrahydrofuran, 18 ml, 54.2 mmol) was added to a solution of 5-bromo-2-fluorobenzaldehyde (10.0 g, 49.3 mmol) under nitrogen at 0°C over 30 min. The resulting solution was allowed to warm to room temperature over 14 h, upon which TLC analysis showed no remaining starting material, and two new products. The mixture was quenched with water, diluted with ethyl acetate and the organic phase removed and dried over sodium sulfate. This was then concentrated and purified by column chromatography (120 g ISCO column eluting with hexanes and ethyl acetate; gradient 100% hexanes to 50% hexanes). The second fraction was collected as the product, providing the alcohol (5.8 g, 53%) as an oil; ¹H NMR (500 MHz, CDCl₃) δ 7.65-7.63 (dd, *J*= 6.5, 2.6 Hz, 1H), 7.36-7.33 (ddd, *J* =8.7, 4.6, 2.6 Hz, 1H), 6.93-6.89 (dd, *J* = 9.9, 8.7 Hz, 1H), 5.17-5.16 (m, 1H), 1.91-1.90 (m, 1H), 1.51-1.49 (d, *J=* 6.4 Hz, 3H).

### Step B: 1-(5-Bromo-2-fluorophenyl)ethanone

Chromium trioxide (2.6 g, 26.0 mmol) was dissolved in water (3.7 ml) and cooled in an ice bath. Concentrated sulfuric acid (2.2 ml) was added over 5 min, and the solution was diluted with water (7.4 ml). The mixture was then added dropwise to a solution of the product from Step A (5.7 g, 26.0 mmol) in acetone (17 ml) at 0-20°C over 30 min. The resultant solution was allowed to warm to room temperature over 14 h. It was then partitioned between ether (300 ml) and water (300 ml) and the organic phase removed. The aqueous phase was washed with ether (100 ml) and the combined organic phases were dried over sodium sulfate then concentrated, providing the ketone (5.2 g, 92%) as a dark liquid: ¹H NMR (300 MHz, CDCl₃) δ 8.01-7.98 (dd, *J* = 6.2, 2.3 Hz, 1H), 7.64-7.59 (m, 1H), 7.08-7.02 (t, *J*= 10.0 Hz, 1H), 2.65-2.63 (d, *J* = 4.9 Hz, 3H).

### Step C: 5-Bromo-3-methyl-1H-indazole

Hydrazine (20 ml) and the product from Step B (5.1 g, 24.0 mmol) were heated to reflux and held for 24 h. The mixture was then cooled to room temperature and quenched with water (250 ml). A precipitate formed; this was isolated by filtration, washing with water, and then the solids were dissolved in ethyl acetate. The organic mixture was dried over sodium sulfate then concentrated, providing the indazole (3.54 g, 71%) as a beige solid; ¹H NMR (300 MHz, CDCl₃) δ 9.95 (br s, 1H), 7.83-7.82 (dd, *J =* 1.7, 0.4 Hz, 1H), 7.47-7.44 (dd, *J =* 8.7, 1.8 Hz, 1H), 7.33-7.31 (d, *J* = 8.7 Hz, 1H), 2.56 (s, 3H); ES-MS: (M + H) = 211, 213 m/z.

### Step D: tert-Butyl 4-(6-(3-methyl-1H-indazol-5-yl)-3-(pyrrolidin-1-yl)pyrazin-2-yl)-1,4-diazepane-1-carboxylate

Prepared from the product of Step A (example 41) and the product of Step C according to general procedure 8. Purification by column chromatography (12 g ISCO column eluting with hexanes and ethyl acetate; gradient 100% hexanes to 25% hexanes) provided the coupled product (109 mg, 47%) as a yellow foamy solid; ¹H NMR (300 MHz, CDCl₃) δ 9.80 (br s, 1H), 8.18-8.16 (m, 2H), 8.00-7.97 (d, *J* = 8.7 Hz, 1H), 7.48-7.45 (d, *J* = 8.7 Hz, 1H), 3.70-3.65 (m, 4H), 3.59-3.53 (m, 2H), 3.46-3.33 (m, 6H), 2.64 (s, 3H), 1.99-1.94 (m, 6H), 1.46-1.44 (d, *J*= 5.3 Hz, 9H).

**Step E**: Prepared from the product of Step D in a similar manner to that described for Step C (example 40). Purification by column chromatography (4 g ISCO column eluting with methylene chloride and a 10:1 methanol/ammonium hydroxide mixture; gradient 100% methylene chloride to 90% then 80% methylene chloride) provided the free base of the title compound. This was converted to the HCl salt (2N HCl in ether, 1 equiv.) providing the salt (94 mg, 99%) as a yellow solid; ¹H NMR (500 MHz, CD₃OD) δ 8.43 (s, 1H), 8.16-8.14 (d, *J* = 8.9 Hz, 1H), 8.00 (s, 1H), 7.68-7.66 (d, *J* = 8.9 Hz, 1H), 3.97-3.96 (m, 2H), 3.82-3.81 (m, 4H), 3.72-3.70 (m, 2H), 3.61-3.60 (m, 2H), 3.45-3.43 (m, 2H), 2.72 (s, 3H), 2.23-2.21 (m, 2H), 2.13-2.12 (m, 4H); ¹³C NMR (125 MHz, CD₃OD) δ 150.8, 144.3, 142.5, 142.4, 139.4, 129.8, 128.3, 123.2, 119.3, 118.0, 112.5, 52.2, 46.8, 46.6, 26.4, 26.3, 11.2 (two aliphatic signals masked by solvent); HPLC t_{R} = 12.0 min, 98.1%; ES-MS: (M + H) = 378 m/z.

### Example 44

### 2-(4-(6-(Pyridin-4-yl)-3-(pyrrolidin-1-yl)pyrazin-2-yl)piperazin-1-yl)ethanol) trihydrochloride

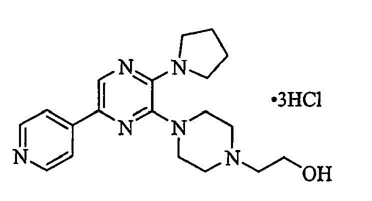

### Step A: 2-(4-(6-Bromo-3-(pyrrolidin-1-yl)pyrazin-2-yl)piperazin-1-yl)ethanol

Prepared from the product of Step B (example 38) and 1-methylhomopiperazine according to general procedure 4 (method 1) and purified by column chromatography (12 g ISCO column eluting with methylene chloride and methanol; gradient 100% methylene chloride to 85% methylene chloride) providing the diaminopyrazine (186 mg, 72%) as a yellow oil; ¹H NMR (300 MHz, CDCl₃) δ 7.73 (s, 1H), 3.66-3.63 (t, *J* = 5.3 Hz, 2H), 3.42-3.38 (t, *J* = 6.7 Hz, 4H), 3.23 (br, 4H), 2.66-2.59 (m, 6H), 1.94-1.89 (m, 4H).

**Step B**: Prepared from the product of Step A and 4-pyridylboronic acid according to general procedure 6 (method 2). Purification by column chromatography (12g ISCO column eluting with methylene chloride and 10% ammonium hydroxide in methanol; gradient 100% methylene chloride to 80% methylene chloride) followed by preparative TLC (eluting with 90:10:1methylene chloride/methanol/ammonium hydroxide) provided the free base of the title compound. Conversion to the tris-HCl salt with 2M HCl in diethyl ether followed by trituration with methylene chloride/hexanes provided the salt (141 mg, 59%) as an orange-yellow solid; ¹H NMR (300 MHz, CD₃OD) δ 8.78 (br, 2H), 8.67-8.62 (m, 3H), 3.97 (br, 4H), 3.86-3.77 (m, 6H), 3.41-3.31 (m, 6H), 2.09 (br, 4H); ¹³C NMR (75 MHz, CD₃OD) δ 154.9, 148.4, 148.1, 142.9, 133.5, 132.0, 123.2, 60.2, 56.9, 52.9, 52.2, 47.4, 23.7; HPLC t_{R} = 9.1min, >99%; ES-MS: (M + H) = 355m/z.

### Example 45

### N-(Piperidin-3-yl)-6-(pyridin-4-yl)-3-(pyrrolidin-1-yl)pyrazin-2-amine dihydrochloride

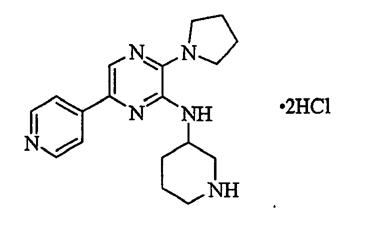

### Step A: tert-Butyl 3-(6-bromo-3-(pyrrolidin-1-yl)pyrazin-2-ylamino)piperidine-1-carboxylate

Prepared from the product of Step B (example 38) and 3-amino-*N-*Boc-piperidine according to general procedure 4 (method 2) and the product purified by column chromatography (12 g ISCO column eluting with hexanes and ethyl acetate; gradient 100% hexanes to 40% hexanes over 30 min at 25 mL/min) providing the diaminopyrazine (168 mg, 24%) as a clear oil; ¹H NMR (300 MHz, CDCl₃) δ 7.48 (s, 1H), 4.12-4.06 (br s, 1H), 3.67-3.56 (br s, 2H), 3.47-3.21 (br m, 7H), 1.93-1.83 (m, 5H), 1.41-1.29 (m, 11H).

### Step B: tert-Butyl 3-(6-(pyridin-4-yl)-3-(pyrrolidin-1-yl)pyrazin-2-ylamino)piperidine-1-carboxylate

Prepared from the product of Step A and 4-pyridylboronic acid according to general procedure 6 (method 2) and the product purified by column chromatography (12 g ISCO column eluting with hexanes and ethyl acetate; gradient 90% hexanes to 10% hexanes over 30 min at 25 mL/min) providing the coupled product (95 mg, 57%) as a yellow oil; ¹H NMR (300 MHz, CD₃OD) δ 8.49-8.48 (d, *J* = 5.4 Hz, 2H), 8.05 (s, 1H), 7.97 (br s, 2H), 4.11-4.07 (m, 1H), 3.59-3.44 (m, 7H), 2.04-1.92 (m, 5H), 1.88-1.78 (m, 2H), 1.64-1.59 (m, 1H), 1.48-1.36 (m, 4H), 1.27-1.22 (m, 6H).

**Step C**: The product from Step B (95 mg, 0.24 mmol) was stirred in TFA (2 mL) for 2 h and the reaction mixture concentrated and partitioned between methylene chloride and saturated sodium carbonate solution. The organic layer was removed, dried over sodium sulfate and concentrated to provide a yellow oil. This oil was treated with 2N HCl in diethyl ether to provide the title compound (73 mg, 76%) as an orange solid; ¹H NMR (500 MHz, CD₃OD) δ 8.79-8.77 (d, *J* = 6.9 Hz, 2H), 8.70-8.69 (d, *J* = 6.9 Hz, 2H), 8.29 (s, 1H), 4.67-4.63 (m, 1H), 3.99-3.97 (m, 4H), 3.71-3.67 (dd, *J* = 12.0, 3.2 Hz 1H), 3.41-3.38 (m, 1H), 3.10-3.14 (m, 2H), 2.23-2.20 (m, 1H), 2.14-2.04 (m, 6H), 1.95-1.90 (m, 1H); ¹³C NMR (125 MHz, CD₃OD) δ 154.4, 146.2, 144.5, 142.5, 133.0, 124.0, 123.2, 52.7, 47.2, 47.0, 45.0, 28.8, 26.5, 22.2; HPLC t_{R} = 9.3 min, >99%; ES-MS: (M + H) = 325 m/z.

### Example 46

### 3-(Piperidin-4-yloxy)-5-(pyridin-4-yl)-2-(pyrrolidin-1-yl)pyrazine

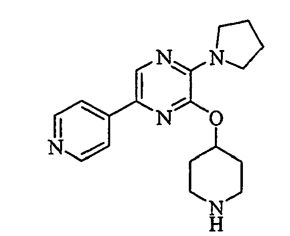

### Step A: tert-Butyl 4-(6-bromo-3-(pyrrolidin-1-yl)pyrazin-2-yloxy)piperidine-1-carboxylate

Prepared from the product of Step B (example 38) and 4-hydroxy-*N-*Boc-piperidine according to general procedure 5 and the product purified by column chromatography (40 g ISCO column eluting with hexanes and ethyl acetate; gradient 100% hexanes to 30% hexanes over 40 min at 40 mL/min) providing the alkoxypyrazine (1.10 g, 52%) as a yellow solid; ¹H NMR (500 MHz, CDCl₃) δ 7.64 (s, 1H), 5.26-5.23 (qui, *J*= 3.6 Hz, 1H), 3.65-3.63 (m, 5H), 3.61-3.58 (m, 1H), 3.45-3.40 (m, 2H), 1.99-1.95 (m, 2H), 1.93-1.91 (m, 4H), 1.80-1.75 (m , 2H), 1.47 (s, 9H).

### Step B: tert-Butyl 4-(6-(pyridin-4-yl)-3-(pyrrolidin-1-yl)pyrazin-2-yloxy)piperidine-1-carboxylate

Prepared from the product of Step A and 4-pyridylboronic acid according to general procedure 6 (method 2) and the product purified by column chromatography (12 g ISCO column eluting with hexanes and ethyl acetate; gradient 100% hexanes to 20% hexanes over 30 min at 25 mL/min) providing the coupled product (103 mg, 51%) as yellow oil; ¹H NMR (500 MHz, CD₃OD) δ 8.48-8.47 (d, *J* = 5.8 Hz, 2H), 8.25 (s, 1H), 7.90-7.88 (dd, *J* = 4.9, 1.4 Hz, 2H), 5.48-5.45 (m, 1H), 3.81-3.78 (t, *J* = 6.8 Hz, 4H), 3.66-3.64 (br m, 2H), 3.53-3.52 (br m, 2H), 2.10-2.05 (m, 2H), 2.01-1.96 (m, 4H), 1.86-1.83 (m, 2H), 1.47 (s, 9H).

**Step C**: Prepared from the product of Step B in a similar manner to that described for Step C (example 45) to provide the title compound (62 mg, 79%) as a white solid; ¹H NMR (500 MHz, CD₃OD) δ 8.48-8.47 (dd , *J*= 4.7, 1.5 Hz, 2H), 8.22 (s , 1H), 7.88-7.86 (dd , *J* = 4.7, 1.5 Hz, 2H), 5.39-5.34 (m, 1H), 3.81-3.78 (t, *J* = 6.5 Hz, 4H), 3.10-3.06 (m, 2H), 2.86-2.81 (m, 2H), 2.15-2.11 (m, 2H), 2.01-1.95 (m, 4H), 1.86-1.79 (m, 2H); ¹³C NMR (125 MHz, CD₃OD) δ 150.3, 148.5, 147.2, 147.1, 132.6, 132.4, 120.3, 72.9, 50.7, 44.3, 32.2, 26.4; HPLC t_{R} = 9.5 min, >99%; ES-MS: (M + H) = 326 m/z.

### Example 47

### 4-(6-(Piperidin-4-yloxy)-5-(pyrrolidin-1-yl)pyrazin-2-yl)-1H-pyrrolo[2,3-b]pyridine

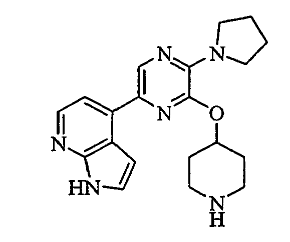

### Step A: tert-Butyl 4-(3-(pyrrolidin-1-yl)-6-(trimethylstannyl)pyrazin-2-yloxy)piperidine-1-carboxylate

Prepared from the product of Step A (example 46) and hexamethylditin according to general procedure 7. Purification by column chromatography (12 g ISCO column eluting with hexanes and ethyl acetate; gradient 100% hexanes to 60% hexanes over 30 min at 25 mL/min) provided the aryl stannane (285 mg, 79%) as colorless oil; ¹H NMR (500 MHz, CDCl₃) δ 7.59 (t, *J=* 4.2 Hz, 1H), 5.32 (m, 1H), 3.68-3.65 (t, *J*= 6.7 Hz, 4H), 3.62-3.57 (m, 2H), 3.47-3.44 (m, 2H), 1.98-1.88 (m, 6H), 1.83-1.78 (m, 2H), 1.47 (s, 9H), 0.31-0.19 (s, 9H).

### Step B: tert-Butyl 4-(3-(pyrrolidin-1-yl)-6-(1H-pyrrolo[2,3-b]pyridin-4-yl)pyrazin-2-yloxy)piperidine-1-carboxylate

Prepared from the product of Step A and 4-bromoazaindole according to general procedure 8. Purification by column chromatography (12 g ISCO column eluting with hexanes and ethyl acetate; gradient 100% hexanes to 0% hexanes over 30 min at 25 mL/min) provided the coupled product (118 mg, 45%) as a colorless oil; ¹H NMR (500 MHz, CDCl₃) δ 8.31 (s, 1H), 8.20-8.19 (d, *J*= 5.3 Hz, 1H), 7.51-7.50 (d, *J*= 5.2 Hz, 1H), 7.42-7.41 (d, *J* = 3.5 Hz, 1H), 6.98-6.97 (d, *J* = 3.5 Hz, 1H), 5.52-5.49 (m, 1H), 3.84-3.81 (t, *J*= 6.5 Hz, 4H), 3.71-3.67 (m, 2H), 3.55-3.50 (m, 2H), 2.15-2.09 (m, 2H), 2.03-1.99 (m, 4H), 1.96-1.89 (m, 2H), 1.49 (s, 9H).

**Step C**: Prepared from the product of Step B in a similar manner to that described for Step C (example 45). Purification by column chromatography (12 g ISCO column eluting with methylene chloride and methanol/ammonia mixture (10:1); gradient 100% methylene chloride to 80% methylene chloride over 30 min at 25 mL/min) provided the title compound (50 mg, 55%) as a yellow solid; ¹H NMR (500 MHz, CDCl₃) δ 9.72 (br s, 1H), 8.39-8.38 (d, *J*= 4.1 Hz, 1H), 8.33-8.32 (d, *J*= 5.1 Hz, 1H), 7.51-7.50 (d, *J* = 4.1 Hz, 1H), 7.38-7.37 (d, *J*= 3.6 Hz, 1H), 7.03-7.02 (d, *J*= 3.6 Hz, 1H), 5.44-5.39 (m, 1H), 3.84-3.81 (t, *J* = 6.7 Hz, 4H), 3.18-3.14 (m, 2H), 2.91-2.86 (m, 2H), 2.19-2.17 (m, 2H), 2.00-1.95 (m, 4H), 1.87-1.81 (m, 2H); ¹³C NMR (125 MHz, CDCl₃) δ 149.7, 147.0, 145.2, 143.1, 138.0, 134.0, 133.3, 124.7, 116.5, 112.6, 101.6, 71.4, 49.4, 43.9, 31.9, 25.5; HPLC t_{R} = 9.5 min, >99%; ES-MS: (M + H) = 365 m/z.

### Example 48

### 1-Methyl-4-(3-(piperidin-1-yl)-6-(pyridin-4-yl)pyrazin-2-yl)-1,4-diazepane hydrochloride

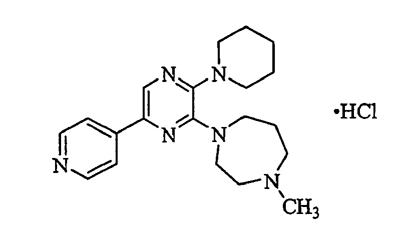

### Step A: 2-(Piperidin-1-yl)pyrazine

Prepared from chloropyrazine and piperidine according to general procedure 2 providing the aminopyrazine (1.74 g, crude) as a tan solid; ¹H NMR (500 MHz, CDCl₃) δ 8.13-8.12 (d, *J* = 1.4 Hz, 1H), 8.03-8.02 (dd, *J* = 2.5, 1.5 Hz, 1H), 7.77-7.76 (d, *J* = 2.6 Hz, 1H), 3.58-3.56 (m, 4H), 1.67-1.63 (m, 6H); ES-MS: (M + H) = 164 m/z.

### Step B: 3, 5-Dibromo-2-(piperidin-1-yl)pyrazine

Prepared from the product of Step A and *N-*bromosuccinimide according to general procedure 3 providing the dibromopyrazine (1.0 g, 31%) as a yellow solid; ¹H NMR (500 MHz, CDCl₃) δ 8.15 (s, 1H), 3.36-3.34 (m, 4H), 1.74-1.70 (m, 4H), 1.67-1.62 (m, 2H); ES-MS: (M + H)= 320 m/z.

### Step C: 1-(6-Bromo-3-(piperidin-1-yl)pyrazin-2-yl)-4-methyl-1,4-diazepane

Prepared from the product of Step B and 1-methylhomopiperazine according to general procedure 4 (method 1) and purified by column chromatography (12 g ISCO column eluting with methylene chloride and methanol/concentrated ammonium hydroxide (10:1); gradient 100% methylene chloride to 90% methylene chloride) to provide the diaminopyrazine (0.23 g, 65%) as a greenish yellow viscous oil; ¹H NMR (300 MHz, CDCl₃) δ 7.63 (s, 1H), 3.88-3.84 (t, *J* = 4.8 Hz, 2H), 3.75-3.71 (t, *J* = 6.1 Hz, 2H), 3.06-3.03 (m, 4H), 2.62-2.59 (m, 2H), 2.55-2.51 (t, *J* = 5.6 Hz, 2H), 2.36 (s, 3H), 1.97-1.90 (m, 2H), 1.67-1.66 (m, 6H); ES-MS: (M + H) = 354 m/z.

**Step D**: Prepared from the product of Step C and 4-pyridylboronic acid according to general procedure 6 (method 2) and purified by column chromatography (12 g ISCO column eluting with methylene chloride and methanol/concentrated ammonium hydroxide (10:1); gradient 100% methylene chloride to 90% methylene chloride) providing the free base of the title compound. This was converted to the HCl salt using 1M HCl in ether providing the salt (56 mg, 21%) as a red solid; ¹H NMR (500 MHz, CD₃OD) δ 8.62-8.61 (d, *J* = 4.5 Hz, 2H), 8.49 (s, 1H), 8.24-8.218 (d, *J* = 5.5 Hz, 2H), 3.93-3.91 (m, 2H), 3.77-3.32 (m, 10H), 2.93 (s, 3H), 2.27-2.22 (m, 2H), 1.73-1.67 (m, 6H); ¹³C NMR (75 MHz, CD₃OD) δ 150.2, 149.6, 148.1, 147.3, 137.6, 132.7, 122.1, 57.9, 57.6, 50.0, 49.5, 45.5, 45.2, 26.9, 26.1, 25.7; HPLC t_{R} = 10.35 min, 96.5%; ES-MS: (M + H) = 353 m/z.

### Example 49

### 3-(Piperidin-1-yl)-N-(piperidin-3-yl)-6-(pyridin-4-yl)pyrazin-2-amine

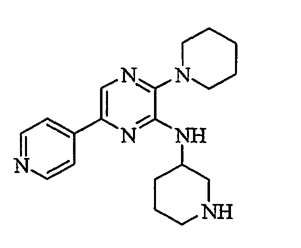

### Step A: 6-Bromo-3-(piperidin-1-yl)-N-(piperidin-3-yl)pyrazin-2-amine

Prepared from the product of Step B (example 48) and 3-amino-1-Boc-piperidine according to general procedure 4 (method 2). The crude product was obtained as a brown syrup (0.54 g) and used in the next step without purification.

### Step B: tert-Butyl 3-(3-(piperidin-1-yl)-6-(pyridin-4-yl)pyrazin-2-ylamino)piperidine-1-carboxylate

Prepared from the product of Step A and 4-pyridylboronic acid according to general procedure 6 (method 2). The crude product was obtained as a brownish yellow oil (0.18 g, 41 %) and used in the next step without purification.

**Step C**: Prepared from the product of Step B in a manner similar to that described in Step C (example 22) and purified by column chromatography (12 g ISCO column eluting with methylene chloride and methanol/concentrated ammonium hydroxide (10:1); gradient 100% methylene chloride to 90% methylene chloride) to provide a the title compound (41 mg, 29%) as a yellow solid; ¹H NMR (500 MHz, DMSO-*d*₆) δ 8.55-8.54 (dd, *J* = 4.8, 1.4 Hz, 2H), 8.12 (s, 1H), 8.03-8.01 (dd, *J* = 4.7, 1.4 Hz, 2H), 4.90 (br s, 1H), 4.64-4.56 (br s, 1H), 4.20-4.15 (m, 1H), 3.34-3.32 (d, *J*= 9.8 Hz, 1H), 3.14-3.12 (t, *J* = 5.0 Hz, 4H), 2.97-2.95 (br d, *J* = 12.5 Hz, 1H), 2.67-2.63 (m, 1H), 2.59-2.54 (dd, *J* = 11.6, 9.3 Hz, 1H), 2.11-2.09 (br d, *J*= 12.0 Hz, 1H), 1.82-1.72 (m, 5H), 1.71-1.57 (m, 4H); ¹³C NMR (125 MHz, CD₃OD) δ 150.4, 149.7, 148.2, 147.5, 140.9, 127.6, 121.7, 51.8, 50.9, 48.9, 46.9, 31.8, 26.9, 26.1, 25.6; HPLC t_{R} = 11.12 min, 96.7%; ES-MS: (M + H) = 339 m/z.

### Example 50

### 1-(3-(Azepan-1-yl)-6-(pyridin-4-yl)pyrazin-2-yl)-4-methyl-1,4-diazepane hydrochloride

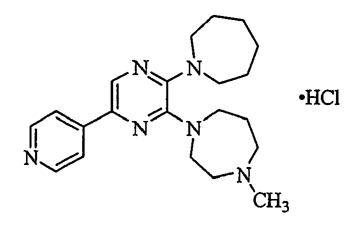

### Step A: 1-(Pyrazin-2-yl)azepane

Prepared from chloropyrazine and hexamethyleneimine according to general procedure 2 providing the aminopyrazine (1.74 g, crude) as a tan oil; ES-MS: (M + H) = 178 m/z.

### Step B: 1-(3, 5-Dibromopyrazin-2-yl)azepane .

Prepared from the product of Step A and *N-*bromosuccinimide according to general procedure 3 providing the dibromopyrazine (0.78 g, 42%) as a tan solid. This was taken forward without characterization.

### Step C: 1-(3-(Azepan-1-yl)-6-bromopyrazin-2-yl)-4-methyl-1,4-diazepane

Prepared from the product of Step B and 1-methylhomopiperazine according to general procedure 4 (method 1) and purified by column chromatography (12 g ISCO column eluting with methylene chloride and methanol/concentrated ammonium hydroxide (10:1); gradient 100% methylene chloride to 90% methylene chloride) to provide the diaminopyrazine (0.20 g, 54%) as a brownish yellow viscous oil; ¹H NMR (500 MHz, CDCl₃) δ 7.63 (s, 1H), 3.67-3.65 (t, *J*= 4.7 Hz, 2H), 3.58-3.56 (t, *J* = 6.0 Hz, 2H), 3.45-3.43 (t, *J* = 5.8 Hz, 4H), 2.59-2.54 (m, 4H), 2.36 (s, 3H), 1.94-1.89 (m, 2H), 1.67 (br s, 4H), 1.55-1.51 (m, 4H); ES-MS: (M + H) = 368 m/z.

**Step D**: Prepared from the product of Step C and 4-pyridylboronic acid according to general procedure 6 (method 2) and purified by column chromatography (12 g ISCO column eluting with methylene chloride and methanol/concentrated ammonium hydroxide (10:1); gradient 100% methylene chloride to 90% methylene chloride) providing the free base of the title compound. This was converted to the HCl salt using 1M HCl in ether providing the salt (110 mg, 50%) as a red solid; ¹H NMR (500 MHz, CD₃OD) δ 8.71 (s, 1H), 8.69-8.64 (d, *J* = 7.0 Hz, 2H), 8.56-8.54 (d, *J* = 7.0 Hz, 2H), 4.13-4.08 (m, 1H), 3.95-3.82 (m, 5H), 3.72-3.58 (m, 4H), 3.50-3.44 (m, 1H), 3.27-3.23 (m, 1H), 2.94 (s, 3H), 2.23-2.18 (m, 2H), 1.87-1.74 (m, 4H), 1.50-1.59 (m, 4H); ¹³C NMR (125 MHz, CD₃OD) δ 155.6, 150.5, 145.4, 142.2, 136.6, 132.1, 122.4, 57.8, 57.4, 51.5, 49.9, 46.0, 45.2, 29.4, 27.7, 25.6; HPLC t_{R} = 10.91 min, 95.7%; ES-MS: (M + H)= 367 m/z.

### Example 51

### 3-(Azepan-1-yl)-N-(piperidin-3-yl)-6-(pyridin-4-yl)pyrazin-2-amine dihydrochloride

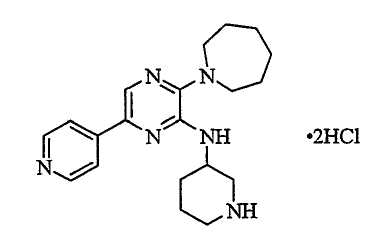

### Step A: tert-Butyl 3-(3-(azepan-1-yl)-6-bromopyrazin-2-ylamino)piperidine-1-carboxylate:

Prepared from the product of Step B (example 50) and 3-amino-1-Boc-piperidine according to general procedure 4. The crude product was obtained as a tan viscous oil (175 mg) and used in the next step without purification.

### Step B: tert-Butyl 3-(3-(azepan-1-yl)-6-(pyridin-4-yl)pyrazin-2-ylamino)piperidine-1-carboxylate

Prepared from the product of Step A and 4-pyridylboronic acid according to general procedure 6 (method 2). The crude product was obtained as a brown viscous oil (124 mg) and used in the next step without purification.

**Step C**: Prepared from the product of Step B in a manner similar to that described in Step C (example 22) and purified by column chromatography (12 g ISCO column eluting with methylene chloride and methanol/concentrated ammonium hydroxide (10:1); gradient 100% methylene chloride to 90% methylene chloride) to provide the free base. This was converted to the bis-HCl salt using 1M HCl in ether providing the title compound (43 mg, 37%) as a red solid; ¹H NMR (500 MHz, CD₃OD) δ 8.70-8.69 (d, *J* = 6.9 Hz, 2H), 8.65-8.63 (d, *J* = 7.0 Hz, 2H), 8.48 (s, 1H), 4.56-4.50 (m, 1H), 3.79-3.70 (m, 5H), 3.44-3.40 (m, 1H), 3.06-3.01 (td, *J* = 12.0, 3.5 Hz, 1H), 2.99-2.94 (m, 1H), 2.22-2.19 (m, 1H), 2.11-1.98 (m, 2H), 1.85-1.77 (m, 5H), 1.64-1.63 (m, 4H); ¹³C NMR (125 MHz, CD₃OD) δ 155.5, 149.6, 145.3, 142.2, 133.2, 130.7, 123.0, 52.1, 47.8, 46.9, 45.1, 29.4, 29.3, 28.4, 22.6; HPLC t_{R} = 11.50 min, 97.4%; ES-MS: (M + H) = 353 m/z.

### Example 52

### 4-(3-(4-Methyl-1,4-diazepan-1-yl)-5-(pyridin-4-yl)pyrazin-2-yl)morpholine trihydrochloride

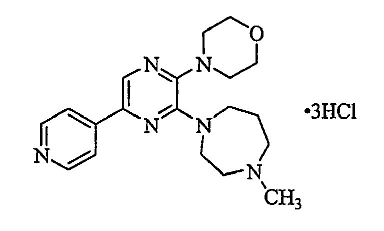

### Step A: 4-(Pyrazin-2-yl)morpholine

Prepared from 2-chloropyrazine and morpholine according to general procedure 2 providing the aminopyrazine (953 mg, 100%) as a brown solid, ¹H NMR (300 MHz, CDCl₃) δ 8.14-8.13 (m, 1H), 8.09-8.08 (m, 1H), 7.90-7.89 (m, 1H), 3.85-3.82 (t, *J* = 4.9 Hz, 4H), 3.58-3.55 (t, *J*= 4.9 Hz, 4H).

### Step B: 4-(3,5-Dibromopyrazin-2-yl)morpholine

Prepared from the product of Step A and *N-*bromosuccinimide according to general procedure 3. After the reaction was complete, the mixture was poured into ice-water (50 g) and stirred for 1h, neutralized with sodium bicarbonate, extracted with ethyl acetate (3x25 mL), dried organics over sodium sulfate and concentrated. Purification by column chromatography (40 g ISCO column eluting with methylene chloride and methanol; gradient 100% methylene chloride to 80% methylene chloride) provided the dibromopyrazine (531 mg, 28%) as a yellow-green oil, ¹H NMR (300 MHz, CDCl₃) δ 8.21 (s, 1H), 3.87-3.84 (t, *J*=4.7 Hz, 4H), 3.44-3.42 (t, *J*= 4.7 Hz, 4H).

### Step C: 4-(5-Bromo-3-(4-methyl-1,4-diazepan-1-yl)pyrazin-2-yl)morpholine

Prepared from the product of Step B and 1-methylhomopiperazine according to general procedure 4 (method 2). Purification by column chromatography (12 g ISCO column eluting with methylene chloride and methanol; gradient 100% methylene chloride to 85% methylene chloride) provided the diaminopyrazine (144 mg, 59%) as a yellow oil; ¹H NMR (500 MHz, CDCl₃) δ 7.67 (s, 1H), 3.84-3.80 (m, 6H), 3.71-3.69 (t, *J* = 6.0 Hz, 2H), 3.16-3.14 (m, 4H), 2.63-2.61 (m, 2H), 2.56-2.53 (m, 2H), 2.37 (s, 3H), 1.95-1.92 (m, 2H).

**Step D**: Prepared from the product of Step C and 4-pyridylboronic acid according to general procedure 6 (method 2). Purification by column chromatography (40g/12g ISCO columns eluting with methylene chloride and 10% ammonium hydroxide in methanol; gradient 100% methylene chloride to 80% methylene chloride) followed by conversion to the tris-HCl salt with 2M HCl in diethyl ether and trituration with methylene chloride/hexanes provided the title compound (28 mg, 49%) as an orange solid; ¹H NMR (300 MHz, CD₃OD) δ 8.79-8.76 (m, 2H), 8.70 (s, 1H), 8.63-8.60 (m, 2H), 4.40-4.27 (m, 1H), 3.95-3.72 (m, 9H), 3.55-3.47 (m, 6H), 2.95 (s, 3H), 2.26-2.25 (m, 2H); ¹³C NMR (125 MHz, CD₃OD) δ 155.3, 150.5, 147.6, 143.2, 137.7, 134.9, 123.6, 67.9, 57.9, 57.7, 57.6, 45.6, 45.4, 26.0 (one aliphatic carbon signal masked by solvent); HPLC t_{R} = 13.4min, >99%; ES-MS: (M + H) = 355m/z.

### MODES OF DELIVERY:

The compounds of Formula (I) can be incorporated into various types of ophthalmic formulations for delivery. The Formula (I) compounds may be delivered directly to the eye (for example: topical ocular drops or ointments; slow release devices such as pharmaceutical drug delivery sponges implanted in the cul-de-sac or implanted adjacent to the sclera or within the eye; periocular, conjunctival, sub-tenons, intracameral, intravitreal, or intracanalicular injections) or systemically (for example: orally, intravenous, subcutaneous or intramuscular injections; parenterally, dermal or nasal delivery) using techniques well known by those of ordinary skill in the art. It is further contemplated that the agents of the invention may be formulated in intraocular insert or implant devices.

The compounds of Formula (I) are preferably incorporated into topical ophthalmic formulations for delivery to the eye. The compounds may be combined with ophthalmologically acceptable preservatives, surfactants, viscosity enhancers, penetration enhancers, buffers, sodium chloride, and water to form an aqueous, sterile ophthalmic suspension or solution. Ophthalmic solution formulations may be prepared by dissolving a compound in a physiologically acceptable isotonic aqueous buffer. Further, the ophthalmic solution may include an ophthalmologically acceptable surfactant to assist in dissolving the compound. Furthermore, the ophthalmic solution may contain an agent to increase viscosity such as hydroxymethylcellulose, hydroxyethylcellulose, hydroxypropylmethylcellulose, methylcellulose, polyvinylpyrrolidone, or the like, to improve the retention of the formulation in the conjunctival sac. Gelling agents can also be used, including, but not limited to, gellan and xanthan gum. In order to prepare sterile ophthalmic ointment formulations, the active ingredient is combined with a preservative in an appropriate vehicle such as mineral oil, liquid lanolin, or white petrolatum. Sterile ophthalmic gel formulations may be prepared by suspending the compound in a hydrophilic base prepared from the combination of, for example, carbopol-974, or the like, according to the published formulations for analogous ophthalmic preparations; preservatives and tonicity agents can be incorporated.

The compounds of Formula (I) are preferably formulated as topical ophthalmic suspensions or solutions, with a pH of about 4 to 8. The compounds are contained in the composition in amounts sufficient to lower IOP in patients experiencing elevated IOP and/or maintaining normal IOP levels in glaucoma patients. Such amounts are referred to herein as "an amount effective to control IOP," or more simply "an effective amount." The compounds will normally be contained in these formulations in an amount 0.01 to 5 percent by weight/volume ("w/v %"), but preferably in an amount of 0.25 to 2 w/v %. Thus, for topical presentation 1 to 2 drops of these formulations would be delivered to the surface of the eye 1 to 4 times per day, according to the discretion of a skilled clinician.

The compounds of Formula (I) can also be used in combination with other glaucoma treatment agents, such as, but not limited to, β-blockers, prostaglandin analogs, carbonic anhydrase inhibitors, α₂ agonists, miotics, and neuroprotectants.

The following examples are provided to illustrate certain embodiments of the invention, but should not be construed as implying any limitations to the claims. The phrase "Compound of Formula (I)" in Examples 1-5 means that the formulation described in the respective Example is believed to be suitable for any compound according to Formula (I).

### COMPOSITION EXAMPLE 1

| **Ingredients** | **Concentration (w/v %)** |
|---|---|
| Compound of Formula (I) | 0.01 - 2% |
| Hydroxypropyl methylcellulose | 0.5% |
| Dibasic sodium phosphate (anhydrous) | 0.2% |
| Sodium chloride | 0.5% |
| Disodium EDTA (Edetate disodium) | 0.01 % |
| Polysorbate 80 | 0.05% |
| Benzalkonium chloride | 0.01 % |
| Sodium hydroxide / Hydrochloric acid | For adjusting pH to 7.3 - 7.4 |
| Purified water | q.s. to 100% |

### COMPOSITION EXAMPLE 2

| **Ingredients** | **Concentration (w/v %)** |
|---|---|
| Compound of Formula (I) | 0.01 - 2% |
| Methyl cellulose | 4.0% |
| Dibasic sodium phosphate (anhydrous) | 0.2% |
| Sodium chloride | 0.5% |
| Disodium EDTA (Edetate disodium) | 0.01% |
| Polysorbate 80 | 0.05% |
| Benzalkonium chloride | 0.01% |
| Sodium hydroxide / Hydrochloric acid | For adjusting pH to 7.3 - 7.4 |
| Purified water | q.s. to 100% |

### COMPOSITION EXAMPLE 3

| **Ingredients** | **Concentration (w/v %)** |
|---|---|
| Compound of Formula (I) | 0.01 - 2% |
| Guar gum | 0.4- 6.0% |
| Dibasic sodium phosphate (anhydrous) | 0.2% |
| Sodium chloride | 0.5% |
| Disodium EDTA (Edetate disodium) | 0.01 % |
| Polysorbate 80 | 0.05% |
| Benzalkonium chloride | 0.01% |
| Sodium hydroxide / Hydrochloric acid | For adjusting pH to 7.3 - 7.4 |
| Purified water | q.s. to 100% |

### COMPOSITION EXAMPLE 4

| **Ingredients** | **Concentration (w/v %)** |
|---|---|
| Compound of Formula (I) | 0.01 - 2% |
| White petrolatum and mineral oil and lanolin | Ointment consistency |
| Dibasic sodium phosphate (anhydrous) | 0.2% |
| Sodium chloride | 0.5% |
| Disodium EDTA (Edetate disodium) | 0.01% |
| Polysorbate 80 | 0.05% |
| Benzalkonium chloride | 0.01 % |
| Sodium hydroxide / Hydrochloric acid | For adjusting pH to 7.3 - 7.4 |

### ROCK-II INHIBITION DATA:

The ability of certain compounds of Formula (I) to inhibit rho kinase was evaluated by means of *in vitro* assays. Human recombinant Rho kinase (ROKα/ROCK-II, (aa 11-552), human active, catalog #14-451, Upstate Biotechnology Co., Lake Placid, NY), MgCl₂/ATP cocktail, and enzyme substrate (Upstate) are used.

The fluorescence polarization assays are performed using a Biomek 2000 Robotic Workstation (Beckman Instruments, Palo Alto, CA) in a 96-well plate format. The assays are performed utilizing the IMAP ROCK II kit (Molecular Devices, Sunnyvale, CA) as follows. Substrate and ATP concentrations used are 200nM and 10µM, respectively, while the enzyme concentration is 3.96x10⁻³ units per well. The substrate, enzyme, and ATP dilutions are made with the reaction buffer provided by the vendor. Test compounds are diluted in 10:10 DMSO-ethanol (vol/vol). For the actual assays, the various components are added into black, clear bottom, 96-well plates (Costar, Coming, NY) in a final volume of 20µl per well. After the enzyme reaction (60 min at 23°C), 60µl of the binding solution (IMAP kit, provided by vendor) is added per well and incubated for an additional 30 minutes in the dark at 23°C. Fluorescence polarization of the reaction mixtures is then measured on the Analyst™ HT instrument (Molecular Devices, Sunnyvale, CA).

The data generated are then analyzed using a non-linear, iterative, sigmoidal-fit computer program purchased from IDBS (Emeryville, CA) and as previously described (Sharif et al., J. Pharmacol. Exp. Ther. 286:1094-1102, 1998; Sharif et al., J. Pharmacol. Expt. Ther. 293:321-328, 2000; Sharif et al., J. Ocular Pharmacol. Ther. 18:141-162, 2002a; Sharif et al., J. Pharmac. Pharmacol. 54:539-547, 2002b) to generate the inhibition constants for the test compounds. TABLE 3 below shows inhibition constants for the example compounds listed above under the heading of "SYNTHESIS." The inhibition constants of TABLE 3 below are IC₅₀ or Ki (the concentration of the compound that inhibits the enzyme activity by 50% of the maximum) (Sharif et al., ibid.).

**TABLE 3. Enzyme Inhibition Constants (IC₅₀) Obtained for Compounds against Human Recombinant ROCK-II Enzyme**

| **EXAMPLE** | **IC₅₀ (nM)** | **EXAMPLE** | **IC₅₀ (nM)** |
|---|---|---|---|
| 1 | 3.0 | 27 | 5.2 |
| 2 | 58 | 28 | 3.0 |
| 3 | 2.8 | 29 | 45 |
| 4 | 0.53 | 30 | 0.30 |
| 5 | 25 | 31 | 0.053 |
| 6 | >100 | 32 | 1.7 |
| 7 | 0.95 | 33 | 0.54 |
| 8 | 53 | 34 | 3.1 |
| 9 | 22 | 35 | 0.94 |
| 10 | 59 | 36 | 11 |
| 11 | 17 | 37 | 6.7 |
| 12 | 6.9 | 38 | 0.33 |
| 13 | 6.4 | 39 | 0.16 |
| 14 | 3.5 | 40 | 0.33 |
| 15 | 5.5 | 41 | 0.021 |
| 16 | 0.82 | 42 | 0.033 |
| 17 | 0.21 | 43 | 0.11 |
| 18 | 9.6 | 44 | 0.078 |
| 19 | 0.064 | 45 | 0.25 |
| 20 | 5.6 | 46 | 0.084 |
| 21 | 4.2 | 47 | 0.054 |
| 22 | 1.1 | 48 | 0.56 |
| 23 | 0.68 | 49 | 0.30 |
| 24 | 1.7 | 50 | 0.02 |
| 25 | 2.4 | 51 | 0.19 |
| 26 | 2.1 | 52 | 6.3 |

## Claims

1. An ophthalmic pharmaceutical composition for use in the treatment of glaucoma and/or control of intraocular pressure, comprising an effective amount of a compound (I) of the following formula: 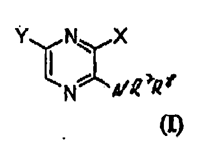 in which Y is selected from the following groups: 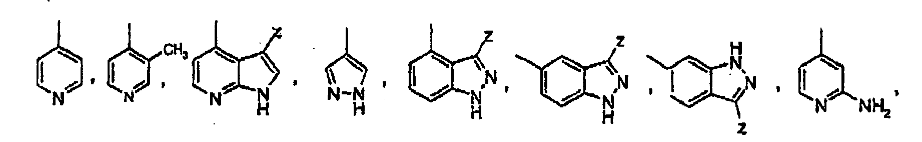 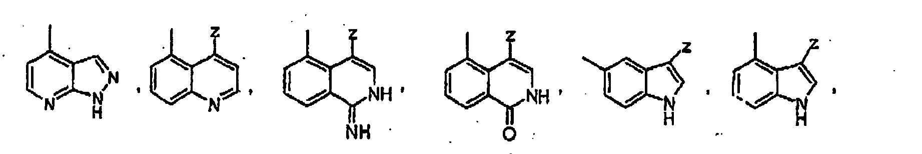 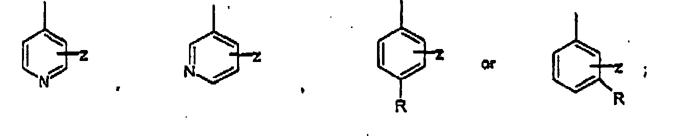 where:
X = OR¹, NR²R³;
z = H, OR⁶, halogen, CF₃, or C₁-C₄ alkyl;
R is OH, OR⁴, or S(O)ₙR⁶;
n is 0, 1 or 2;
R¹, R², R³ independently = H, C₁-C₆ alkyl optionally substituted by NR⁴R⁵, OH, OR⁶, aryl, heterocyclyl or heteroaryl, C₃₋₈ cyclic alkyl optionally substituted by NR⁴R⁵, OH, OR⁶, aryl, heterocyclyl, or heteroaryl, and heterocyclyl;
R² and R³ together can form a heterocyclic ring;
R⁴, R⁵ independently = H, C₁-C₆ alkyl optionally substituted by OH, OR⁶, aryl, heterocyclyl, or heteroaryl;
R⁶ =C₁-C₆ alkyl, aryl, or CF₃;
R⁷, R⁸ independently = H, C₁-C₆ alkyl optionally substituted by NR⁴R⁵, OH, OR⁶, or heterocycl, C₃-C₈ cyclic alkyl optionally substituted by NR⁴R⁵, OH, OR⁶, or heterocyclyl, and heterocyclyl; and
R⁷ and R⁸ together can form a heterocyclic ring, and
a pharmaceutically acceptable vehicle therefor.

2. The composition of claim 1 comprising a pharmaceutically acceptable salt of compound (I).

3. The composition of claim 1 further comprising a compound selected from the group consisting of:
ophthalmologically acceptable preservatives, surfactants, viscosity enhancers, penetration enhancers, gelling agents, hydrophobic bases, vehicles, buffers, sodium chloride, and water.

4. The composition of claim 1 wherein said composition comprises a plurality of glaucoma treatment agents.

5. The composition of claim 4 wherein at least one glaucoma treatment agent is selected from the group consisting of:
β-blockers, carbonic anhydrase inhibitors, α₂ agonists, miotics, and neuroprotectants.

6. The composition of claim 1 wherein said composition comprises from about 0.01 percent weight/volume to about 5 percent weight/volume of said compound.

7. The composition of claim 1 wherein said composition comprises from about 0.25 percent weight/volume to about 2 percent weight/volume of said compound.

8. Use of a compound of the following formula: 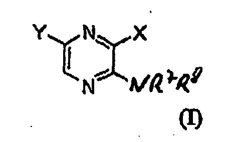 in which Y is selected from the following groups: 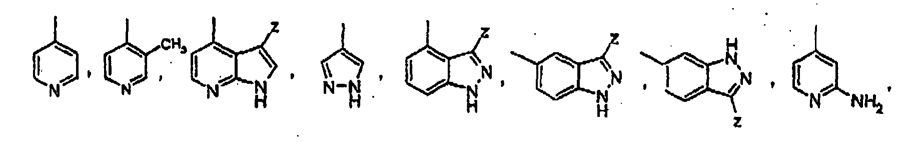 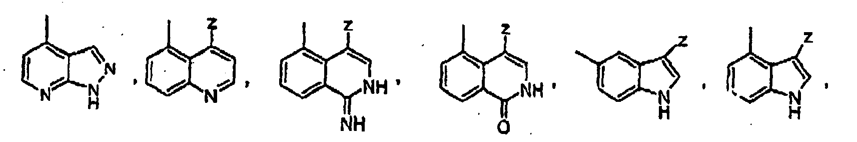 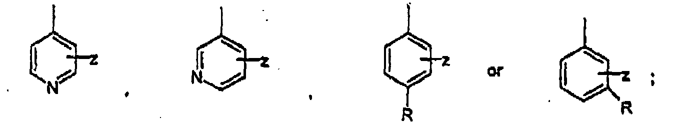 where:
X = OR¹, NR²R³;
z = H, OR⁶, halogen, CF₃, or C₁-C,₄ alkyl;
R is OH, OR⁴, or S(O)ₙR⁶;
n is 0, 1 or 2;
R¹, R², R³ independently = H, C₁-C₆ alkyl optionally substituted by NR⁴R⁵, OH, OR⁶, aryl, heterocyclyl or heteroaryl, C₃-C₈ cyclic alkyl optionally substituted by NR⁴R⁵, OH, OR⁶, aryl, heterocyclyl, or heteroaryl, and heterocyclyl;
R² and R³ together can form a heterocyclic ring;
R⁴, R⁵ independently = H, C₁-C₆ alkyl optionally substituted by OH, OR⁶, aryl, heterocyclyl, or heteroaryl;
R⁶= C₁-C₆ alkyl, aryl, or CF₃;
R⁷, R⁸ independently = H, C₁-C₆ alkyl optionally substituted by NR⁴R⁵, OH, OR⁶, or heterocycl, C₃-C₈ cyclic alkyl optionally substituted by NR⁴R⁵, OH, OR⁶, or heterocyclyl, and heterocyclyl; and
R⁷ and R⁸ together can form a heterocyclic ring; and
a pharmaceutically acceptable vehicle therefor,
for the manufacture of an ophthalmic pharmaceutical composition for the treatment of glaucoma and/or control of intraocular pressure.

9. The use of claim 8 wherein the composition is adapted for applying 1 to 2 drops of a composition comprising from about 0.01 percent weight/volume to about 5 percent weight/volume of compound (I) 1 to 4 times daily to the affected eye of a human or other mammal.

10. The use of claim 8 wherein said composition comprises a plurality of glaucoma treatment agents.

11. The use of claim 10 wherein at least one glaucoma treatment agent is selected from the group consisting of:
β-blockers, carbonic anhydrase inhibitors, α₂ agonists, miotics, and neuroprotectants.

12. Use of a compound of the following formula: 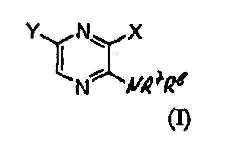 in which Y is selected from the following groups: 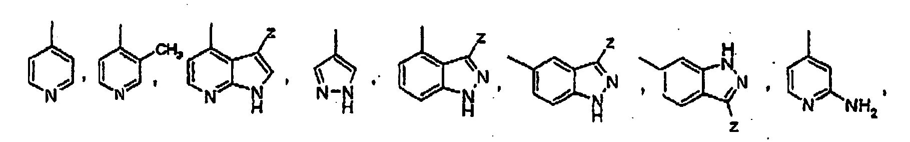 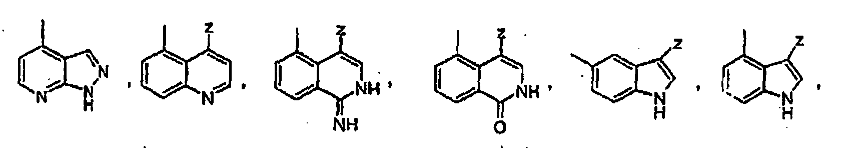 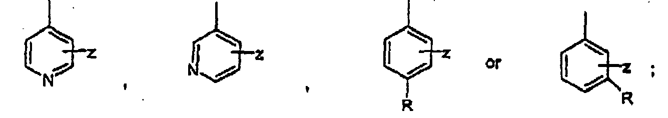 where:
X = OR¹, NR²R³;
z = H, OR⁶, halogen, CF₃, or C₁-C₄ alkyl;
R is OH, OR⁴, or S(O)ₙR⁶;
n is 0,1 or 2;
R¹, R², R³ independently = H, C₁-C₆ alkyl optionally substituted by NR⁴R⁵, OH, OR⁶, aryl, heterocyclyl or heteroaryl, C₃-C₈ cyclic alkyl optionally substituted by NR⁴R⁵, OH, OR⁶, aryl, heterocyclyl, or heteroaryl, and heterocyclyl;
R² and R³ together can form a heterocyclic ring;
R⁴, R⁵ independently = H, C₁-C₆ alkyl optionally substituted by OH, OR⁶, aryl, heterocyclyl, or heteroaryl;
R⁶ = C₁-C₆ alkyl, aryl, or CF₃;
R⁷, R⁸ independently = H, C₁-C₆ alkyl optionally substituted by NR⁴R⁵, OH, OR⁶, or heterocycl, C₃-C₈ cyclic alkyl optionally substituted by NR⁴,R⁵, OH, OR⁶, or heterocyclyl, and heterocyclyl; and
R⁷ and R⁸ together can form a heterocyclic ring; and
a pharmaceutically acceptable vehicle therefor,
for the manufacture of a pharmaceutical preparation for treating glaucoma, retinopathy, cerebrovascular contraction, ocular hypertension, normal-tension glaucoma, chronic obstructive pulmonary disease, angina pectoris, peripheral circulation disorder, immature birth, osteoporosis and/or cancer.

13. The use of claim 12 wherein the composition is adapted for applying 1 to 2 drops of a composition comprising from about 0.01 percent weight/volume to about 5 percent weight/volume of compound (I) 1 to 4 times daily to a human or other mammal.

14. The use of claim 12 wherein said composition comprises a plurality of glaucoma treatment agents.

15. The use of claim 14 wherein at least one glaucoma treatment agent is selected from the group consisting of:
β-blockers, carbonic anhydrase inhibitors, α₂ agonists, miotics, and neuroprotectants.

16. A compound represented by Formula (I): 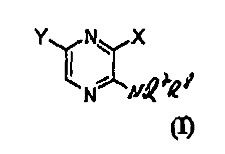 in which Y is selected from the following groups: 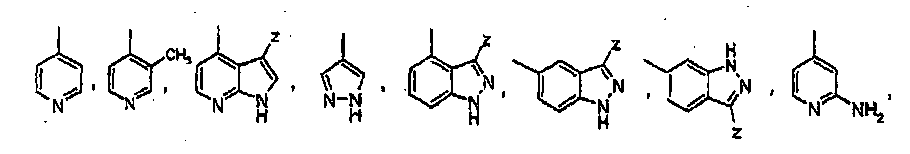 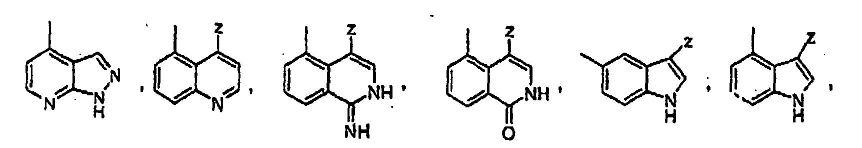 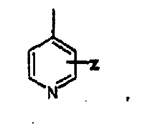 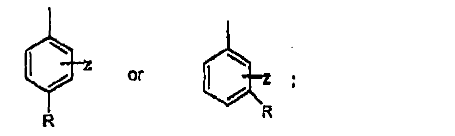 where:
X = OR¹, NR²R³;
z = H, OR⁶, halogen, CF₃, or C₁-C₄ alkyl;
R is OH;
n is 0, 1 or 2;
R¹, R², R³ independently = H, C₁-C₆ alkyl optionally substituted by NR⁴R⁵, OH, OR⁶, aryl, heterocyclyl or heteroaryl, C₃-C₈ cyclic alkyl optionally substituted by NR⁴R⁵, OH, OR⁶, aryl, heterocyclyl, or heteroaryl, and heterocyclyl;
R² and R³ together can form a heterocyclic ring;
R⁴, R⁵ independently = H, C₁-C₆alkyl optionally substituted by OH, OR⁶, aryl, heterocyclyl, or heteroaryl;
R⁶ = C₁-C₆ alkyl, aryl, or CF₃;
R⁷, R⁸ independently = H, C₁-C₆ alkyl optionally substituted by NR⁴R⁵, OH, OR⁶, or heterocycl, C₃-C₈ cyclic alkyl optionally substituted by NR⁴R⁵, OH, OR⁶, or heterocyclyl, and heterocyclyl; and
R⁷ and R⁸ together can form a heterocyclic ring.

17. The compound of claim 16 wherein the compound is a pharmaceutically acceptable salt of a compound according to Formula (I).

## Patentansprüche

1. Ophthalmische pharmazeutische Zusammensetzung zur Verwendung bei der Behandlung von Glaukom und/oder zur Kontrolle des Augeninnendrucks, umfassend eine wirksame Menge einer Verbindung (I) der folgenden Formel: 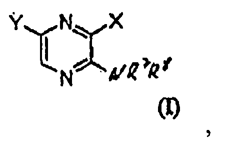 worin Y ausgewählt ist aus den folgenden Gruppen: 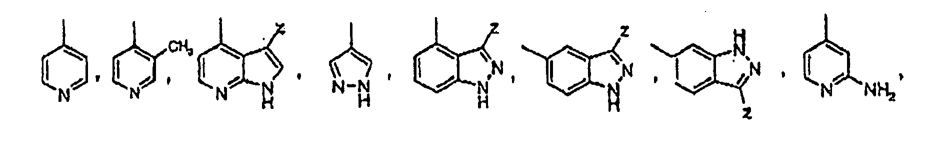 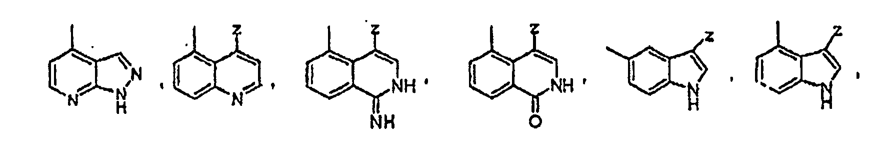 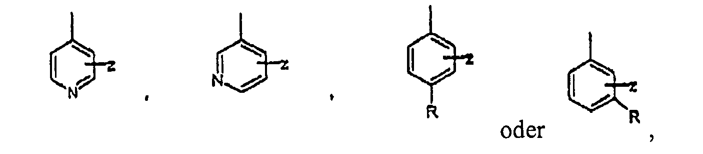 worin:
X = OR¹, NR²R³;
z = H, OR⁶, Halogen, CF₃ oder C₁-C₄-Alkyl;
R OH, OR⁴ oder S(O)ₙR⁶ ist;
n 0, 1 oder 2 ist;
R¹, R², R³ unabhängig = H, C₁-C₆-Alkyl, gegebenenfalls substituiert durch NR⁴R⁵, OH, OR⁶, Aryl, Heterocyclyl oder Heteroaryl, cyclisches C₃-C₈-Alkyl, gegebenenfalls substituiert durch NR⁴R⁵, OH, OR⁶, Aryl, Heterocyclyl oder Heteroaryl, und Heterocyclyl;
R² und R³ zusammen einen heterocyclischen Ring bilden können;
R⁴, R⁵ unabhängig = H, C₁-C₆-Alkyl, gegebenenfalls substituiert durch OH, OR⁶, Aryl, Heterocyclyl oder Heteroaryl;
R⁶ = C₁-C₆-Alkyl, Aryl oder CF₃;
R⁷, R⁸ unabhängig = H, C₁-C₆-Alkyl, gegebenenfalls substituiert durch NR⁴R⁵, OH, OR⁶ oder Heterocyclyl, cyclisches C₃-C₈-Alkyl, gegebenenfalls substituiert durch NR⁴R⁵, OH, OR⁶ oder Heterocyclyl, und Heterocyclyl und
R⁷und R⁸ zusammen einen heterocyclischen Ring bilden können; und
ein pharmazeutisch akzeptables Vehikel dafür.

2. Zusammensetzung nach Anspruch 1, umfassend ein pharmazeutisch akzeptables Salz von Verbindung (I).

3. Zusammensetzung nach Anspruch 1, ferner umfassend eine Verbindung, ausgewählt aus der Gruppe, bestehend aus: ophthalmologisch akzeptablen Konservierungsstoffen, oberflächenaktiven Mitteln, Viskositätsverstärkern, Penetrationsverstärkern, Geliermitteln, hydrophoben Basen, Vehikeln, Puffern, Natriumchlorid und Wasser.

4. Zusammensetzung nach Anspruch 1, wobei die Zusammensetzung mehrere Mittel zur Behandlung von Glaukom umfasst.

5. Zusammensetzung nach Anspruch 4, wobei mindestens ein Mittel zur Behandlung von Glaukom ausgewählt ist aus der Gruppe, bestehend aus: β-Blockern, Carboanhydraseinhibitoren, α₂-Agonisten, Miotika und Neuroprotektiva.

6. Zusammensetzung nach Anspruch 1, wobei die Zusammensetzung etwa 0,01 % Gewicht/Volumen bis etwa 5 % Gewicht/Volumen der Verbindung umfasst.

7. Zusammensetzung nach Anspruch 1, wobei die Zusammensetzung etwa 0,25 % GewichtNolumen bis etwa 2 % Gewicht/Volumen der Verbindung umfasst.

8. Verwendung einer Verbindung der folgenden Formel: 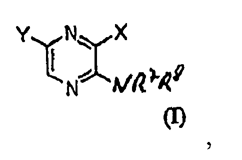 worin Y ausgewählt ist aus den folgenden Gruppen: 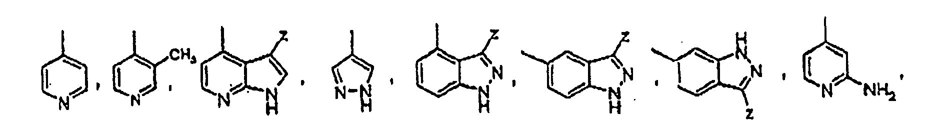 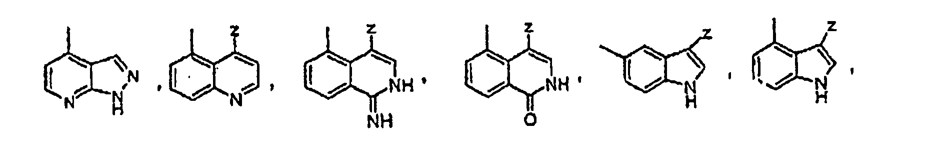 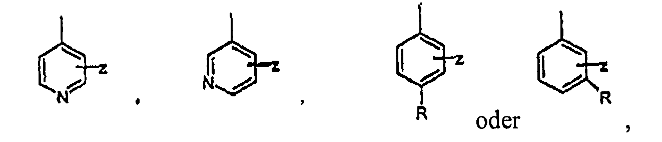 worin:
X = OR¹, NR²R³;
z = H, OR⁶, Halogen, CF₃ oder C₁-C₄-Alkyl;
R OH, OR⁴ oder S(O)ₙR⁶ ist;
n 0, 1 oder 2 ist;
R¹, R², R³ unabhängig = H, C₁-C₆-Alkyl, gegebenenfalls substituiert durch NR⁴R⁵, OH, OR⁶, Aryl, Heterocyclyl oder Heteroaryl, cyclisches C₃-C₈-Alkyl, gegebenenfalls substituiert durch NR⁴R⁵, OH, OR⁶, Aryl, Heterocyclyl oder Heteroaryl, und Heterocyclyl;
R² und R³ zusammen einen heterocyclischen Ring bilden können;
R⁴, R⁵ unabhängig = H, C₁-C₆-Alkyl, gegebenenfalls substituiert durch OH, OR⁶, Aryl, Heterocyclyl oder Heteroaryl;
R⁶ = C₁-C₆-Alkyl, Aryl oder CF₃;
R⁷, R⁸ unabhängig = H, C₁-C₆-Alkyl, gegebenenfalls substituiert durch NR⁴R⁵, OH, OR⁶ oder Heterocyclyl, cyclisches C₃-C₈-Alkyl, gegebenenfalls substituiert durch NR⁴R⁵, OH, OR⁶ oder Heterocyclyl, und Heterocyclyl und
R⁷ und R⁸ zusammen einen heterocyclischen Ring bilden können; und
eines pharmazeutisch akzeptablen Vehikels dafür
zur Herstellung einer ophthalmischen pharmazeutischen Zusammensetzung zur Behandlung von Glaukom und/oder zur Kontrolle des Augeninnendrucks.

9. Verwendung nach Anspruch 8, wobei die Zusammensetzung für das Aufbringen von 1 bis 2 Tropfen einer Zusammensetzung, umfassend etwa 0,01 % Gewicht/Volumen bis etwa 5 % Gewicht/Volumen von Verbindung (I), 1- bis 4-mal täglich auf das betroffene Auge eines Menschen oder anderen Säugers angepasst ist.

10. Verwendung nach Anspruch 8, wobei die Zusammensetzung mehrere Mittel zur Behandlung von Glaukom umfasst.

11. Verwendung nach Anspruch 10, wobei mindestens ein Mittel zur Behandlung von Glaukom ausgewählt ist aus der Gruppe, bestehend aus: β-Blockern, Carboanhydraseinhibitoren, α₂-Agonisten, Miotika und Neuroprotektiva.

12. Verwendung einer Verbindung der folgenden Formel: 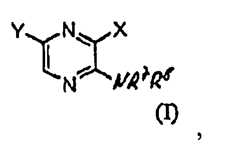 worin Y ausgewählt ist aus den folgenden Gruppen: 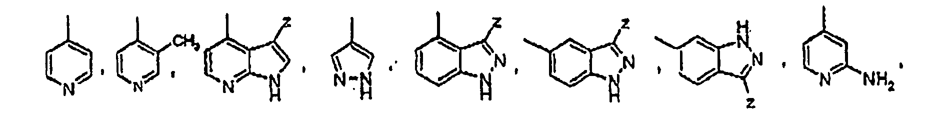 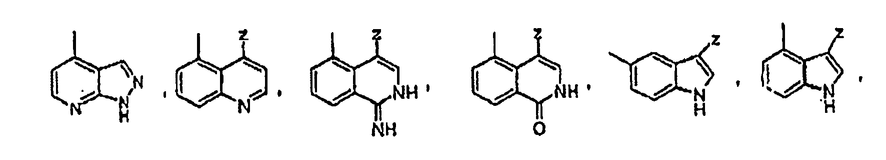 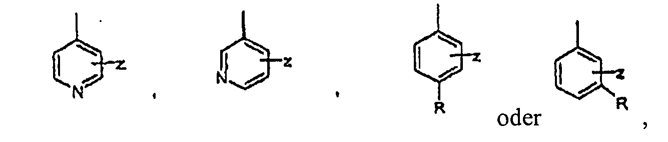 worin:
X = OR¹, NR²R³;
z = H, OR⁶, Halogen, CF₃ oder C₁-C₄-Alkyl;
R OH, OR⁴ oder S(O)ₙR⁶ ist;
n 0, 1 oder 2 ist;
R¹, R², R³ unabhängig = H, C₁-C₆-Alkyl, gegebenenfalls substituiert durch NR⁴R⁵, OH, OR⁶, Aryl, Heterocyclyl oder Heteroaryl, cyclisches C₃-C₈-Alkyl, gegebenenfalls substituiert durch NR⁴R⁵, OH, OR⁶, Aryl, Heterocyclyl oder Heteroaryl, und Heterocyclyl;
R² und R³ zusammen einen heterocyclischen Ring bilden können;
R⁴, R⁵ unabhängig = H, C₁-C₆-Alkyl, gegebenenfalls substituiert durch OH, OR⁶, Aryl, Heterocyclyl oder Heteroaryl;
R⁶ = C₁-C₆-Alkyl, Aryl oder CF₃;
R⁷, R⁸ unabhängig = H, C₁-C₆-Alkyl, gegebenenfalls substituiert durch NR⁴R⁵, OH, OR⁶ oder Heterocyclyl, cyclisches C₃-C₈-Alkyl, gegebenenfalls substituiert durch NR⁴R⁵, OH, OR⁶ oder Heterocyclyl, und Heterocyclyl und
R⁷ und R⁸ zusammen einen heterocyclischen Ring bilden können; und
eines pharmazeutisch akzeptablen Vehikels dafür
zur Herstellung eines pharmazeutischen Präparats zur Behandlung von Glaukom, Retinopathie, zerebrovaskulärer Kontraktion, Augenhypertension, Normaldruckglaukom, chronischer obstruktiver Lungenerkrankung, Angina pectoris, peripherer Kreislaufstörung, Frühgeburt, Osteoporose und/oder Krebs.

13. Verwendung nach Anspruch 12, wobei die Zusammensetzung für die Anwendung von 1 bis 2 Tropfen einer Zusammensetzung, umfassend etwa 0,01 % Gewicht/Volumen bis etwa 5 % Gewicht/Volumen von Verbindung (I), 1- bis 4-mal täglich bei einem Menschen oder anderen Säuger angepasst ist.

14. Verwendung nach Anspruch 12, wobei die Zusammensetzung mehrere Mittel zur Behandlung von Glaukom umfasst.

15. Verwendung nach Anspruch 14, wobei mindestens ein Mittel zur Behandlung von Glaukom ausgewählt ist aus der Gruppe, bestehend aus: β-Blockern, Carboanhydraseinhibitoren, α₂-Agonisten, Miotika und Neuroprotektiva.

16. Verbindung, dargestellt durch die Formel (I): 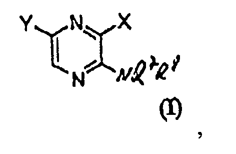 worin Y ausgewählt ist aus den folgenden Gruppen: 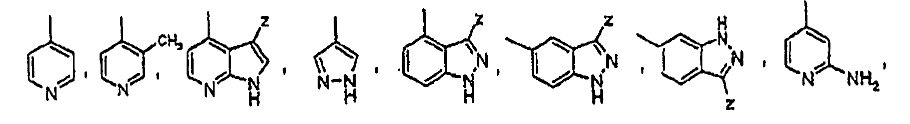 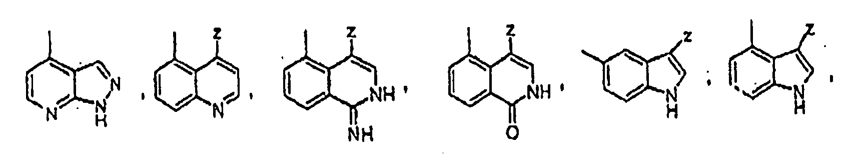 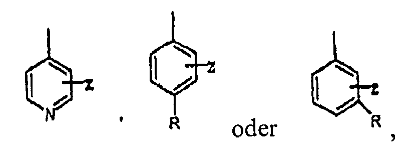 worin:
X == OR¹, NR²R³;
z = H, OR⁶, Halogen, CF₃ oder C₁-C₄-Alkyl;
R OH ist;
n 0, 1 oder 2 ist;
R¹, R², R³ unabhängig = H, C₁-C₆-Alkyl, gegebenenfalls substituiert durch NR⁴R⁵, OH, OR⁶, Aryl, Heterocyclyl oder Heteroaryl, cyclisches C₃-C₈-Alkyl, gegebenenfalls substituiert durch NR⁴R⁵, OH, OR⁶, Aryl, Heterocyclyl oder Heteroaryl, und Heterocyclyl;
R² und R³ zusammen einen heterocyclischen Ring bilden können;
R⁴, R⁵ unabhängig = H, C₁-C₆-Alkyl, gegebenenfalls substituiert durch OH, OR⁶, Aryl, Heterocyclyl oder Heteroaryl;
R⁶ = C₁-C₆-Alkyl, Aryl oder CF₃;
R⁷, R⁸ unabhängig = H, C₁-C₆-Alkyl, gegebenenfalls substituiert durch NR⁴R⁵, OH, OR⁶ oder Heterocyclyl, cyclisches C₃-C₈-Alkyl, gegebenenfalls substituiert durch NR⁴R⁵, OH, OR⁶ oder Heterocyclyl, und Heterocyclyl und
R⁷ und R⁸ zusammen einen heterocyclischen Ring bilden können.

17. Verbindung nach Anspruch 16, wobei die Verbindung ein pharmazeutisch akzeptables Salz einer Verbindung gemäß Formel (I) ist.

## Revendications

1. Composition ophtalmique pharmaceutique pour une utilisation dans le traitement du glaucome et pour réguler la pression intraoculaire, comprenant une quantité efficace d'un composé (I) répondant à la formule suivants : 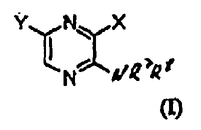 dans laquelle Y est sélectionné parmi les groupements suivantes : 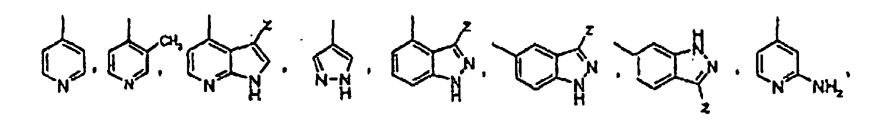 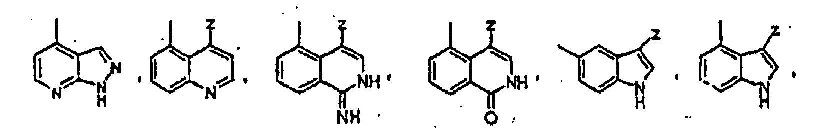 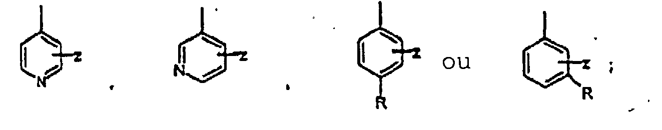 dans lesquels :
X = OR¹, NR²R³ ;
z = H, OR⁶ un atome d'halogène, CF₃ ou un alkyle en C₁-C₄ ;
R représente OH, OR⁴ ou S(O)ₙR⁶
n vaut 0, 1 ou 2 ;
R¹, R², R³ représentent indépendamment H, un alkyle en C₁-C₆ éventuellement substitué par un groupement NR⁴R⁵, OH, OR⁶, aryle, hétérocyclyle ou hétéroaryle, un alkyle cyclique en C₃-C₈ éventuellement substitué par un groupement NR⁴R⁵, OH, OR⁶, aryle, hétérocyclyle ou hétéroaryle, et un hétérocyclyle ;
R² et R³ peuvent former conjointement un cycle hétérocyclique ;
R⁴ et R⁵ représentent indépendamment H, un alkyle en C₁-C₆ éventuellement substitué par un groupement OH, OR⁶, aryle, hétérocyclyle ou hétéroaryle ;
R⁶ représente un alkyle en C₁-C₆, un aryle ou CF₃ ;
R⁷ et R⁸ représentent indépendamment H, un alkyle en C₁-C₆ éventuellement substitué par un groupement NR⁴R⁵, OH, OR⁶ ou hétérocyclyle, un alkyle cyclique en C₃-C₈ éventuellement substitué par un groupement NR⁴R⁵, OH, OR⁶ ou hétérocyclyle, et un hétérocyclyle ; et
R⁷ et R⁸ peuvent former conjointement un cycle hétérocyclique ; et
un véhicule acceptable au plan pharmaceutique pour celui-ci.

2. Composition selon la revendication 1, comprenant un sel du composé (I) acceptable au plan pharmaceutique.

3. Composition selon la revendication 1, comprenant en outre un composé sélectionné dans le groupe constitué des composés suivantes :
des conservateurs, des tensioactifs, des agents de renforcement de la viscosité, des agents améliorant la pénétration, des agents gélifiants des bases hydrophobes, des véhicules, des tampons, du chlorure de sodium et de l'eau, acceptables au plan ophtalmologique.

4. Composition selon la revendication 1, dans laquelle ladite composition comprend une pluralité d'agents permettant le traitement du glaucome.

5. Composition selon la revendication 4, dans laquelle au moins un agent de traitement du glaucome est sélectionné dans le groupe constitué des composés suivantes :
les β-bloquants, les inhibiteurs de l'anhydrase carbonique, les agonistes de type α₂, les miotiques et les agents neuroprotecteurs.

6. Composition selon la revendication 1, dans laquelle ladite composition comprend environ 0,01 pour cent en poids/volume à environ 5 pour cent en poids/volume dudit composé.

7. Composition selon la revendication 1, dans laquelle ladite composition comprend environ 0,25 pour cent en poids/volume à environ 2 pour cent en poids/volume dudit composé.

8. Utilisation d'un composé de formule suivante : 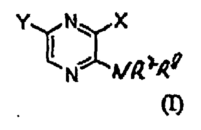 dans laquelle Y est sélectionné parmi les groupements suivantes : 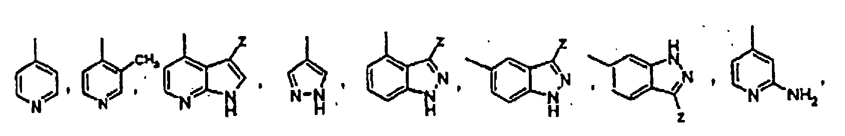 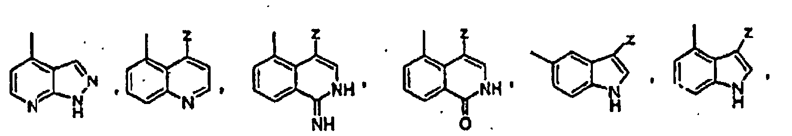 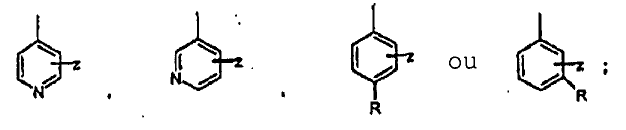 dans lesquels
X = OR¹, NR²R³ ;
z = H, OR⁶ un atome d'halogène, CF₃ ou un alkyle en C₁-C₄ ;
R représente OH, OR⁴ ou S(O)ₙR⁶;
n vaut 0, 1 ou 2 ;
R¹ R², R³ représentent indépendamment H, un alkyle en C₁-C₆ éventuellement substitué par un groupement NR⁴R⁵, OH, OR⁶, aryle, hétérocyclyle ou hétéroaryle, un alkyle cyclique en C₃-C₈ éventuellement substitué par un groupement NR⁴R⁵, OH, OR⁶, aryle, hétérocyclyle ou hétéroaryle, et un hétérocyclyle ;
R² et R³ peuvent former conjointement un cycle hétérocyclique ;
R⁴ et R⁵ représentent indépendamment H, un alkyle en C₁-C₆ éventuellement substitué par un groupement OH, OR⁶, aryle, hétérocyclyle ou hétéroaryle ;
R⁶ - un alkyle en C₁-C₆, un aryle ou CF₃ ;
R⁷ et R⁸ représentent indépendamment H, un alkyle en C₁-C₆ éventuellement substitué par un groupement NR⁴R⁵, OH, OR⁶ ou hétérocyclyle, un alkyle cyclique en C₃-C₈ éventuellement substitué par un groupement NR⁴R⁵, OH, OR⁶ ou hétérocyclyle, et un hétérocyclyle ; et
R⁷ et R⁸ peuvent former conjointement un cycle hétérocyclique ; et
un véhicule acceptable au plan pharmaceutique pour celui-ci,
pour la fabrication d'une composition ophtalmique pharmaceutique dans le but de traiter le glaucome et de réguler la pression intraoculaire.

9. Utilisation selon la revendication 8, dans laquelle la composition est adaptée pour appliquer 1 à 2 gouttes d'une composition comprenant environ 0,01 pour cent en poids/volume à environ 5 pour cent en poids/volume du composé (I), à raison de 1 à 4 fois par jour dans l'oeil affecté d'un être humain ou d'un autre mammifère.

10. Utilisation selon la revendication 8, dans laquelle ladite composition comprend une pluralité d'agents de traitement du glaucome.

11. Utilisation selon la revendication 10, dans laquelle au moins un agent de traitement du glaucome est sélectionné dans le groupe constitué des composés suivantes :
les β-bloquants, les inhibiteurs de l'anhydrase carbonique, les agonistes de type α₂, les miotiques et les agents neuroprotecteurs.

12. Utilisation d'un composé de formule suivante : 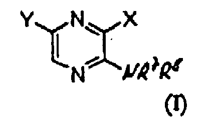 dans laquelle Y est sélectionné parmi les groupements suivantes : 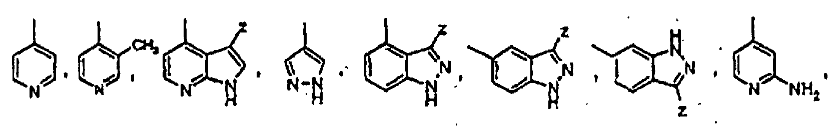 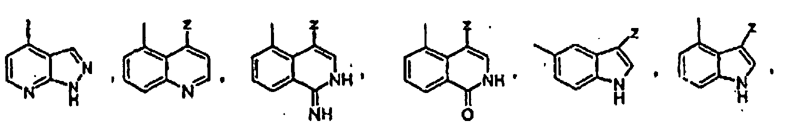 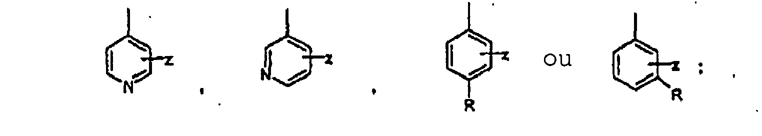 dans lesquels
X = OR¹, NR²R³ ;
z = H, OR⁶ un atome d'halogène, CF₃ ou un alkyle en C₁-C₄ ;
R représente OH, OR⁴ ou S(O)ₙR⁶ ;
n vaut 0, 1 ou 2 ;
R¹, R², R³ représentent indépendamment H, un alkyle en C₁-C₆ éventuellement substitué par un groupement NR⁴R⁵, OH, OR⁶ aryle, hétérocyclyle ou hétéroaryle, un alkyle cyclique en C₃-C₈ éventuellement substitué par un groupement NR⁴R⁵, OH, OR⁶, aryle, hétérocyclyle ou hétéroaryle, et un hétérocyclyle ;
R² et R³ peuvent former conjointement un cycle hétérocyclique ;
R⁴ et R⁵ représentent indépendamment H, un alkyle en C₁-C₆ éventuellement substitué par un groupement OH, OR⁶, aryle, hétérocyclyle ou hétéroaryle ;
R⁶ = un alkyle en C₁-C₆, un aryle ou CF₃ ;
R⁷ et R⁸ représentent indépendamment H, un alkyle en C₁-C₆ éventuellement substitué par un groupement NR⁴R⁵, OH, OR⁶ ou hétérocyclyle, un alkyle cyclique en C₃-C₈ éventuellement substitué par un groupement NR⁴R⁵, OH, OR⁶ ou hétérocyclyle, et un hétérocyclyle ; et
R⁷ et R⁸ peuvent former conjointement un cycle hétérocyclique ; et
un véhicule acceptable au plan pharmaceutique pour celui-ci
pour la fabrication d'une préparation pharmaceutique dans le but de traiter le glaucome, une rétinopathie, une contraction cérébro-vasculaire, une hypertension oculaire, un glaucome à tension normale, une maladie pulmonaire obstructive chronique, une angine de poitrine, un trouble de la circulation périphérique, une naissance prématurée, l'ostéoporose et/ou un cancer.

13. Utilisation selon la revendication 12, dans laquelle la composition est adaptée pour appliquer 1 à 2 gouttes d'une composition comprenant environ 0,01 pour cent en poids/volume à environ 5 pour cent en poids/volume du composé (I), à raison de 1 à 4 fois par jour à un être humain ou à un autre mammifère .

14. Utilisation selon la revendication 12, dans laquelle ladite composition comprend une pluralité d'agents de traitement du glaucome.

15. Utilisation selon la revendication 14, dans laquelle au moins un agent de traitement du glaucome est sélectionné dans le groupe comprenant les composés suivantes :
les β-bloquants, les inhibiteurs de l'anhydrase carbonique, les agonistes de type α₂, les miotiques et les agents neuroprotecteurs.

16. Composé représenté par la formule (I) suivante : 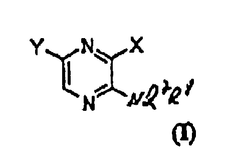 dans laquelle Y est sélectionné parmi les groupements suivantes : 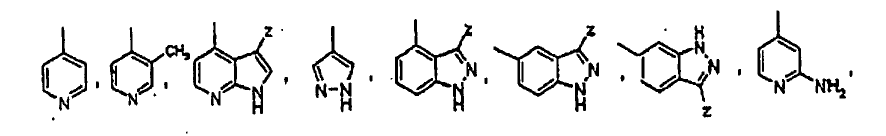 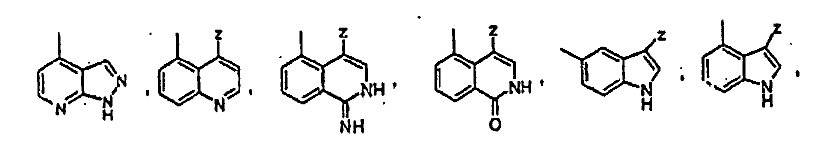 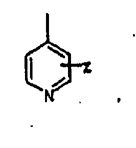 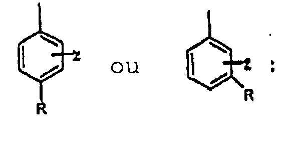 dans lesquels
X = OR¹, NR²R³ ;
z = H, OR⁶ un atome d'halogène, CF₃ ou un alkyle en C₁-C₄ ;
R représente OH ;
n vaut 0, 1 ou 2 ;
R¹, R², R³ représentent indépendamment H, un alkyle en C₁-C₆ éventuellement substitué par un groupement NR⁴R⁵, OH, OR⁶, aryle, hétérocyclyle ou hétéroaryle, un alkyle cyclique en C₃-C₈ éventuellement substitué par un groupement NR⁴R⁵, OH, OR⁶ aryle, hétérocyclyle ou hétéroaryle, et un hétérocyclyle ;
R² et R³ peuvent former conjointement un cycle hétérocyclique ;
R⁴ et R⁵ représentent indépendamment H, un alkyle en C₁-C₆ éventuellement substitué par un groupement OH, OR⁶, aryle, hétérocyclyle ou hétéroaryle ;
R⁶ = un alkyle en C₁-C₆, un aryle ou CF₃ ;
R⁷ et R⁸ représentent indépendamment H, un alkyle en C₁-C₆ éventuellement substitué par un groupement NR⁴R⁵, OH, OR⁶ ou hétérocyclyle, un alkyle cyclique en C₃-C₈ éventuellement substitué par un groupement NR⁴R⁵, OH, OR⁶ ou hétérocyclyle, et un hétérocyclyle ; et
R⁷ et R⁸ peuvent former conjointement un cycle hétérocyclique.

17. Composé selon la revendication 16, dans lequel le composé est un sel acceptable au plan pharmaceutique d'un composé répondant à la formule (I).
